# EUROPEAN PATENT APPLICATION

(11) **EP 3 725 322 A1**
(43) Date of publication of application: **21.10.2020**
(21) Application number: 19169818.2
(22) Date of filing: 17.04.2019
(51) Int. Cl.: A61K 35/761, A61K 48/00, C07K 14/47, C12N 9/64, C12N 15/86

(54) **ONCOLYTIC ADENOVIRAL VECTOR EXPRESSING GRANZYME AND PERFORIN**

(71) Applicant: TARGOVAX ASA, 0283 Oslo (NO)
(72) Inventor: KURYK, Lukasz, 00180 Helsinki (FI); ERIKSEN, Jon Amund, 3916 Porsgrunn (NO); SKORPIL, Peter, 1447 Drøbak (NO); BURNS, Robert, Oxford, OX1 5ER (GB); MØLLER, Anne-Sophie, 1358 Jar (NO)
(74) Representative: Boco IP Oy Ab

(57) **Abstract**

The present invention relates to cancer therapies. More specifically, the present invention relates to oncolytic adenoviral vectors and cells and pharmaceutical compositions comprising said vectors. The present invention also relates to a use of said vectors in the manufacture of a medicament for treating cancer in a subject and a method of treating cancer in a subject. Furthermore, the present invention relates to methods of producing granzyme and perforin in a cell and increasing anti-tumor effect and induction of specific immune response in a subject, as well as uses of the oncolytic adenoviral vector of the invention for producing transgenes in a cell and increasing anti-tumor effect and generation of specific immune response in a subject.

SEQUENCE LISTING

<110> Targovax ASA

<120> ONCOLYTIC ADENOVIRAL VECTOR EXPRESSING GRANZYME AND PERFORIN

<130> b8373PEP

<160> 47

<170> BiSSAP 1.3.6

<210> 1
<211> 37146
<212> DNA
<213> Artificial Sequence

<220>
<223> Ad5/3-D24-Perf-Grb (TGX-14)

<400> 1
attaacatca tcaataatat accttatttt ggattgaagc caatatgata atgagggggt 60

ggagtttgtg acgtggcgcg gggcgtggga acggggcggg tgacgtagta gtgtggcgga 120

agtgtgatgt tgcaagtgtg gcggaacaca tgtaagcgac ggatgtggca aaagtgacgt 180

ttttggtgtg cgccggtgta cacaggaagt gacaattttc gcgcggtttt aggcggatgt 240

tgtagtaaat ttgggcgtaa ccgagtaaga tttggccatt ttcgcgggaa aactgaataa 300

gaggaagtga aatctgaata attttgtgtt actcatagcg cgtaatattt gtctagggcc 360

gcggggactt tgaccgttta cgtggagact cgcccaggtg tttttctcag gtgttttccg 420

cgttccgggt caaagttggc gttttattat tatagtcagc tgacgtgtag tgtatttata 480

cccggtgagt tcctcaagag gccactcttg agtgccagcg agtagagttt tctcctccga 540

gccgctccga caccgggact gaaaatgaga catattatct gccacggagg tgttattacc 600

gaagaaatgg ccgccagtct tttggaccag ctgatcgaag aggtactggc tgataatctt 660

ccacctccta gccattttga accacctacc cttcacgaac tgtatgattt agacgtgacg 720

gcccccgaag atcccaacga ggaggcggtt tcgcagattt ttcccgactc tgtaatgttg 780

gcggtgcagg aagggattga cttactcact tttccgccgg cgcccggttc tccggagccg 840

cctcaccttt cccggcagcc cgagcagccg gagcagagag ccttgggtcc ggtttctatg 900

ccaaaccttg taccggaggt gatcgatcca cccagtgacg acgaggatga agagggtgag 960

gagtttgtgt tagattatgt ggagcacccc gggcacggtt gcaggtcttg tcattatcac 1020

cggaggaata cgggggaccc agatattatg tgttcgcttt gctatatgag gacctgtggc 1080

atgtttgtct acagtaagtg aaaattatgg gcagtgggtg atagagtggt gggtttggtg 1140

tggtaatttt ttttttaatt tttacagttt tgtggtttaa agaattttgt attgtgattt 1200

ttttaaaagg tcctgtgtct gaacctgagc ctgagcccga gccagaaccg gagcctgcaa 1260

gacctacccg ccgtcctaaa atggcgcctg ctatcctgag acgcccgaca tcacctgtgt 1320

ctagagaatg caatagtagt acggatagct gtgactccgg tccttctaac acacctcctg 1380

agatacaccc ggtggtcccg ctgtgcccca ttaaaccagt tgccgtgaga gttggtgggc 1440

gtcgccaggc tgtggaatgt atcgaggact tgcttaacga gcctgggcaa cctttggact 1500

tgagctgtaa acgccccagg ccataaggtg taaacctgtg attgcgtgtg tggttaacgc 1560

ctttgtttgc tgaatgagtt gatgtaagtt taataaaggg tgagataatg tttaacttgc 1620

atggcgtgtt aaatggggcg gggcttaaag ggtatataat gcgccgtggg ctaatcttgg 1680

ttacatctga cctcatggag gcttgggagt gtttggaaga tttttctgct gtgcgtaact 1740

tgctggaaca gagctctaac agtacctctt ggttttggag gtttctgtgg ggctcatccc 1800

aggcaaagtt agtctgcaga attaaggagg attacaagtg ggaatttgaa gagcttttga 1860

aatcctgtgg tgagctgttt gattctttga atctgggtca ccaggcgctt ttccaagaga 1920

aggtcatcaa gactttggat ttttccacac cggggcgcgc tgcggctgct gttgcttttt 1980

tgagttttat aaaggataaa tggagcgaag aaacccatct gagcgggggg tacctgctgg 2040

attttctggc catgcatctg tggagagcgg ttgtgagaca caagaatcgc ctgctactgt 2100

tgtcttccgt ccgcccggcg ataataccga cggaggagca gcagcagcag caggaggaag 2160

ccaggcggcg gcggcaggag cagagcccat ggaacccgag agccggcctg gaccctcggg 2220

aatgaatgtt gtacaggtgg ctgaactgta tccagaactg agacgcattt tgacaattac 2280

agaggatggg caggggctaa agggggtaaa gagggagcgg ggggcttgtg aggctacaga 2340

ggaggctagg aatctagctt ttagcttaat gaccagacac cgtcctgagt gtattacttt 2400

tcaacagatc aaggataatt gcgctaatga gcttgatctg ctggcgcaga agtattccat 2460

agagcagctg accacttact ggctgcagcc aggggatgat tttgaggagg ctattagggt 2520

atatgcaaag gtggcactta ggccagattg caagtacaag atcagcaaac ttgtaaatat 2580

caggaattgt tgctacattt ctgggaacgg ggccgaggtg gagatagata cggaggatag 2640

ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaaggttcc ggggctgtgc cttttactgc tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga agtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aacggtccgg 4020

gccagagtgg ccaacataaa taaaaaacca gactctgttt ggatttggat caagcaagtg 4080

tcttgctgtc tttatttagg ggttttgcgc gcgcggtagg cccgggacca gcggtctcgg 4140

tcgttgaggg tcctgtgtat tttttccagg acgtggtaaa ggtgactctg gatgttcaga 4200

tacatgggca taagcccgtc tctggggtgg aggtagcacc actgcagagc ttcatgctgc 4260

ggggtggtgt tgtagatgat ccagtcgtag caggagcgct gggcgtggtg cctaaaaatg 4320

tctttcagta gcaagctgat tgccaggggc aggcccttgg tgtaagtgtt tacaaagcgg 4380

ttaagctggg atgggtgcat acgtggggat atgagatgca tcttggactg tatttttagg 4440

ttggctatgt tcccagccat atccctccgg ggattcatgt tgtgcagaac caccagcaca 4500

gtgtatccgg tgcacttggg aaatttgtca tgtagcttag aaggaaatgc gtggaagaac 4560

ttggagacgc ccttgtgacc tccaagattt tccatgcatt cgtccataat gatggcaatg 4620

ggcccacggg cggcggcctg ggcgaagata tttctgggat cactaacgtc atagttgtgt 4680

tccaggatga gatcgtcata ggccattttt acaaagcgcg ggcggagggt gccagactgc 4740

ggtataatgg ttccatccgg cccaggggcg tagttaccct cacagatttg catttcccac 4800

gctttgagtt cagatggggg gatcatgtct acctgcgggg cgatgaagaa aacggtttcc 4860

ggggtagggg agatcagctg ggaagaaagc aggttcctga gcagctgcga cttaccgcag 4920

ccggtgggcc cgtaaatcac acctattacc ggctgcaact ggtagttaag agagctgcag 4980

ctgccgtcat ccctgagcag gggggccact tcgttaagca tgtccctgac tcgcatgttt 5040

tccctgacca aatccgccag aaggcgctcg ccgcccagcg atagcagttc ttgcaaggaa 5100

gcaaagtttt tcaacggttt gagaccgtcc gccgtaggca tgcttttgag cgtttgacca 5160

agcagttcca ggcggtccca cagctcggtc acctgctcta cggcatctcg atccagcata 5220

tctcctcgtt tcgcgggttg gggcggcttt cgctgtacgg cagtagtcgg tgctcgtcca 5280

gacgggccag ggtcatgtct ttccacgggc gcagggtcct cgtcagcgta gtctgggtca 5340

cggtgaaggg gtgcgctccg ggctgcgcgc tggccagggt gcgcttgagg ctggtcctgc 5400

tggtgctgaa gcgctgccgg tcttcgccct gcgcgtcggc caggtagcat ttgaccatgg 5460

tgtcatagtc cagcccctcc gcggcgtggc ccttggcgcg cagcttgccc ttggaggagg 5520

cgccgcacga ggggcagtgc agacttttga gggcgtagag cttgggcgcg agaaataccg 5580

attccgggga gtaggcatcc gcgccgcagg ccccgcagac ggtctcgcat tccacgagcc 5640

aggtgagctc tggccgttcg gggtcaaaaa ccaggtttcc cccatgcttt ttgatgcgtt 5700

tcttacctct ggtttccatg agccggtgtc cacgctcggt gacgaaaagg ctgtccgtgt 5760

ccccgtatac agacttgaga ggcctgtcct cgagcggtgt tccgcggtcc tcctcgtata 5820

gaaactcgga ccactctgag acaaaggctc gcgtccaggc cagcacgaag gaggctaagt 5880

gggaggggta gcggtcgttg tccactaggg ggtccactcg ctccagggtg tgaagacaca 5940

tgtcgccctc ttcggcatca aggaaggtga ttggtttgta ggtgtaggcc acgtgaccgg 6000

gtgttcctga aggggggcta taaaaggggg tgggggcgcg ttcgtcctca ctctcttccg 6060

catcgctgtc tgcgagggcc agctgttggg gtgagtactc cctctgaaaa gcgggcatga 6120

cttctgcgct aagattgtca gtttccaaaa acgaggagga tttgatattc acctggcccg 6180

cggtgatgcc tttgagggtg gccgcatcca tctggtcaga aaagacaatc tttttgttgt 6240

caagcttggt ggcaaacgac ccgtagaggg cgttggacag caacttggcg atggagcgca 6300

gggtttggtt tttgtcgcga tcggcgcgct ccttggccgc gatgtttagc tgcacgtatt 6360

cgcgcgcaac gcaccgccat tcgggaaaga cggtggtgcg ctcgtcgggc accaggtgca 6420

cgcgccaacc gcggttgtgc agggtgacaa ggtcaacgct ggtggctacc tctccgcgta 6480

ggcgctcgtt ggtccagcag aggcggccgc ccttgcgcga gcagaatggc ggtagggggt 6540

ctagctgcgt ctcgtccggg gggtctgcgt ccacggtaaa gaccccgggc agcaggcgcg 6600

cgtcgaagta gtctatcttg catccttgca agtctagcgc ctgctgccat gcgcgggcgg 6660

caagcgcgcg ctcgtatggg ttgagtgggg gaccccatgg catggggtgg gtgagcgcgg 6720

aggcgtacat gccgcaaatg tcgtaaacgt agaggggctc tctgagtatt ccaagatatg 6780

tagggtagca tcttccaccg cggatgctgg cgcgcacgta atcgtatagt tcgtgcgagg 6840

gagcgaggag gtcgggaccg aggttgctac gggcgggctg ctctgctcgg aagactatct 6900

gcctgaagat ggcatgtgag ttggatgata tggttggacg ctggaagacg ttgaagctgg 6960

cgtctgtgag acctaccgcg tcacgcacga aggaggcgta ggagtcgcgc agcttgttga 7020

ccagctcggc ggtgacctgc acgtctaggg cgcagtagtc cagggtttcc ttgatgatgt 7080

catacttatc ctgtcccttt tttttccaca gctcgcggtt gaggacaaac tcttcgcggt 7140

ctttccagta ctcttggatc ggaaacccgt cggcctccga acggtaagag cctagcatgt 7200

agaactggtt gacggcctgg taggcgcagc atcccttttc tacgggtagc gcgtatgcct 7260

gcgcggcctt ccggagcgag gtgtgggtga gcgcaaaggt gtccctgacc atgactttga 7320

ggtactggta tttgaagtca gtgtcgtcgc atccgccctg ctcccagagc aaaaagtccg 7380

tgcgcttttt ggaacgcgga tttggcaggg cgaaggtgac atcgttgaag agtatctttc 7440

ccgcgcgagg cataaagttg cgtgtgatgc ggaagggtcc cggcacctcg gaacggttgt 7500

taattacctg ggcggcgagc acgatctcgt caaagccgtt gatgttgtgg cccacaatgt 7560

aaagttccaa gaagcgcggg atgcccttga tggaaggcaa ttttttaagt tcctcgtagg 7620

tgagctcttc aggggagctg agcccgtgct ctgaaagggc ccagtctgca agatgagggt 7680

tggaagcgac gaatgagctc cacaggtcac gggccattag catttgcagg tggtcgcgaa 7740

aggtcctaaa ctggcgacct atggccattt tttctggggt gatgcagtag aaggtaagcg 7800

ggtcttgttc ccagcggtcc catccaaggt tcgcggctag gtctcgcgcg gcagtcacta 7860

gaggctcatc tccgccgaac ttcatgacca gcatgaaggg cacgagctgc ttcccaaagg 7920

cccccatcca agtataggtc tctacatcgt aggtgacaaa gagacgctcg gtgcgaggat 7980

gcgagccgat cgggaagaac tggatctccc gccaccaatt ggaggagtgg ctattgatgt 8040

ggtgaaagta gaagtccctg cgacgggccg aacactcgtg ctggcttttg taaaaacgtg 8100

cgcagtactg gcagcggtgc acgggctgta catcctgcac gaggttgacc tgacgaccgc 8160

gcacaaggaa gcagagtggg aatttgagcc cctcgcctgg cgggtttggc tggtggtctt 8220

ctacttcggc tgcttgtcct tgaccgtctg gctgctcgag gggagttacg gtggatcgga 8280

ccaccacgcc gcgcgagccc aaagtccaga tgtccgcgcg cggcggtcgg agcttgatga 8340

caacatcgcg cagatgggag ctgtccatgg tctggagctc ccgcggcgtc aggtcaggcg 8400

ggagctcctg caggtttacc tcgcatagac gggtcagggc gcgggctaga tccaggtgat 8460

acctaatttc caggggctgg ttggtggcgg cgtcgatggc ttgcaagagg ccgcatcccc 8520

gcggcgcgac tacggtaccg cgcggcgggc ggtgggccgc gggggtgtcc ttggatgatg 8580

catctaaaag cggtgacgcg ggcgagcccc cggaggtagg gggggctccg gacccgccgg 8640

gagagggggc aggggcacgt cggcgccgcg cgcgggcagg agctggtgct gcgcgcgtag 8700

gttgctggcg aacgcgacga cgcggcggtt gatctcctga atctggcgcc tctgcgtgaa 8760

gacgacgggc ccggtgagct tgaacctgaa agagagttcg acagaatcaa tttcggtgtc 8820

gttgacggcg gcctggcgca aaatctcctg cacgtctcct gagttgtctt gataggcgat 8880

ctcggccatg aactgctcga tctcttcctc ctggagatct ccgcgtccgg ctcgctccac 8940

ggtggcggcg aggtcgttgg aaatgcgggc catgagctgc gagaaggcgt tgaggcctcc 9000

ctcgttccag acgcggctgt agaccacgcc cccttcggca tcgcgggcgc gcatgaccac 9060

ctgcgcgaga ttgagctcca cgtgccgggc gaagacggcg tagtttcgca ggcgctgaaa 9120

gaggtagttg agggtggtgg cggtgtgttc tgccacgaag aagtacataa cccagcgtcg 9180

caacgtggat tcgttgatat cccccaaggc ctcaaggcgc tccatggcct cgtagaagtc 9240

cacggcgaag ttgaaaaact gggagttgcg cgccgacacg gttaactcct cctccagaag 9300

acggatgagc tcggcgacag tgtcgcgcac ctcgcgctca aaggctacag gggcctcttc 9360

ttcttcttca atctcctctt ccataagggc ctccccttct tcttcttctg gcggcggtgg 9420

gggagggggg acacggcggc gacgacggcg caccgggagg cggtcgacaa agcgctcgat 9480

catctccccg cggcgacggc gcatggtctc ggtgacggcg cggccgttct cgcgggggcg 9540

cagttggaag acgccgcccg tcatgtcccg gttatgggtt ggcggggggc tgccatgcgg 9600

cagggatacg gcgctaacga tgcatctcaa caattgttgt gtaggtactc cgccgccgag 9660

ggacctgagc gagtccgcat cgaccggatc ggaaaacctc tcgagaaagg cgtctaacca 9720

gtcacagtcg caaggtaggc tgagcaccgt ggcgggcggc agcgggcggc ggtcggggtt 9780

gtttctggcg gaggtgctgc tgatgatgta attaaagtag gcggtcttga gacggcggat 9840

ggtcgacaga agcaccatgt ccttgggtcc ggcctgctga atgcgcaggc ggtcggccat 9900

gccccaggct tcgttttgac atcggcgcag gtctttgtag tagtcttgca tgagcctttc 9960

taccggcact tcttcttctc cttcctcttg tcctgcatct cttgcatcta tcgctgcggc 10020

ggcggcggag tttggccgta ggtggcgccc tcttcctccc atgcgtgtga ccccgaagcc 10080

cctcatcggc tgaagcaggg ctaggtcggc gacaacgcgc tcggctaata tggcctgctg 10140

cacctgcgtg agggtagact ggaagtcatc catgtccaca aagcggtggt atgcgcccgt 10200

gttgatggtg taagtgcagt tggccataac ggaccagtta acggtctggt gacccggctg 10260

cgagagctcg gtgtacctga gacgcgagta agccctcgag tcaaatacgt agtcgttgca 10320

agtccgcacc aggtactggt atcccaccaa aaagtgcggc ggcggctggc ggtagagggg 10380

ccagcgtagg gtggccgggg ctccgggggc gagatcttcc aacataaggc gatgatatcc 10440

gtagatgtac ctggacatcc aggtgatgcc ggcggcggtg gtggaggcgc gcggaaagtc 10500

gcggacgcgg ttccagatgt tgcgcagcgg caaaaagtgc tccatggtcg ggacgctctg 10560

gccggtcagg cgcgcgcaat cgttgacgct ctagaccgtg caaaaggaga gcctgtaagc 10620

gggcactctt ccgtggtctg gtggataaat tcgcaagggt atcatggcgg acgaccgggg 10680

ttcgagcccc gtatccggcc gtccgccgtg atccatgcgg ttaccgcccg cgtgtcgaac 10740

ccaggtgtgc gacgtcagac aacgggggag tgctcctttt ggcttccttc caggcgcggc 10800

ggctgctgcg ctagcttttt tggccactgg ccgcgcgcag cgtaagcggt taggctggaa 10860

agcgaaagca ttaagtggct cgctccctgt agccggaggg ttattttcca agggttgagt 10920

cgcgggaccc ccggttcgag tctcggaccg gccggactgc ggcgaacggg ggtttgcctc 10980

cccgtcatgc aagaccccgc ttgcaaattc ctccggaaac agggacgagc cccttttttg 11040

cttttcccag atgcatccgg tgctgcggca gatgcgcccc cctcctcagc agcggcaaga 11100

gcaagagcag cggcagacat gcagggcacc ctcccctcct cctaccgcgt caggaggggc 11160

gacatccgcg gttgacgcgg cagcagatgg tgattacgaa cccccgcggc gccgggcccg 11220

gcactacctg gacttggagg agggcgaggg cctggcgcgg ctaggagcgc cctctcctga 11280

gcggcaccca agggtgcagc tgaagcgtga tacgcgtgag gcgtacgtgc cgcggcagaa 11340

cctgtttcgc gaccgcgagg gagaggagcc cgaggagatg cgggatcgaa agttccacgc 11400

agggcgcgag ctgcggcatg gcctgaatcg cgagcggttg ctgcgcgagg aggactttga 11460

gcccgacgcg cgaaccggga ttagtcccgc gcgcgcacac gtggcggccg ccgacctggt 11520

aaccgcatac gagcagacgg tgaaccagga gattaacttt caaaaaagct ttaacaacca 11580

cgtgcgtacg cttgtggcgc gcgaggaggt ggctatagga ctgatgcatc tgtgggactt 11640

tgtaagcgcg ctggagcaaa acccaaatag caagccgctc atggcgcagc tgttccttat 11700

agtgcagcac agcaggacaa cgaggcatt cagggatgcg ctgctaaaca tagtagagcc 11760

cgagggccgc tggctgctcg atttgataaa catcctgcag agcatagtgg tgcaggagcg 11820

cagcttgagc ctggctgaca aggtggccgc catcaactat tccatgctta gcctgggcaa 11880

gttttacgcc cgcaagatat accatacccc ttacgttccc atagacaagg aggtaaagat 11940

cgaggggttc tacatgcgca tggcgctgaa ggtgcttacc ttgagcgacg acctgggcgt 12000

ttatcgcaac gagcgcatcc acaaggccgt gagcgtgagc cggcggcgcg agctcagcga 12060

ccgcgagctg atgcacagcc tgcaaagggc cctggctggc acgggcagcg gcgatagaga 12120

ggccgagtcc tactttgacg cgggcgctga cctgcgctgg gccccaagcc gacgcgccct 12180

ggaggcagct ggggccggac ctgggctggc ggtggcaccc gcgcgcgctg gcaacgtcgg 12240

cggcgtggag gaatatgacg aggacgatga gtacgagcca gaggacggcg agtactaagc 12300

ggtgatgttt ctgatcagat gatgcaagac gcaacggacc cggcggtgcg ggcggcgctg 12360

cagagccagc cgtccggcct taactccacg gacgactggc gccaggtcat ggaccgcatc 12420

atgtcgctga ctgcgcgcaa tcctgacgcg ttccggcagc agccgcaggc caaccggctc 12480

tccgcaattc tggaagcggt ggtcccggcg cgcgcaaacc ccacgcacga gaaggtgctg 12540

gcgatcgtaa acgcgctggc cgaaaacagg gccatccggc ccgacgaggc cggcctggtc 12600

tacgacgcgc tgcttcagcg cgtggctcgt tacaacagcg gcaacgtgca gaccaacctg 12660

gaccggctgg tgggggatgt gcgcgaggcc gtggcgcagc gtgagcgcgc gcagcagcag 12720

ggcaacctgg gctccatggt tgcactaaac gccttcctga gtacacagcc cgccaacgtg 12780

ccgcggggac aggaggacta caccaacttt gtgagcgcac tgcggctaat ggtgactgag 12840

acaccgcaaa gtgaggtgta ccagtctggg ccagactatt ttttccagac cagtagacaa 12900

ggcctgcaga ccgtaaacct gagccaggct ttcaaaaact tgcaggggct gtggggggtg 12960

cgggctccca caggcgaccg cgcgaccgtg tctagcttgc tgacgcccaa ctcgcgcctg 13020

ttgctgctgc taatagcgcc cttcacggac agtggcagcg tgtcccggga cacataccta 13080

ggtcacttgc tgacactgta ccgcgaggcc ataggtcagg cgcatgtgga cgagcatact 13140

ttccaggaga ttacaagtgt cagccgcgcg ctggggcagg aggacacggg cagcctggag 13200

gcaaccctaa actacctgct gaccaaccgg cggcagaaga tcccctcgtt gcacagttta 13260

aacagcgagg aggagcgcat tttgcgctac gtgcagcaga gcgtgagcct taacctgatg 13320

cgcgacgggg taacgcccag cgtggcgctg gacatgaccg cgcgcaacat ggaaccgggc 13380

atgtatgcct caaaccggcc gtttatcaac cgcctaatgg actacttgca tcgcgcggcc 13440

gccgtgaacc ccgagtattt caccaatgcc atcttgaacc cgcactggct accgccccct 13500

ggtttctaca ccgggggatt cgaggtgccc gagggtaacg atggattcct ctgggacgac 13560

atagacgaca gcgtgttttc cccgcaaccg cagaccctgc tagagttgca acagcgcgag 13620

caggcagagg cggcgctgcg aaaggaaagc ttccgcaggc caagcagctt gtccgatcta 13680

ggcgctgcgg ccccgcggtc agatgctagt agcccatttc caagcttgat agggtctctt 13740

accagcactc gcaccacccg cccgcgcctg ctgggcgagg aggagtacct aaacaactcg 13800

ctgctgcagc cgcagcgcga aaaaaacctg cctccggcat ttcccaacaa cgggatagag 13860

agcctagtgg acaagatgag tagatggaag acgtacgcgc aggagcacag ggacgtgcca 13920

ggcccgcgcc cgcccacccg tcgtcaaagg cacgaccgtc agcggggtct ggtgtgggag 13980

gacgatgact cggcagacga cagcagcgtc ctggatttgg gagggagtgg caacccgttt 14040

gcgcaccttc gccccaggct ggggagaatg ttttaaaaaa aaaaaaaagc atgatgcaaa 14100

ataaaaaact caccaaggcc atggcaccga gcgttggttt tcttgtattc cccttagtat 14160

gcggcgcgcg gcgatgtatg aggaaggtcc tcctccctcc tacgagagtg tggtgagcgc 14220

ggcgccagtg gcggcggcgc tgggttctcc cttcgatgct cccctggacc cgccgtttgt 14280

gcctccgcgg tacctgcggc ctaccggggg gagaaacagc atccgttact ctgagttggc 14340

acccctattc gacaccaccc gtgtgtacct ggtggacaac aagtcaacgg atgtggcatc 14400

cctgaactac cagaacgacc acagcaactt tctgaccacg gtcattcaaa acaatgacta 14460

cagcccgggg gaggcaagca cacagaccat caatcttgac gaccggtcgc actggggcgg 14520

cgacctgaaa accatcctgc ataccaacat gccaaatgtg aacgagttca tgtttaccaa 14580

taagtttaag gcgcgggtga tggtgtcgcg cttgcctact aaggacaatc aggtggagct 14640

gaaatacgag tgggtggagt tcacgctgcc cgagggcaac tactccgaga ccatgaccat 14700

agaccttatg aacaacgcga tcgtggagca ctacttgaaa gtgggcagac agaacggggt 14760

tctggaaagc gacatcgggg taaagtttga cacccgcaac ttcagactgg ggtttgaccc 14820

cgtcactggt cttgtcatgc ctggggtata tacaaacgaa gccttccatc cagacatcat 14880

tttgctgcca ggatgcgggg tggacttcac ccacagccgc ctgagcaact tgttgggcat 14940

ccgcaagcgg caacccttcc aggagggctt taggatcacc tacgatgatc tggagggtgg 15000

taacattccc gcactgttgg atgtggacgc ctaccaggcg agcttgaaag atgacaccga 15060

acagggcggg ggtggcgcag gcggcagcaa cagcagtggc agcggcgcgg aagagaactc 15120

caacgcggca gccgcggcaa tgcagccggt ggaggacatg aacgatcatg ccattcgcgg 15180

cgacaccttt gccacacggg ctgaggagaa gcgcgctgag gccgaagcag cggccgaagc 15240

tgccgccccc gctgcgcaac ccgaggtcga gaagcctcag aagaaaccgg tgatcaaacc 15300

cctgacagag gacagcaaga aacgcagtta caacctaata agcaatgaca gcaccttcac 15360

ccagtaccgc agctggtacc ttgcatacaa ctacggcgac cctcagaccg gaatccgctc 15420

atggaccctg ctttgcactc ctgacgtaac ctgcggctcg gagcaggtct actggtcgtt 15480

gccagacatg atgcaagacc ccgtgacctt ccgctccacg cgccagatca gcaactttcc 15540

ggtggtgggc gccgagctgt tgcccgtgca ctccaagagc ttctacaacg accaggccgt 15600

ctactcccaa ctcatccgcc agtttacctc tctgacccac gtgttcaatc gctttcccga 15660

gaaccagatt ttggcgcgcc cgccagcccc caccatcacc accgtcagtg aaaacgttcc 15720

tgctctcaca gatcacggga cgctaccgct gcgcaacagc atcggaggag tccagcgagt 15780

gaccattact gacgccagac gccgcacctg cccctacgtt tacaaggccc tgggcatagt 15840

ctcgccgcgc gtcctatcga gccgcacttt ttgagcaagc atgtccatcc ttatatcgcc 15900

cagcaataac acaggctggg gcctgcgctt cccaagcaag atgtttggcg gggccaagaa 15960

gcgctccgac caacacccag tgcgcgtgcg cgggcactac cgcgcgccct ggggcgcgca 16020

caaacgcggc cgcactgggc gcaccaccgt cgatgacgcc atcgacgcgg tggtggagga 16080

ggcgcgcaac tacacgccca cgccgccacc agtgtccaca gtggacgcgg ccattcagac 16140

cgtggtgcgc ggagcccggc gctatgctaa aatgaagaga cggcggaggc gcgtagcacg 16200

tcgccaccgc cgccgacccg gcactgccgc ccaacgcgcg gcggcggccc tgcttaaccg 16260

cgcacgtcgc accggccgac gggcggccat gcgagcagct cgaaggctgg ccgcgggtat 16320

tgtcactgtg ccccccaggt ccaggcgacg agcggccgcc gcagcagccg cggccattag 16380

tgctatgact cagggtcgca ggggcaacgt gtattgggtg cgcgactcgg ttagcggcct 16440

gcgcgtgccc gtgcgcaccc gccccccgcg caactagatt gcaagaaaaa actacttaga 16500

ctcgtactgt tgtatgtatc cagcggcggc ggcgcgcaac gaagctatgt ccaagcgcaa 16560

aatcaaagaa gagatgctcc aggtcatcgc gccggagatc tatggccccc cgaagaagga 16620

agagcaggat tacaagcccc gaaagctaaa gcgggtcaaa aagaaaaaga aagatgatga 16680

tgatgaactt gacgacgagg tggaactgct gcacgctacc gcgcccaggc gacgggtaca 16740

gtggaaaggt cgacgcgtaa aacgtgtttt gcgacccggc accaccgtag tctttacgcc 16800

cggtgagcgc tccacccgca cctacaagcg cgtgtatgat gaggtgtacg gcgacgagga 16860

cctgcttgag caggccaacg agcgcctcgg ggagtttgcc tacggaaagc ggcataagga 16920

catgctggcg ttgccgctgg acgagggcaa cccaacacct agcctaaagc ccgtaacact 16980

gcagcaggtg ctgcccgcgc ttgcaccgtc cgaagaaaag cgcggcctaa agcgcgagtc 17040

tggtgacttg gcacccaccg tgcagctgat ggtacccaag cgccagcgac tggaagatgt 17100

cttggaaaaa atgaccgtgg aacctgggct ggagcccgag gtccgcgtgc ggccaatcaa 17160

gcaggtggcg ccgggactgg gcgtgcagac cgtggacgtt cagataccca ctaccagtag 17220

caccagtatt gccaccgcca cagagggcat ggagacacaa acgtccccgg ttgcctcagc 17280

ggtggcggat gccgcggtgc aggcggtcgc tgcggccgcg tccaagacct ctacggaggt 17340

gcaaacggac ccgtggatgt ttcgcgtttc agccccccgg cgcccgcgcc gttcgaggaa 17400

gtacggcgcc gccagcgcgc tactgcccga atatgcccta catccttcca ttgcgcctac 17460

ccccggctat cgtggctaca cctaccgccc cagaagacga gcaactaccc gacgccgaac 17520

caccactgga acccgccgcc gccgtcgccg tcgccagccc gtgctggccc cgatttccgt 17580

gcgcagggtg gctcgcgaag gaggcaggac cctggtgctg ccaacagcgc gctaccaccc 17640

cagcatcgtt taaaagccgg tctttgtggt tcttgcagat atggccctca cctgccgcct 17700

ccgtttcccg gtgccgggat tccgaggaag aatgcaccgt aggaggggca tggccggcca 17760

cggcctgacg ggcggcatgc gtcgtgcgca ccaccggcgg cggcgcgcgt cgcaccgtcg 17820

catgcgcggc ggtatcctgc ccctccttat tccactgatc gccgcggcga ttggcgccgt 17880

gcccggaatt gcatccgtgg ccttgcaggc gcagagacac tgattaaaaa caagttgcat 17940

gtggaaaaat caaaataaaa agtctggact ctcacgctcg cttggtcctg taactatttt 18000

gtagaatgga agacatcaac tttgcgtctc tggccccgcg acacggctcg cgcccgttca 18060

tgggaaactg gcaagatatc ggcaccagca atatgagcgg tggcgccttc agctggggct 18120

cgctgtggag cggcattaaa aatttcggtt ccaccgttaa gaactatggc agcaaggcct 18180

ggaacagcag cacaggccag atgctgaggg ataagttgaa agagcaaaat ttccaacaaa 18240

aggtggtaga tggcctggcc tctggcatta gcggggtggt ggacctggcc aaccaggcag 18300

tgcaaaataa gattaacagt aagcttgatc cccgccctcc cgtagaggag cctccaccgg 18360

ccgtggagac agtgtctcca gaggggcgtg gcgaaaagcg tccgcgcccc gacagggaag 18420

aaactctggt gacgcaaata gacgagcctc cctcgtacga ggaggcacta aagcaaggcc 18480

tgcccaccac ccgtcccatc gcgcccatgg ctaccggagt gctgggccag cacacacccg 18540

taacgctgga cctgcctccc cccgccgaca cccagcagaa acctgtgctg ccaggcccga 18600

ccgccgttgt tgtaacccgt cctagccgcg cgtccctgcg ccgcgccgcc agcggtccgc 18660

gatcgttgcg gcccgtagcc agtggcaact ggcaaagcac actgaacagc atcgtgggtc 18720

tgggggtgca atccctgaag cgccgacgat gcttctgata gctaacgtgt cgtatgtgtg 18780

tcatgtatgc gtccatgtcg ccgccagagg agctgctgag ccgccgcgcg cccgctttcc 18840

aagatggcta ccccttcgat gatgccgcag tggtcttaca tgcacatctc gggccaggac 18900

gcctcggagt acctgagccc cgggctggtg cagtttgccc gcgccaccga gacgtacttc 18960

agcctgaata acaagtttag aaaccccacg gtggcgccta cgcacgacgt gaccacagac 19020

cggtcccagc gtttgacgct gcggttcatc cctgtggacc gtgaggatac tgcgtactcg 19080

tacaaggcgc ggttcaccct agctgtgggt gataaccgtg tgctggacat ggcttccacg 19140

tactttgaca tccgcggcgt gctggacagg ggccctactt ttaagcccta ctctggcact 19200

gcctacaacg ccctggctcc caagggtgcc ccaaatcctt gcgaatggga tgaagctgct 19260

actgctcttg aaataaacct agaagaagag gacgatgaca acgaagacga agtagacgag 19320

caagctgagc agcaaaaaac tcacgtattt gggcaggcgc cttattctgg tataaatatt 19380

acaaaggagg gtattcaaat aggtgtcgaa ggtcaaacac ctaaatatgc cgataaaaca 19440

tttcaacctg aacctcaaat aggagaatct cagtggtacg aaacagaaat taatcatgca 19500

gctgggagag tcctaaaaaa gactacccca atgaaaccat gttacggttc atatgcaaaa 19560

cccacaaatg aaaatggagg gcaaggcatt cttgtaaagc aacaaaatgg aaagctagaa 19620

agtcaagtgg aaatgcaatt tttctcaact actgaggcag ccgcaggcaa tggtgataac 19680

ttgactccta aagtggtatt gtacagtgaa gatgtagata tagaaacccc agacactcat 19740

atttcttaca tgcccactat taaggaaggt aactcacgag aactaatggg ccaacaatct 19800

atgcccaaca ggcctaatta cattgctttt agggacaatt ttattggtct aatgtattac 19860

aacagcacgg gtaatatggg tgttctggcg ggccaagcat cgcagttgaa tgctgttgta 19920

gatttgcaag acagaaacac agagctttca taccagcttt tgcttgattc cattggtgat 19980

agaaccaggt acttttctat gtggaatcag gctgttgaca gctatgatcc agatgttaga 20040

attattgaaa atcatggaac tgaagatgaa cttccaaatt actgctttcc actgggaggt 20100

gtgattaata cagagactct taccaaggta aaacctaaaa caggtcagga aaatggatgg 20160

gaaaaagatg ctacagaatt ttcagataaa aatgaaataa gagttggaaa taattttgcc 20220

atggaaatca atctaaatgc caacctgtgg agaaatttcc tgtactccaa catagcgctg 20280

tatttgcccg acaagctaaa gtacagtcct tccaacgtaa aaatttctga taacccaaac 20340

acctacgact acatgaacaa gcgagtggtg gctcccgggc tagtggactg ctacattaac 20400

cttggagcac gctggtccct tgactatatg gacaacgtca acccatttaa ccaccaccgc 20460

aatgctggcc tgcgctaccg ctcaatgttg ctgggcaatg gtcgctatgt gcccttccac 20520

atccaggtgc ctcagaagtt ctttgccatt aaaaacctcc ttctcctgcc gggctcatac 20580

acctacgagt ggaacttcag gaaggatgtt aacatggttc tgcagagctc cctaggaaat 20640

gacctaaggg ttgacggagc cagcattaag tttgatagca tttgccttta cgccaccttc 20700

ttccccatgg cccacaacac cgcctccacg cttgaggcca tgcttagaaa cgacaccaac 20760

gaccagtcct ttaacgacta tctctccgcc gccaacatgc tctaccctat acccgccaac 20820

gctaccaacg tgcccatatc catcccctcc cgcaactggg cggctttccg cggctgggcc 20880

ttcacgcgcc ttaagactaa ggaaacccca tcactgggct cgggctacga cccttattac 20940

acctactctg gctctatacc ctacctagat ggaacctttt acctcaacca cacctttaag 21000

aaggtggcca ttacctttga ctcttctgtc agctggcctg gcaatgaccg cctgcttacc 21060

cccaacgagt ttgaaattaa gcgctcagtt gacggggagg gttacaacgt tgcccagtgt 21120

aacatgacca aagactggtt cctggtacaa atgctagcta actataacat tggctaccag 21180

ggcttctata tcccagagag ctacaaggac cgcatgtact ccttctttag aaacttccag 21240

cccatgagcc gtcaggtggt ggatgatact aaatacaagg actaccaaca ggtgggcatc 21300

ctacaccaac acaacaactc tggatttgtt ggctaccttg cccccaccat gcgcgaagga 21360

caggcctacc ctgctaactt cccctatccg cttataggca agaccgcagt tgacagcatt 21420

acccagaaaa agtttctttg cgatcgcacc ctttggcgca tcccattctc cagtaacttt 21480

atgtccatgg gcgcactcac agacctgggc caaaaccttc tctacgccaa ctccgcccac 21540

gcgctagaca tgacttttga ggtggatccc atggacgagc ccacccttct ttatgttttg 21600

tttgaagtct ttgacgtggt ccgtgtgcac cagccgcacc gcggcgtcat cgaaaccgtg 21660

tacctgcgca cgcccttctc ggccggcaac gccacaacat aaagaagcaa gcaacatcaa 21720

caacagctgc cgccatgggc tccagtgagc aggaactgaa agccattgtc aaagatcttg 21780

gttgtgggcc atattttttg ggcacctatg acaagcgctt tccaggcttt gtttctccac 21840

acaagctcgc ctgcgccata gtcaatacgg ccggtcgcga gactgggggc gtacactgga 21900

tggcctttgc ctggaacccg cactcaaaaa catgctacct ctttgagccc tttggctttt 21960

ctgaccagcg actcaagcag gtttaccagt ttgagtacga gtcactcctg cgccgtagcg 22020

ccattgcttc ttcccccgac cgctgtataa cgctggaaaa gtccacccaa agcgtacagg 22080

ggcccaactc ggccgcctgt ggactattct gctgcatgtt tctccacgcc tttgccaact 22140

ggccccaaac tcccatggat cacaacccca ccatgaacct tattaccggg gtacccaact 22200

ccatgctcaa cagtccccag gtacagccca ccctgcgtcg caaccaggaa cagctctaca 22260

gcttcctgga gcgccactcg ccctacttcc gcagccacag tgcgcagatt aggagcgcca 22320

cttctttttg tcacttgaaa aacatgtaaa aataatgtac tagagacact ttcaataaag 22380

gcaaatgctt ttatttgtac actctcgggt gattatttac ccccaccctt gccgtctgcg 22440

ccgtttaaaa atcaaagggg ttctgccgcg catcgctatg cgccactggc agggacacgt 22500

tgcgatactg gtgtttagtg ctccacttaa actcaggcac aaccatccgc ggcagctcgg 22560

tgaagttttc actccacagg ctgcgcacca tcaccaacgc gtttagcagg tcgggcgccg 22620

atatcttgaa gtcgcagttg gggcctccgc cctgcgcgcg cgagttgcga tacacagggt 22680

tgcagcactg gaacactatc agcgccgggt ggtgcacgct ggccagcacg ctcttgtcgg 22740

agatcagatc cgcgtccagg tcctccgcgt tgctcagggc gaacggagtc aactttggta 22800

gctgccttcc caaaaagggc gcgtgcccag gctttgagtt gcactcgcac cgtagtggca 22860

tcaaaaggtg accgtgcccg gtctgggcgt taggatacag cgcctgcata aaagccttga 22920

tctgcttaaa agccacctga gcctttgcgc cttcagagaa gaacatgccg caagacttgc 22980

cggaaaactg attggccgga cacgcggcgt cgtgcacgca gcaccttgcg tcggtgttgg 23040

agatctgcac cacatttcgg ccccaccggt tcttcacgat cttggccttg ctagactgct 23100

ccttcagcgc gcgctgcccg ttttcgctcg tcacatccat ttcaatcacg tgctccttat 23160

ttatcataat gcttccgtgt agacacttaa gctcgccttc gatctcagcg cagcggtgca 23220

gccacaacgc gcagcccgtg ggctcgtgat gcttgtaggt cacctctgca aacgactgca 23280

ggtacgcctg caggaatcgc cccatcatcg tcacaaaggt cttgttgctg gtgaaggtca 23340

gctgcaaccc gcggtgctcc tcgttcagcc aggtcttgca tacggccgcc agagcttcca 23400

cttggtcagg cagtagtttg aagttcgcct ttagatcgtt atccacgtgg tacttgtcca 23460

tcagcgcgcg cgcagcctcc atgcccttct cccacgcaga cacgatcggc acactcagcg 23520

ggttcatcac cgtaatttca ctttccgctt cgctgggctc ttcctcttcc tcttgcgtcc 23580

gcataccacg cgccactggg tcgtcttcat tcagccgccg cactgtgcgc ttacctcctt 23640

tgccatgctt gattagcacc ggtgggttgc tgaaacccac catttgtagc gccacatctt 23700

ctctttcttc ctcgctgtcc acgattacct ctggtgatgg cgggcgctcg ggcttgggag 23760

aagggcgctt ctttttcttc ttgggcgcaa tggccaaatc cgccgccgag gtcgatggcc 23820

gcgggctggg tgtgcgcggc accagcgcgt cttgtgatga gtcttcctcg tcctcggact 23880

cgatacgccg cctcatccgc ttttttgggg gcgcccgggg aggcggcggc gacggggacg 23940

gggacgacac gtcctccatg gttgggggac gtcgcgccgc accgcgtccg cgctcggggg 24000

tggtttcgcg ctgctcctct tcccgactgg ccatttcctt ctcctatagg cagaaaaaga 24060

tcatggagtc agtcgagaag aaggacagcc taaccgcccc ctctgagttc gccaccaccg 24120

cctccaccga tgccgccaac gcgcctacca ccttccccgt cgaggcaccc ccgcttgagg 24180

aggaggaagt gattatcgag caggacccag gttttgtaag cgaagacgac gaggaccgct 24240

cagtaccaac agaggataaa aagcaagacc aggacaacgc agaggcaaac gaggaacaag 24300

tcgggcgggg ggacgaaagg catggcgact acctagatgt gggagacgac gtgctgttga 24360

agcatctgca gcgccagtgc gccattatct gcgacgcgtt gcaagagcgc agcgatgtgc 24420

ccctcgccat agcggatgtc agccttgcct acgaacgcca cctattctca ccgcgcgtac 24480

cccccaaacg ccaagaaaac ggcacatgcg agcccaaccc gcgcctcaac ttctaccccg 24540

tatttgccgt gccagaggtg cttgccacct atcacatctt tttccaaaac tgcaagatac 24600

ccctatcctg ccgtgccaac cgcagccgag cggacaagca gctggccttg cggcagggcg 24660

ctgtcatacc tgatatcgcc tcgctcaacg aagtgccaaa aatctttgag ggtcttggac 24720

gcgacgagaa gcgcgcggca aacgctctgc aacaggaaaa cagcgaaaat gaaagtcact 24780

ctggagtgtt ggtggaactc gagggtgaca acgcgcgcct agccgtacta aaacgcagca 24840

tcgaggtcac ccactttgcc tacccggcac ttaacctacc ccccaaggtc atgagcacag 24900

tcatgagtga gctgatcgtg cgccgtgcgc agcccctgga gagggatgca aatttgcaag 24960

aacaaacaga ggagggccta cccgcagttg gcgacgagca gctagcgcgc tggcttcaaa 25020

cgcgcgagcc tgccgacttg gaggagcgac gcaaactaat gatggccgca gtgctcgtta 25080

ccgtggagct tgagtgcatg cagcggttct ttgctgaccc ggagatgcag cgcaagctag 25140

aggaaacatt gcactacacc tttcgacagg gctacgtacg ccaggcctgc aagatctcca 25200

acgtggagct ctgcaacctg gtctcctacc ttggaatttt gcacgaaaac cgccttgggc 25260

aaaacgtgct tcattccacg ctcaagggcg aggcgcgccg cgactacgtc cgcgactgcg 25320

tttacttatt tctatgctac acctggcaga cggccatggg cgtttggcag cagtgcttgg 25380

aggagtgcaa cctcaaggag ctgcagaaac tgctaaagca aaacttgaag gacctatgga 25440

cggccttcaa cgagcgctcc gtggccgcgc acctggcgga catcattttc cccgaacgcc 25500

tgcttaaaac cctgcaacag ggtctgccag acttcaccag tcaaagcatg ttgcagaact 25560

ttaggaactt tatcctagag cgctcaggaa tcttgcccgc cacctgctgt gcacttccta 25620

gcgactttgt gcccattaag taccgcgaat gccctccgcc gctttggggc cactgctacc 25680

ttctgcagct agccaactac cttgcctacc actctgacat aatggaagac gtgagcggtg 25740

acggtctact ggagtgtcac tgtcgctgca acctatgcac cccgcaccgc tccctggttt 25800

gcaattcgca gctgcttaac gaaagtcaaa ttatcggtac ctttgagctg cagggtccct 25860

cgcctgacga aaagtccgcg gctccggggt tgaaactcac tccggggctg tggacgtcgg 25920

cttaccttcg caaatttgta cctgaggact accacgccca cgagattagg ttctacgaag 25980

accaatcccg cccgcctaat gcggagctta ccgcctgcgt cattacccag ggccacattc 26040

ttggccaatt gcaagccatc aacaaagccc gccaagagtt tctgctacga aagggacggg 26100

gggtttactt ggacccccag tccggcgagg agctcaaccc aatccccccg ccgccgcagc 26160

cctatcagca gcagccgcgg gcccttgctt cccaggatgg cacccaaaaa gaagctgcag 26220

ctgccgccgc cacccacgga cgaggaggaa tactgggaca gtcaggcaga ggaggttttg 26280

gacgaggagg aggaggacat gatggaagac tgggagagcc tagacgagga agcttccgag 26340

gtcgaagagg tgtcagacga aacaccgtca ccctcggtcg cattcccctc gccggcgccc 26400

cagaaatcgg caaccggttc cagcatggct acaacctccg ctcctcaggc gccgccggca 26460

ctgcccgttc gccgacccaa ccgtagatgg gacaccactg gaaccagggc cggtaagtcc 26520

aagcagccgc cgccgttagc ccaagagcaa caacagcgcc aaggctaccg ctcatggcgc 26580

gggcacaaga acgccatagt tgcttgcttg caagactgtg ggggcaacat ctccttcgcc 26640

cgccgctttc ttctctacca tcacggcgtg gccttccccc gtaacatcct gcattactac 26700

cgtcatctct acagcccata ctgcaccggc ggcagcggca gcaacagcag cggccacaca 26760

gaagcaaagg cgaccggata gcaagactct gacaaagccc aagaaatcca cagcggcggc 26820

agcagcagga ggaggagcgc tgcgtctggc gcccaacgaa cccgtatcga cccgcgagct 26880

tagaaacagg atttttccca ctctgtatgc tatatttcaa cagagcaggg gccaagaaca 26940

agagctgaaa ataaaaaaca ggtctctgcg atccctcacc cgcagctgcc tgtatcacaa 27000

aagcgaagat cagcttcggc gcacgctgga agacgcggag gctctcttca gtaaatactg 27060

cgcgctgact cttaaggact agtttcgcgc cctttctcaa atttaagcgc gaaaactacg 27120

tcatctccag cggccacacc cggcgccagc acctgttgtc agcgccatta tgagcaagga 27180

aattcccacg ccctacatgt ggagttacca gccacaaatg ggacttgcgg ctggagctgc 27240

ccaagactac tcaacccgaa taaactacat gagcgcggga ccccacatga tatcccgggt 27300

caacggaata cgcgcccacc gaaaccgaat tctcctggaa caggcggcta ttaccaccac 27360

acctcgtaat aaccttaatc cccgtagttg gcccgctgcc ctggtgtacc aggaaagtcc 27420

cgctcccacc actgtggtac ttcccagaga cgcccaggcc gaagttcaga tgactaactc 27480

aggggcgcag cttgcgggcg gctttcgtca cagggtgcgg tcgcccgggc agggtataac 27540

tcacctgaca atcagagggc gaggtattca gctcaacgac gagtcggtga gctcctcgct 27600

tggtctccgt ccggacggga catttcagat cggcggcgcc ggccgctctt cattcacgcc 27660

tcgtcaggca atcctaactc tgcagacctc gtcctctgag ccgcgctctg gaggcattgg 27720

aactctgcaa tttattgagg agtttgtgcc atcggtctac tttaacccct tctcgggacc 27780

tcccggccac tatccggatc aatttattcc taactttgac gcggtaaagg actcggcgga 27840

cggctacgac tgaggccaat gaggccatgt taagtggaga ggcagagcaa ctgcgcctga 27900

aacacctggt ccactgtcgc cgccacaagt gctttgcccg cgactccggt gagttttgct 27960

actttgaatt gcccgaggat catatcgagg gcccggcgca cggcgtccgg cttaccgccc 28020

agggagagct tgcccgtagc ctgattcggg agtttaccca gcgccccctg ctagttgagc 28080

gggacagggg accctgtgtt ctcactgtga tttgcaactg tcctaaccct ggattacatc 28140

aagatccaga tcttctagac ccgggagcgg ccgctgtcga cctgcaggat ccgaattcga 28200

tatcactagt tattaatagt aatcaattac ggggtcatta gttcatagcc catatatgga 28260

gttccgcgtt acataactta cggtaaatgg cccgcctggc tgaccgccca acgacccccg 28320

cccattgacg tcaataatga cgtatgttcc catagtaacg ccaataggga ctttccattg 28380

acgtcaatgg gtggagtatt tacggtaaac tgcccacttg gcagtacatc aagtgtatca 28440

tatgccaagt ccgcccccta ttgacgtcaa tgacggtaaa tggcccgcct ggcattatgc 28500

ccagtacatg accttacggg actttcctac ttggcagtac atctacgtat tagtcatcgc 28560

tattaccatg gtgatgcggt tttggcagta caccaatggg cgtggatagc ggtttgactc 28620

acggggattt ccaagtctcc accccattga cgtcaatggg agtttgtttt ggcaccaaaa 28680

tcaacgggac tttccaaaat gtcgtaatac ccccgccccg ttgacgcaaa tgggcaagct 28740

tgccgggtcg aggtaggcgt gtacggtggg aggcctatat aagcaaccgg tataatcaaa 28800

cagaccagat tgtctgtttg ttaccggtgt ttagtgaacc gggcgcgcct catatcgcct 28860

ggagacgcca tccacgctgt tttgacctcc atagaagaca ccgggaccga tccagcctcc 28920

gcgggccgcc accatgcaac caatcctgct tctgctggcc ttcctcctgc tgcccagggc 28980

agatgcaggg gagatcatcg ggggacatga ggccaagccc cactcccgcc cctacatggc 29040

ttatcttatg atctgggatc agaagtctct gaagaggtgc ggtggcttcc tgatacaaga 29100

cgacttcgtg ctgacagctg ctcactgttg gggaagctcc ataaatgtca ccttgggggc 29160

ccacaatatc aaagaacagg agccgaccca gcagtttatc cctgtgaaaa gacccatccc 29220

ccatccagcc tataatccta agaacttctc caacgacatc atgctactgc agctggagag 29280

aaaggccaag cggaccagag ctgtgcagcc cctcaggcta cctagcaaca aggcccaggt 29340

gaagccaggg cagacatgca gtgtggccgg ctgggggcag acggcccccc tgggaaaaca 29400

ctcacacaca ctacaagagg tgaagatgac agtgcaggaa gatcgaaagt gcgaatctga 29460

cttacgccat tattacgaca gtaccattga gttgtgcgtg ggggacccag agattaaaaa 29520

gacttccttt aagggggact ctggaggccc tcttgtgtgt aacaaggtgg cccagggcat 29580

tgtctcctat ggacgaaaca atggcatgcc tccacgagcc tgcaccaaag tctcaagctt 29640

tgtacactgg ataaagaaaa ccatgaaacg ccactaaaac ttgtttattg cagcttataa 29700

tggttacaaa taaagcaata gcatcacaaa tttcacaaat aaagcatttt tttcactgca 29760

ttctagttgt ggtttgtcca aactcatcaa tgtatcttaa ctagtggtac cgatcttatt 29820

ccctttaact aataaaaaaa aataataaag catcacttac ttaaaatcag ttagcaaatt 29880

tctgtccagt ttattcagca gcacctcctt gccctcctcc cagctctggt attgcagctt 29940

cctcctggct gcaaactttc tccacaatct aaatggaatg tcagtttcct cctgttcctg 30000

tccatccgca cccactatct tcatgttgtt gcagatgaag cgcgcaagac cgtctgaaga 30060

taccttcaac cccgtgtatc catatgacac ggaaaccggt cctccaactg tgccttttct 30120

tactcctccc tttgtatccc ccaatgggtt tcaagagagt ccccctgggg tactctcttt 30180

gcgcctatcc gaacctctag ttacctccaa tggcatgctt gcgctcaaaa tgggcaacgg 30240

cctctctctg gacgaggccg gcaaccttac ctcccaaaat gtaaccactg tgagcccacc 30300

tctcaaaaaa accaagtcaa acataaacct ggaaatatct gcacccctca cagttacctc 30360

agaagcccta actgtggctg ccgccgcacc tctaatggtc gcgggcaaca cactcaccat 30420

gcaatcacag gccccgctaa ccgtgcacga ctccaaactt agcattgcca cccaaggacc 30480

cctcacagtg tcagaaggaa agctagccct gcaaacatca ggccccctca ccaccaccga 30540

tagcagtacc cttactatca ctgcctcacc ccctctaact actgccactg gtagcttggg 30600

cattgacttg aaagagccca tttatacaca aaatggaaaa ctaggactaa agtacggggc 30660

tcctttgcat gtaacagacg acctaaacac tttgaccgta gcaactggtc caggtgtgac 30720

tattaataat acttccttgc aaactaaagt tactggagcc ttgggttttg attcacaagg 30780

caatatgcaa cttaatgtag caggaggact aaggattgat tctcaaaaca gacgccttat 30840

acttgatgtt agttatccgt ttgatgctca aaaccaacta aatctaagac taggacaggg 30900

ccctcttttt ataaactcag cccacaactt ggatattaac tacaacaaag gcctttactt 30960

gtttacagct tcaaacaatt ccaaaaagct tgaggttaac ctaagcactg ccaaggggtt 31020

gatgtttgac gctacagcca tagccattaa tgcaggagat gggcttgaat ttggttcacc 31080

taatgcacca aacacaaatc ccctcaaaac aaaaattggc catggcctag aatttgattc 31140

aaacaaggct atggttccta aactaggaac tggccttagt tttgacagca caggtgccat 31200

tacagtagga aacaaaaata atgataagct aaccctatgg acaggtccaa aaccagaagc 31260

caactgcata attgaatacg ggaaacaaaa cccagatagc aaactaactt taatccttgt 31320

aaaaaatgga ggaattgtta atggatatgt aacgctaatg ggagcctcag actacgttaa 31380

caccttattt aaaaacaaaa atgtctccat taatgtagaa ctatactttg atgccactgg 31440

tcatatatta ccagactcat cttctcttaa aacagatcta gaactaaaat acaagcaaac 31500

cgctgacttt agtgcaagag gttttatgcc aagtactaca gcgtatccat ttgtccttcc 31560

taatgcggga acacataatg aaaattatat ttttggtcaa tgctactaca aagcaagcga 31620

tggtgccctt tttccgttgg aagttactgt tatgcttaat aaacgcctgc cagatagtcg 31680

cacatcctat gttatgactt ttttatggtc cttgaatgct ggtctagctc cagaaactac 31740

tcaggcaacc ctcataacct ccccatttac cttttcctat attagagaag atgactaata 31800

aagaatcgtt tgtgactagt atgtagtctt atgcaatact cttgtagtct tgcaacatgg 31860

taacgatgag ttagcaacat gccttacaag gagagaaaaa gcaccgtgca tgccgattgg 31920

tggaagtaag gtggtacgat cgtgccttat taggaaggca acagacgggt ctgacatgga 31980

ttggacgaac cactgaattc cgcattgcag agatattgta tttaagtgcc tagctcgata 32040

caataaacgc catttgacca ttcaccacat tggtgtgcac ctgccgccac catggcagcc 32100

cgtctgctcc tcctgggcat ccttctcctg ctgctgcccc tgcccgtccc tgccccgtgc 32160

cacacagccg cacgctcaga gtgcaagcgc agccacaagt tcgtgcctgg tgcatggctg 32220

gccggggagg gtgtggacgt gaccagcctc cgccgctcgg gctccttccc agtggacaca 32280

caaaggttcc tgcggcccga cggcacctgc accctctgtg aaaatgccct acaggagggc 32340

accctccagc gcctgcctct ggcgctcacc aactggcggg cccagggctc tggctgccag 32400

cgccatgtaa ccagggccaa agtcagctcc actgaagctg tggcccggga tgcggctcgt 32460

agcatccgca acgactggaa ggtcgggctg gacgtgactc ctaagcccac cagcaatgtg 32520

catgtgtctg tggccggctc acactcacag gcagccaact ttgcagccca gaagacccac 32580

caggaccagt acagcttcag cactgacacg gtggagtgcc gcttctacag tttccatgtg 32640

gtacacactc ccccgctgca ccctgacttc aagagggccc tcggggacct gccccaccac 32700

ttcaacgcct ccacccagcc cgcctacctc aggcttatct ccaactacgg cacccacttc 32760

atccgggctg tggagctggg tggccgcata tcggccctca ctgccctgcg cacctgcgag 32820

ctggccctgg aagggctcac ggacaacgag gtggaggact gcctgactgt cgaggcccag 32880

gtcaacatag gcatccacgg cagcatctct gccgaagcca aggcctgtga ggagaagaag 32940

aagaagcaca agatgacggc ctccttccac caaacctacc gggagcgcca ttcggaagtg 33000

gttggcggcc atcacacctc cattaacgac ctgctgttcg ggatccaggc cgggcccgag 33060

cagtactcag cctgggtaaa ctcgctgccc ggcagccctg gcctggtgga ctacaccctg 33120

gaacccctgc acgtgctgct ggacagccag gacccgcggc gggaggcact gaggagggcc 33180

ctgagtcagt acctgacgga cagggctcgc tggagggact gcagccggcc gtgcccacca 33240

gggcggcaga agagcccccg agacccatgc cagtgtgtgt gccatggctc agcggtcacc 33300

acccaggact gctgccctcg gcagaggggc ctggcccagc tggaggtgac cttcatccaa 33360

gcatggggcc tgtgggggga ctggttcact gccacggatg cctatgtgaa gctcttcttt 33420

ggtggccagg agctgaggac gagcaccgtg tgggacaata acaaccccat ctggtcagtg 33480

cggctggatt ttggggatgt gctcctggcc acaggggggc ccctgaggtt gcaggtctgg 33540

gatcaggact ctggcaggga cgatgacctc cttggcacct gtgatcaggc tcccaagtct 33600

ggttcccatg aggtgagatg caacctgaat catggccacc taaaattccg ctatcatgcc 33660

aggtgcttgc cccacctggg aggaggcacc tgcctggact atgtccccca aatgcttctg 33720

ggggagcctc caggaaaccg gagtggggcc gtgtggtgac tgtgccttct agttgccagc 33780

catctgttgt ttgcccctcc cccgtgcctt ccttgaccct ggaaggtgcc actcccactg 33840

tcctttccta ataaaatgag gaaattgcat cgcattgtct gagtaggtgt cattctattc 33900

tggggggtgg ggtggggcag gacagcaagg gggaggattg ggaagacaat agcaggcatg 33960

ctggggatgc ggtgggctct atggactagt ggccaccatg gccttatgtt tcaacgtgtt 34020

tatttttcaa ttgcagaaaa tttcaagtca tttttcattc agtagtatag ccccaccacc 34080

acatagctta tacagatcac cgtaccttaa tcaaactcac agaaccctag tattcaacct 34140

gccacctccc tcccaacaca cagagtacac agtcctttct ccccggctgg ccttaaaaag 34200

catcatatca tgggtaacag acatattctt aggtgttata ttccacacgg tttcctgtcg 34260

agccaaacgc tcatcagtga tattaataaa ctccccgggc agctcactta agttcatgtc 34320

gctgtccagc tgctgagcca caggctgctg tccaacttgc ggttgcttaa cgggcggcga 34380

aggagaagtc cacgcctaca tgggggtaga gtcataatcg tgcatcagga tagggcggtg 34440

gtgctgcagc agcgcgcgaa taaactgctg ccgccgccgc tccgtcctgc aggaatacaa 34500

catggcagtg gtctcctcag cgatgattcg caccgcccgc agcataaggc gccttgtcct 34560

ccgggcacag cagcgcaccc tgatctcact taaatcagca cagtaactgc agcacagcac 34620

cacaatattg ttcaaaatcc cacagtgcaa ggcgctgtat ccaaagctca tggcggggac 34680

cacagaaccc acgtggccat cataccacaa gcgcaggtag attaagtggc gacccctcat 34740

aaacacgctg gacataaaca ttacctcttt tggcatgttg taattcacca cctcccggta 34800

ccatataaac ctctgattaa acatggcgcc atccaccacc atcctaaacc agctggccaa 34860

aacctgcccg ccggctatac actgcaggga accgggactg gaacaatgac agtggagagc 34920

ccaggactcg taaccatgga tcatcatgct cgtcatgata tcaatgttgg cacaacacag 34980

gcacacgtgc atacacttcc tcaggattac aagctcctcc cgcgttagaa ccatatccca 35040

gggaacaacc cattcctgaa tcagcgtaaa tcccacactg cagggaagac ctcgcacgta 35100

actcacgttg tgcattgtca aagtgttaca ttcgggcagc agcggatgat cctccagtat 35160

ggtagcgcgg gtttctgtct caaaaggagg tagacgatcc ctactgtacg gagtgcgccg 35220

agacaaccga gatcgtgttg gtcgtagtgt catgccaaat ggaacgccgg acgtagtcat 35280

atttcctgaa gcaaaaccag gtgcgggcgt gacaaacaga tctgcgtctc cggtctcgcc 35340

gcttagatcg ctctgtgtag tagttgtagt atatccactc tctcaaagca tccaggcgcc 35400

ccctggcttc gggttctatg taaactcctt catgcgccgc tgccctgata acatccacca 35460

ccgcagaata agccacaccc agccaaccta cacattcgtt ctgcgagtca cacacgggag 35520

gagcgggaag agctggaaga accatgtttt tttttttatt ccaaaagatt atccaaaacc 35580

tcaaaatgaa gatctattaa gtgaacgcgc tcccctccgg tggcgtggtc aaactctaca 35640

gccaaagaac agataatggc atttgtaaga tgttgcacaa tggcttccaa aaggcaaacg 35700

gccctcacgt ccaagtggac gtaaaggcta aacccttcag ggtgaatctc ctctataaac 35760

attccagcac cttcaaccat gcccaaataa ttctcatctc gccaccttct caatatatct 35820

ctaagcaaat cccgaatatt aagtccggcc attgtaaaaa tctgctccag agcgccctcc 35880

accttcagcc tcaagcagcg aatcatgatt gcaaaaattc aggttcctca cagacctgta 35940

taagattcaa aagcggaaca ttaacaaaaa taccgcgatc ccgtaggtcc cttcgcaggg 36000

ccagctgaac ataatcgtgc aggtctgcac ggaccagcgc ggccacttcc ccgccaggaa 36060

ccatgacaaa agaacccaca ctgattatga cacgcatact cggagctatg ctaaccagcg 36120

tagccccgat gtaagcttgt tgcatgggcg gcgatataaa atgcaaggtg ctgctcaaaa 36180

aatcaggcaa agcctcgcgc aaaaaagaaa gcacatcgta gtcatgctca tgcagataaa 36240

ggcaggtaag ctccggaacc accacagaaa aagacaccat ttttctctca aacatgtctg 36300

cgggtttctg cataaacaca aaataaaata acaaaaaaac atttaaacat tagaagcctg 36360

tcttacaaca ggaaaaacaa cccttataag cataagacgg actacggcca tgccggcgtg 36420

accgtaaaaa aactggtcac cgtgattaaa aagcaccacc gacagctcct cggtcatgtc 36480

cggagtcata atgtaagact cggtaaacac atcaggttga ttcacatcgg tcagtgctaa 36540

aaagcgaccg aaatagcccg ggggaataca tacccgcagg cgtagagaca acattacagc 36600

ccccatagga ggtataacaa aattaatagg agagaaaaac acataaacac ctgaaaaacc 36660

ctcctgccta ggcaaaatag caccctcccg ctccagaaca acatacagcg cttccacagc 36720

ggcagccata acagtcagcc ttaccagtaa aaaagaaaac ctattaaaaa aacaccactc 36780

gacacggcac cagctcaatc agtcacagtg taaaaaaggg ccaagtgcag agcgagtata 36840

tataggacta aaaaatgacg taacggttaa agtccacaaa aaacacccag aaaaccgcac 36900

gcgaacctac gcccagaaac gaaagccaaa aaacccacaa cttcctcaaa tcgtcacttc 36960

cgttttccca cgttacgtca cttcccattt taagaaaact acaattccca acacatacaa 37020

gttactccgc cctaaaacct acgtcacccg ccccgttccc acgccccgcg ccacgtcaca 37080

aactccaccc cctcattatc atattggctt caatccaaaa taaggtatat tattgatgat 37140

gttaat                                                             37146

<210> 2
<211> 37243
<212> DNA
<213> Artificial Sequence

<220>
<223> Ad5/3-D24-Perf-Grb (TGX-13)

<400> 2
attaacatca tcaataatat accttatttt ggattgaagc caatatgata atgagggggt      60

ggagtttgtg acgtggcgcg gggcgtggga acggggcggg tgacgtagta gtgtggcgga     120

agtgtgatgt tgcaagtgtg gcggaacaca tgtaagcgac ggatgtggca aaagtgacgt     180

ttttggtgtg cgccggtgta cacaggaagt gacaattttc gcgcggtttt aggcggatgt     240

tgtagtaaat ttgggcgtaa ccgagtaaga tttggccatt ttcgcgggaa aactgaataa     300

gaggaagtga aatctgaata attttgtgtt actcatagcg cgtaatattt gtctagggcc     360

gcggggactt tgaccgttta cgtggagact cgcccaggtg tttttctcag gtgttttccg     420

cgttccgggt caaagttggc gttttattat tatagtcagc tgacgtgtag tgtatttata     480

cccggtgagt tcctcaagag gccactcttg agtgccagcg agtagagttt tctcctccga     540

gccgctccga caccgggact gaaaatgaga catattatct gccacggagg tgttattacc     600

gaagaaatgg ccgccagtct tttggaccag ctgatcgaag aggtactggc tgataatctt     660

ccacctccta gccattttga accacctacc cttcacgaac tgtatgattt agacgtgacg     720

gcccccgaag atcccaacga ggaggcggtt tcgcagattt ttcccgactc tgtaatgttg     780

gcggtgcagg aagggattga cttactcact tttccgccgg cgcccggttc tccggagccg     840

cctcaccttt cccggcagcc cgagcagccg gagcagagag ccttgggtcc ggtttctatg     900

ccaaaccttg taccggaggt gatcgatcca cccagtgacg acgaggatga agagggtgag     960

gagtttgtgt tagattatgt ggagcacccc gggcacggtt gcaggtcttg tcattatcac    1020

cggaggaata cgggggaccc agatattatg tgttcgcttt gctatatgag gacctgtggc    1080

atgtttgtct acagtaagtg aaaattatgg gcagtgggtg atagagtggt gggtttggtg    1140

tggtaatttt ttttttaatt tttacagttt tgtggtttaa agaattttgt attgtgattt    1200

ttttaaaagg tcctgtgtct gaacctgagc ctgagcccga gccagaaccg gagcctgcaa    1260

gacctacccg ccgtcctaaa atggcgcctg ctatcctgag acgcccgaca tcacctgtgt    1320

ctagagaatg caatagtagt acggatagct gtgactccgg tccttctaac acacctcctg    1380

agatacaccc ggtggtcccg ctgtgcccca ttaaaccagt tgccgtgaga gttggtgggc    1440

gtcgccaggc tgtggaatgt atcgaggact tgcttaacga gcctgggcaa cctttggact    1500

tgagctgtaa acgccccagg ccataaggtg taaacctgtg attgcgtgtg tggttaacgc 1560

ctttgtttgc tgaatgagtt gatgtaagtt taataaaggg tgagataatg tttaacttgc 1620

atggcgtgtt aaatggggcg gggcttaaag ggtatataat gcgccgtggg ctaatcttgg 1680

ttacatctga cctcatggag gcttgggagt gtttggaaga tttttctgct gtgcgtaact 1740

tgctggaaca gagctctaac agtacctctt ggttttggag gtttctgtgg ggctcatccc 1800

aggcaaagtt agtctgcaga attaaggagg attacaagtg ggaatttgaa gagcttttga 1860

aatcctgtgg tgagctgttt gattctttga atctgggtca ccaggcgctt ttccaagaga 1920

aggtcatcaa gactttggat ttttccacac cggggcgcgc tgcggctgct gttgcttttt 1980

tgagttttat aaaggataaa tggagcgaag aaacccatct gagcgggggg tacctgctgg 2040

attttctggc catgcatctg tggagagcgg ttgtgagaca caagaatcgc ctgctactgt 2100

tgtcttccgt ccgcccggcg ataataccga cggaggagca gcagcagcag caggaggaag 2160

ccaggcggcg gcggcaggag cagagcccat ggaacccgag agccggcctg gaccctcggg 2220

aatgaatgtt gtacaggtgg ctgaactgta tccagaactg agacgcattt tgacaattac 2280

agaggatggg caggggctaa agggggtaaa gagggagcgg ggggcttgtg aggctacaga 2340

ggaggctagg aatctagctt ttagcttaat gaccagacac cgtcctgagt gtattacttt 2400

tcaacagatc aaggataatt gcgctaatga gcttgatctg ctggcgcaga agtattccat 2460

agagcagctg accacttact ggctgcagcc aggggatgat tttgaggagg ctattagggt 2520

atatgcaaag gtggcactta ggccagattg caagtacaag atcagcaaac ttgtaaatat 2580

caggaattgt tgctacattt ctgggaacgg ggccgaggtg gagatagata cggaggatag 2640

ggtggccttt agatgtagca tgataaatat gtggccgggg gtgcttggca tggacggggt 2700

ggttattatg aatgtaaggt ttactggccc caattttagc ggtacggttt tcctggccaa 2760

taccaacctt atcctacacg gtgtaagctt ctatgggttt aacaatacct gtgtggaagc 2820

ctggaccgat gtaaggttcc ggggctgtgc cttttactgc tgctggaagg gggtggtgtg 2880

tcgccccaaa agcagggctt caattaagaa atgcctcttt gaaaggtgta ccttgggtat 2940

cctgtctgag ggtaactcca gggtgcgcca caatgtggcc tccgactgtg gttgcttcat 3000

gctagtgaaa agcgtggctg tgattaagca taacatggta tgtggcaact gcgaggacag 3060

ggcctctcag atgctgacct gctcggacgg caactgtcac ctgctgaaga ccattcacgt 3120

agccagccac tctcgcaagg cctggccagt gtttgagcat aacatactga cccgctgttc 3180

cttgcatttg ggtaacagga ggggggtgtt cctaccttac caatgcaatt tgagtcacac 3240

taagatattg cttgagcccg agagcatgtc caaggtgaac ctgaacgggg tgtttgacat 3300

gaccatgaag atctggaagg tgctgaggta cgatgagacc cgcaccaggt gcagaccctg 3360

cgagtgtggc ggtaaacata ttaggaacca gcctgtgatg ctggatgtga ccgaggagct 3420

gaggcccgat cacttggtgc tggcctgcac ccgcgctgag tttggctcta gcgatgaaga 3480

tacagattga agtactgaaa tgtgtgggcg tggcttaagg gtgggaaaga atatataagg 3540

tgggggtctt atgtagtttt gtatctgttt tgcagcagcc gccgccgcca tgagcaccaa 3600

ctcgtttgat ggaagcattg tgagctcata tttgacaacg cgcatgcccc catgggccgg 3660

ggtgcgtcag aatgtgatgg gctccagcat tgatggtcgc cccgtcctgc ccgcaaactc 3720

tactaccttg acctacgaga ccgtgtctgg aacgccgttg gagactgcag cctccgccgc 3780

cgcttcagcc gctgcagcca ccgcccgcgg gattgtgact gactttgctt tcctgagccc 3840

gcttgcaagc agtgcagctt cccgttcatc cgcccgcgat gacaagttga cggctctttt 3900

ggcacaattg gattctttga cccgggaact taatgtcgtt tctcagcagc tgttggatct 3960

gcgccagcag gtttctgccc tgaaggcttc ctcccctccc aatgcggttt aacggtccgg 4020

gccagagtgg ccaacataaa taaaaaacca gactctgttt ggatttggat caagcaagtg 4080

tcttgctgtc tttatttagg ggttttgcgc gcgcggtagg cccgggacca gcggtctcgg 4140

tcgttgaggg tcctgtgtat tttttccagg acgtggtaaa ggtgactctg gatgttcaga 4200

tacatgggca taagcccgtc tctggggtgg aggtagcacc actgcagagc ttcatgctgc 4260

ggggtggtgt tgtagatgat ccagtcgtag caggagcgct gggcgtggtg cctaaaaatg 4320

tctttcagta gcaagctgat tgccaggggc aggcccttgg tgtaagtgtt tacaaagcgg 4380

ttaagctggg atgggtgcat acgtggggat atgagatgca tcttggactg tatttttagg 4440

ttggctatgt tcccagccat atccctccgg ggattcatgt tgtgcagaac caccagcaca 4500

gtgtatccgg tgcacttggg aaatttgtca tgtagcttag aaggaaatgc gtggaagaac 4560

ttggagacgc ccttgtgacc tccaagattt tccatgcatt cgtccataat gatggcaatg 4620

ggcccacggg cggcggcctg ggcgaagata tttctgggat cactaacgtc atagttgtgt 4680

tccaggatga gatcgtcata ggccattttt acaaagcgcg ggcggagggt gccagactgc 4740

ggtataatgg ttccatccgg cccaggggcg tagttaccct cacagatttg catttcccac 4800

gctttgagtt cagatggggg gatcatgtct acctgcgggg cgatgaagaa aacggtttcc 4860

ggggtagggg agatcagctg ggaagaaagc aggttcctga gcagctgcga cttaccgcag 4920

ccggtgggcc cgtaaatcac acctattacc ggctgcaact ggtagttaag agagctgcag 4980

ctgccgtcat ccctgagcag gggggccact tcgttaagca tgtccctgac tcgcatgttt 5040

tccctgacca aatccgccag aaggcgctcg ccgcccagcg atagcagttc ttgcaaggaa 5100

gcaaagtttt tcaacggttt gagaccgtcc gccgtaggca tgcttttgag cgtttgacca 5160

agcagttcca ggcggtccca cagctcggtc acctgctcta cggcatctcg atccagcata 5220

tctcctcgtt tcgcgggttg gggcggcttt cgctgtacgg cagtagtcgg tgctcgtcca 5280

gacgggccag ggtcatgtct ttccacgggc gcagggtcct cgtcagcgta gtctgggtca 5340

cggtgaaggg gtgcgctccg ggctgcgcgc tggccagggt gcgcttgagg ctggtcctgc 5400

tggtgctgaa gcgctgccgg tcttcgccct gcgcgtcggc caggtagcat ttgaccatgg 5460

tgtcatagtc cagcccctcc gcggcgtggc ccttggcgcg cagcttgccc ttggaggagg 5520

cgccgcacga ggggcagtgc agacttttga gggcgtagag cttgggcgcg agaaataccg 5580

attccgggga gtaggcatcc gcgccgcagg ccccgcagac ggtctcgcat tccacgagcc 5640

aggtgagctc tggccgttcg gggtcaaaaa ccaggtttcc cccatgcttt ttgatgcgtt 5700

tcttacctct ggtttccatg agccggtgtc cacgctcggt gacgaaaagg ctgtccgtgt 5760

ccccgtatac agacttgaga ggcctgtcct cgagcggtgt tccgcggtcc tcctcgtata 5820

gaaactcgga ccactctgag acaaaggctc gcgtccaggc cagcacgaag gaggctaagt 5880

gggaggggta gcggtcgttg tccactaggg ggtccactcg ctccagggtg tgaagacaca 5940

tgtcgccctc ttcggcatca aggaaggtga ttggtttgta ggtgtaggcc acgtgaccgg 6000

gtgttcctga aggggggcta taaaaggggg tgggggcgcg ttcgtcctca ctctcttccg 6060

catcgctgtc tgcgagggcc agctgttggg gtgagtactc cctctgaaaa gcgggcatga 6120

cttctgcgct aagattgtca gtttccaaaa acgaggagga tttgatattc acctggcccg 6180

cggtgatgcc tttgagggtg gccgcatcca tctggtcaga aaagacaatc tttttgttgt 6240

caagcttggt ggcaaacgac ccgtagaggg cgttggacag caacttggcg atggagcgca 6300

gggtttggtt tttgtcgcga tcggcgcgct ccttggccgc gatgtttagc tgcacgtatt 6360

cgcgcgcaac gcaccgccat tcgggaaaga cggtggtgcg ctcgtcgggc accaggtgca 6420

cgcgccaacc gcggttgtgc agggtgacaa ggtcaacgct ggtggctacc tctccgcgta 6480

ggcgctcgtt ggtccagcag aggcggccgc ccttgcgcga gcagaatggc ggtagggggt 6540

ctagctgcgt ctcgtccggg gggtctgcgt ccacggtaaa gaccccgggc agcaggcgcg 6600

cgtcgaagta gtctatcttg catccttgca agtctagcgc ctgctgccat gcgcgggcgg 6660

caagcgcgcg ctcgtatggg ttgagtgggg gaccccatgg catggggtgg gtgagcgcgg 6720

aggcgtacat gccgcaaatg tcgtaaacgt agaggggctc tctgagtatt ccaagatatg 6780

tagggtagca tcttccaccg cggatgctgg cgcgcacgta atcgtatagt tcgtgcgagg 6840

gagcgaggag gtcgggaccg aggttgctac gggcgggctg ctctgctcgg aagactatct 6900

gcctgaagat ggcatgtgag ttggatgata tggttggacg ctggaagacg ttgaagctgg 6960

cgtctgtgag acctaccgcg tcacgcacga aggaggcgta ggagtcgcgc agcttgttga 7020

ccagctcggc ggtgacctgc acgtctaggg cgcagtagtc cagggtttcc ttgatgatgt 7080

catacttatc ctgtcccttt tttttccaca gctcgcggtt gaggacaaac tcttcgcggt 7140

ctttccagta ctcttggatc ggaaacccgt cggcctccga acggtaagag cctagcatgt 7200

agaactggtt gacggcctgg taggcgcagc atcccttttc tacgggtagc gcgtatgcct 7260

gcgcggcctt ccggagcgag gtgtgggtga gcgcaaaggt gtccctgacc atgactttga 7320

ggtactggta tttgaagtca gtgtcgtcgc atccgccctg ctcccagagc aaaaagtccg 7380

tgcgcttttt ggaacgcgga tttggcaggg cgaaggtgac atcgttgaag agtatctttc 7440

ccgcgcgagg cataaagttg cgtgtgatgc ggaagggtcc cggcacctcg gaacggttgt 7500

taattacctg ggcggcgagc acgatctcgt caaagccgtt gatgttgtgg cccacaatgt 7560

aaagttccaa gaagcgcggg atgcccttga tggaaggcaa ttttttaagt tcctcgtagg 7620

tgagctcttc aggggagctg agcccgtgct ctgaaagggc ccagtctgca agatgagggt 7680

tggaagcgac gaatgagctc cacaggtcac gggccattag catttgcagg tggtcgcgaa 7740

aggtcctaaa ctggcgacct atggccattt tttctggggt gatgcagtag aaggtaagcg 7800

ggtcttgttc ccagcggtcc catccaaggt tcgcggctag gtctcgcgcg gcagtcacta 7860

gaggctcatc tccgccgaac ttcatgacca gcatgaaggg cacgagctgc ttcccaaagg 7920

cccccatcca agtataggtc tctacatcgt aggtgacaaa gagacgctcg gtgcgaggat 7980

gcgagccgat cgggaagaac tggatctccc gccaccaatt ggaggagtgg ctattgatgt 8040

ggtgaaagta gaagtccctg cgacgggccg aacactcgtg ctggcttttg taaaaacgtg 8100

cgcagtactg gcagcggtgc acgggctgta catcctgcac gaggttgacc tgacgaccgc 8160

gcacaaggaa gcagagtggg aatttgagcc cctcgcctgg cgggtttggc tggtggtctt 8220

ctacttcggc tgcttgtcct tgaccgtctg gctgctcgag gggagttacg gtggatcgga 8280

ccaccacgcc gcgcgagccc aaagtccaga tgtccgcgcg cggcggtcgg agcttgatga 8340

caacatcgcg cagatgggag ctgtccatgg tctggagctc ccgcggcgtc aggtcaggcg 8400

ggagctcctg caggtttacc tcgcatagac gggtcagggc gcgggctaga tccaggtgat 8460

acctaatttc caggggctgg ttggtggcgg cgtcgatggc ttgcaagagg ccgcatcccc 8520

gcggcgcgac tacggtaccg cgcggcgggc ggtgggccgc gggggtgtcc ttggatgatg 8580

catctaaaag cggtgacgcg ggcgagcccc cggaggtagg gggggctccg gacccgccgg 8640

gagagggggc aggggcacgt cggcgccgcg cgcgggcagg agctggtgct gcgcgcgtag 8700

gttgctggcg aacgcgacga cgcggcggtt gatctcctga atctggcgcc tctgcgtgaa 8760

gacgacgggc ccggtgagct tgaacctgaa agagagttcg acagaatcaa tttcggtgtc 8820

gttgacggcg gcctggcgca aaatctcctg cacgtctcct gagttgtctt gataggcgat 8880

ctcggccatg aactgctcga tctcttcctc ctggagatct ccgcgtccgg ctcgctccac 8940

ggtggcggcg aggtcgttgg aaatgcgggc catgagctgc gagaaggcgt tgaggcctcc 9000

ctcgttccag acgcggctgt agaccacgcc cccttcggca tcgcgggcgc gcatgaccac 9060

ctgcgcgaga ttgagctcca cgtgccgggc gaagacggcg tagtttcgca ggcgctgaaa 9120

gaggtagttg agggtggtgg cggtgtgttc tgccacgaag aagtacataa cccagcgtcg 9180

caacgtggat tcgttgatat cccccaaggc ctcaaggcgc tccatggcct cgtagaagtc 9240

cacggcgaag ttgaaaaact gggagttgcg cgccgacacg gttaactcct cctccagaag 9300

acggatgagc tcggcgacag tgtcgcgcac ctcgcgctca aaggctacag gggcctcttc 9360

ttcttcttca atctcctctt ccataagggc ctccccttct tcttcttctg gcggcggtgg 9420

gggagggggg acacggcggc gacgacggcg caccgggagg cggtcgacaa agcgctcgat 9480

catctccccg cggcgacggc gcatggtctc ggtgacggcg cggccgttct cgcgggggcg 9540

cagttggaag acgccgcccg tcatgtcccg gttatgggtt ggcggggggc tgccatgcgg 9600

cagggatacg gcgctaacga tgcatctcaa caattgttgt gtaggtactc cgccgccgag 9660

ggacctgagc gagtccgcat cgaccggatc ggaaaacctc tcgagaaagg cgtctaacca 9720

gtcacagtcg caaggtaggc tgagcaccgt ggcgggcggc agcgggcggc ggtcggggtt 9780

gtttctggcg gaggtgctgc tgatgatgta attaaagtag gcggtcttga gacggcggat 9840

ggtcgacaga agcaccatgt ccttgggtcc ggcctgctga atgcgcaggc ggtcggccat 9900

gccccaggct tcgttttgac atcggcgcag gtctttgtag tagtcttgca tgagcctttc 9960

taccggcact tcttcttctc cttcctcttg tcctgcatct cttgcatcta tcgctgcggc 10020

ggcggcggag tttggccgta ggtggcgccc tcttcctccc atgcgtgtga ccccgaagcc 10080

cctcatcggc tgaagcaggg ctaggtcggc gacaacgcgc tcggctaata tggcctgctg 10140

cacctgcgtg agggtagact ggaagtcatc catgtccaca aagcggtggt atgcgcccgt 10200

gttgatggtg taagtgcagt tggccataac ggaccagtta acggtctggt gacccggctg 10260

cgagagctcg gtgtacctga gacgcgagta agccctcgag tcaaatacgt agtcgttgca 10320

agtccgcacc aggtactggt atcccaccaa aaagtgcggc ggcggctggc ggtagagggg 10380

ccagcgtagg gtggccgggg ctccgggggc gagatcttcc aacataaggc gatgatatcc 10440

gtagatgtac ctggacatcc aggtgatgcc ggcggcggtg gtggaggcgc gcggaaagtc 10500

gcggacgcgg ttccagatgt tgcgcagcgg caaaaagtgc tccatggtcg ggacgctctg 10560

gccggtcagg cgcgcgcaat cgttgacgct ctagaccgtg caaaaggaga gcctgtaagc 10620

gggcactctt ccgtggtctg gtggataaat tcgcaagggt atcatggcgg acgaccgggg 10680

ttcgagcccc gtatccggcc gtccgccgtg atccatgcgg ttaccgcccg cgtgtcgaac 10740

ccaggtgtgc gacgtcagac aacgggggag tgctcctttt ggcttccttc caggcgcggc 10800

ggctgctgcg ctagcttttt tggccactgg ccgcgcgcag cgtaagcggt taggctggaa 10860

agcgaaagca ttaagtggct cgctccctgt agccggaggg ttattttcca agggttgagt 10920

cgcgggaccc ccggttcgag tctcggaccg gccggactgc ggcgaacggg ggtttgcctc 10980

cccgtcatgc aagaccccgc ttgcaaattc ctccggaaac agggacgagc cccttttttg 11040

cttttcccag atgcatccgg tgctgcggca gatgcgcccc cctcctcagc agcggcaaga 11100

gcaagagcag cggcagacat gcagggcacc ctcccctcct cctaccgcgt caggaggggc 11160

gacatccgcg gttgacgcgg cagcagatgg tgattacgaa cccccgcggc gccgggcccg 11220

gcactacctg gacttggagg agggcgaggg cctggcgcgg ctaggagcgc cctctcctga 11280

gcggcaccca agggtgcagc tgaagcgtga tacgcgtgag gcgtacgtgc cgcggcagaa 11340

cctgtttcgc gaccgcgagg gagaggagcc cgaggagatg cgggatcgaa agttccacgc 11400

agggcgcgag ctgcggcatg gcctgaatcg cgagcggttg ctgcgcgagg aggactttga 11460

gcccgacgcg cgaaccggga ttagtcccgc gcgcgcacac gtggcggccg ccgacctggt 11520

aaccgcatac gagcagacgg tgaaccagga gattaacttt caaaaaagct ttaacaacca 11580

cgtgcgtacg cttgtggcgc gcgaggaggt ggctatagga ctgatgcatc tgtgggactt 11640

tgtaagcgcg ctggagcaaa acccaaatag caagccgctc atggcgcagc tgttccttat 11700

agtgcagcac agcagggaca acgaggcatt cagggatgcg ctgctaaaca tagtagagcc 11760

cgagggccgc tggctgctcg atttgataaa catcctgcag agcatagtgg tgcaggagcg 11820

cagcttgagc ctggctgaca aggtggccgc catcaactat tccatgctta gcctgggcaa 11880

gttttacgcc cgcaagatat accatacccc ttacgttccc atagacaagg aggtaaagat 11940

cgaggggttc tacatgcgca tggcgctgaa ggtgcttacc ttgagcgacg acctgggcgt 12000

ttatcgcaac gagcgcatcc acaaggccgt gagcgtgagc cggcggcgcg agctcagcga 12060

ccgcgagctg atgcacagcc tgcaaagggc cctggctggc acgggcagcg gcgatagaga 12120

ggccgagtcc tactttgacg cgggcgctga cctgcgctgg gccccaagcc gacgcgccct 12180

ggaggcagct ggggccggac ctgggctggc ggtggcaccc gcgcgcgctg gcaacgtcgg 12240

cggcgtggag gaatatgacg aggacgatga gtacgagcca gaggacggcg agtactaagc 12300

ggtgatgttt ctgatcagat gatgcaagac gcaacggacc cggcggtgcg ggcggcgctg 12360

cagagccagc cgtccggcct taactccacg gacgactggc gccaggtcat ggaccgcatc 12420

atgtcgctga ctgcgcgcaa tcctgacgcg ttccggcagc agccgcaggc caaccggctc 12480

tccgcaattc tggaagcggt ggtcccggcg cgcgcaaacc ccacgcacga gaaggtgctg 12540

gcgatcgtaa acgcgctggc cgaaaacagg gccatccggc ccgacgaggc cggcctggtc 12600

tacgacgcgc tgcttcagcg cgtggctcgt tacaacagcg gcaacgtgca gaccaacctg 12660

gaccggctgg tgggggatgt gcgcgaggcc gtggcgcagc gtgagcgcgc gcagcagcag 12720

ggcaacctgg gctccatggt tgcactaaac gccttcctga gtacacagcc cgccaacgtg 12780

ccgcggggac aggaggacta caccaacttt gtgagcgcac tgcggctaat ggtgactgag 12840

acaccgcaaa gtgaggtgta ccagtctggg ccagactatt ttttccagac cagtagacaa 12900
ggcctgcaga ccgtaaacct gagccaggct ttcaaaaact tgcaggggct gtggggggtg 12960
cgggctccca caggcgaccg cgcgaccgtg tctagcttgc tgacgcccaa ctcgcgcctg 13020
ttgctgctgc taatagcgcc cttcacggac agtggcagcg tgtcccggga cacataccta 13080
ggtcacttgc tgacactgta ccgcgaggcc ataggtcagg cgcatgtgga cgagcatact 13140
ttccaggaga ttacaagtgt cagccgcgcg ctggggcagg aggacacggg cagcctggag 13200
gcaaccctaa actacctgct gaccaaccgg cggcagaaga tcccctcgtt gcacagttta 13260
aacagcgagg aggagcgcat tttgcgctac gtgcagcaga gcgtgagcct taacctgatg 13320
cgcgacgggg taacgcccag cgtggcgctg gacatgaccg cgcgcaacat ggaaccgggc 13380
atgtatgcct caaaccggcc gtttatcaac cgcctaatgg actacttgca tcgcgcggcc 13440
gccgtgaacc ccgagtattt caccaatgcc atcttgaacc cgcactggct accgccccct 13500
ggtttctaca ccgggggatt cgaggtgccc gagggtaacg atggattcct ctgggacgac 13560
atagacgaca gcgtgttttc cccgcaaccg cagaccctgc tagagttgca acagcgcgag 13620
caggcagagg cggcgctgcg aaaggaaagc ttccgcaggc caagcagctt gtccgatcta 13680
ggcgctgcgg ccccgcggtc agatgctagt agcccatttc caagcttgat agggtctctt 13740
accagcactc gcaccacccg cccgcgcctg ctgggcgagg aggagtacct aaacaactcg 13800
ctgctgcagc cgcagcgcga aaaaaacctg cctccggcat ttcccaacaa cgggatagag 13860
agcctagtgg acaagatgag tagatggaag acgtacgcgc aggagcacag ggacgtgcca 13920
ggcccgcgcc cgcccacccg tcgtcaaagg cacgaccgtc agcggggtct ggtgtgggag 13980
gacgatgact cggcagacga cagcagcgtc ctggatttgg gagggagtgg caacccgttt 14040
gcgcaccttc gccccaggct ggggagaatg ttttaaaaaa aaaaaaaagc atgatgcaaa 14100
ataaaaaact caccaaggcc atggcaccga gcgttggttt tcttgtattc cccttagtat 14160
gcggcgcgcg gcgatgtatg aggaaggtcc tcctccctcc tacgagagtg tggtgagcgc 14220
ggcgccagtg gcggcggcgc tgggttctcc cttcgatgct cccctggacc cgccgtttgt 14280
gcctccgcgg tacctgcggc ctaccggggg gagaaacagc atccgttact ctgagttggc 14340
acccctattc gacaccaccc gtgtgtacct ggtggacaac aagtcaacgg atgtggcatc 14400
cctgaactac cagaacgacc acagcaactt tctgaccacg gtcattcaaa acaatgacta 14460
cagcccgggg gaggcaagca cacagaccat caatcttgac gaccggtcgc actggggcgg 14520
cgacctgaaa accatcctgc ataccaacat gccaaatgtg aacgagttca tgtttaccaa 14580
taagtttaag gcgcgggtga tggtgtcgcg cttgcctact aaggacaatc aggtggagct 14640
gaaatacgag tgggtggagt tcacgctgcc cgagggcaac tactccgaga ccatgaccat 14700
agaccttatg aacaacgcga tcgtggagca ctacttgaaa gtgggcagac agaacggggt 14760

tctggaaagc gacatcgggg taaagtttga cacccgcaac ttcagactgg ggtttgaccc 14820

cgtcactggt cttgtcatgc ctggggtata tacaaacgaa gccttccatc cagacatcat 14880

tttgctgcca ggatgcgggg tggacttcac ccacagccgc ctgagcaact tgttgggcat 14940

ccgcaagcgg caacccttcc aggagggctt taggatcacc tacgatgatc tggagggtgg 15000

taacattccc gcactgttgg atgtggacgc ctaccaggcg agcttgaaag atgacaccga 15060

acagggcggg ggtggcgcag gcggcagcaa cagcagtggc agcggcgcgg aagagaactc 15120

caacgcggca gccgcggcaa tgcagccggt ggaggacatg aacgatcatg ccattcgcgg 15180

cgacaccttt gccacacggg ctgaggagaa gcgcgctgag gccgaagcag cggccgaagc 15240

tgccgccccc gctgcgcaac ccgaggtcga gaagcctcag aagaaaccgg tgatcaaacc 15300

cctgacagag gacagcaaga aacgcagtta caacctaata agcaatgaca gcaccttcac 15360

ccagtaccgc agctggtacc ttgcatacaa ctacggcgac cctcagaccg gaatccgctc 15420

atggaccctg ctttgcactc ctgacgtaac ctgcggctcg gagcaggtct actggtcgtt 15480

gccagacatg atgcaagacc ccgtgacctt ccgctccacg cgccagatca gcaactttcc 15540

ggtggtgggc gccgagctgt tgcccgtgca ctccaagagc ttctacaacg accaggccgt 15600

ctactcccaa ctcatccgcc agtttacctc tctgacccac gtgttcaatc gctttcccga 15660

gaaccagatt ttggcgcgcc cgccagcccc caccatcacc accgtcagtg aaaacgttcc 15720

tgctctcaca gatcacggga cgctaccgct gcgcaacagc atcggaggag tccagcgagt 15780

gaccattact gacgccagac gccgcacctg cccctacgtt tacaaggccc tgggcatagt 15840

ctcgccgcgc gtcctatcga gccgcacttt ttgagcaagc atgtccatcc ttatatcgcc 15900

cagcaataac acaggctggg gcctgcgctt cccaagcaag atgtttggcg gggccaagaa 15960

gcgctccgac caacacccag tgcgcgtgcg cgggcactac cgcgcgccct ggggcgcgca 16020

caaacgcggc cgcactgggc gcaccaccgt cgatgacgcc atcgacgcgg tggtggagga 16080

ggcgcgcaac tacacgccca cgccgccacc agtgtccaca gtggacgcgg ccattcagac 16140

cgtggtgcgc ggagcccggc gctatgctaa aatgaagaga cggcggaggc gcgtagcacg 16200

tcgccaccgc cgccgacccg gcactgccgc ccaacgcgcg gcggcggccc tgcttaaccg 16260

cgcacgtcgc accggccgac gggcggccat gcgagcagct cgaaggctgg ccgcgggtat 16320

tgtcactgtg ccccccaggt ccaggcgacg agcggccgcc gcagcagccg cggccattag 16380

tgctatgact cagggtcgca ggggcaacgt gtattgggtg cgcgactcgg ttagcggcct 16440

gcgcgtgccc gtgcgcaccc gccccccgcg caactagatt gcaagaaaaa actacttaga 16500

ctcgtactgt tgtatgtatc cagcggcggc ggcgcgcaac gaagctatgt ccaagcgcaa 16560

aatcaaagaa gagatgctcc aggtcatcgc gccggagatc tatggccccc cgaagaagga 16620

agagcaggat tacaagcccc gaaagctaaa gcgggtcaaa aagaaaaaga aagatgatga 16680

tgatgaactt gacgacgagg tggaactgct gcacgctacc gcgcccaggc gacgggtaca 16740

gtggaaaggt cgacgcgtaa aacgtgtttt gcgacccggc accaccgtag tctttacgcc 16800

cggtgagcgc tccacccgca cctacaagcg cgtgtatgat gaggtgtacg gcgacgagga 16860

cctgcttgag caggccaacg agcgcctcgg ggagtttgcc tacggaaagc ggcataagga 16920

catgctggcg ttgccgctgg acgagggcaa cccaacacct agcctaaagc ccgtaacact 16980

gcagcaggtg ctgcccgcgc ttgcaccgtc cgaagaaaag cgcggcctaa agcgcgagtc 17040

tggtgacttg gcacccaccg tgcagctgat ggtacccaag cgccagcgac tggaagatgt 17100

cttggaaaaa atgaccgtgg aacctgggct ggagcccgag gtccgcgtgc ggccaatcaa 17160

gcaggtggcg ccgggactgg gcgtgcagac cgtggacgtt cagataccca ctaccagtag 17220

caccagtatt gccaccgcca cagagggcat ggagacacaa acgtccccgg ttgcctcagc 17280

ggtggcggat gccgcggtgc aggcggtcgc tgcggccgcg tccaagacct ctacggaggt 17340

gcaaacggac ccgtggatgt ttcgcgtttc agccccccgg cgcccgcgcc gttcgaggaa 17400

gtacggcgcc gccagcgcgc tactgcccga atatgcccta catccttcca ttgcgcctac 17460

ccccggctat cgtggctaca cctaccgccc cagaagacga gcaactaccc gacgccgaac 17520

caccactgga acccgccgcc gccgtcgccg tcgccagccc gtgctggccc cgatttccgt 17580

gcgcagggtg gctcgcgaag gaggcaggac cctggtgctg ccaacagcgc gctaccaccc 17640

cagcatcgtt taaaagccgg tctttgtggt tcttgcagat atggccctca cctgccgcct 17700

ccgtttcccg gtgccgggat tccgaggaag aatgcaccgt aggaggggca tggccggcca 17760

cggcctgacg ggcggcatgc gtcgtgcgca ccaccggcgg cggcgcgcgt cgcaccgtcg 17820

catgcgcggc ggtatcctgc ccctccttat tccactgatc gccgcggcga ttggcgccgt 17880

gcccggaatt gcatccgtgg ccttgcaggc gcagagacac tgattaaaaa caagttgcat 17940

gtggaaaaat caaaataaaa agtctggact ctcacgctcg cttggtcctg taactatttt 18000

gtagaatgga agacatcaac tttgcgtctc tggccccgcg acacggctcg cgcccgttca 18060

tgggaaactg gcaagatatc ggcaccagca atatgagcgg tggcgccttc agctggggct 18120

cgctgtggag cggcattaaa aatttcggtt ccaccgttaa gaactatggc agcaaggcct 18180

ggaacagcag cacaggccag atgctgaggg ataagttgaa agagcaaaat ttccaacaaa 18240

aggtggtaga tggcctggcc tctggcatta gcggggtggt ggacctggcc aaccaggcag 18300

tgcaaaataa gattaacagt aagcttgatc cccgccctcc cgtagaggag cctccaccgg 18360

ccgtggagac agtgtctcca gaggggcgtg gcgaaaagcg tccgcgcccc gacagggaag 18420

aaactctggt gacgcaaata gacgagcctc cctcgtacga ggaggcacta aagcaaggcc 18480

tgcccaccac ccgtcccatc gcgcccatgg ctaccggagt gctgggccag cacacacccg 18540

taacgctgga cctgcctccc cccgccgaca cccagcagaa acctgtgctg ccaggcccga 18600

ccgccgttgt tgtaacccgt cctagccgcg cgtccctgcg ccgcgccgcc agcggtccgc 18660

gatcgttgcg gcccgtagcc agtggcaact ggcaaagcac actgaacagc atcgtgggtc 18720

tgggggtgca atccctgaag cgccgacgat gcttctgata gctaacgtgt cgtatgtgtg 18780

tcatgtatgc gtccatgtcg ccgccagagg agctgctgag ccgccgcgcg cccgctttcc 18840

aagatggcta ccccttcgat gatgccgcag tggtcttaca tgcacatctc gggccaggac 18900

gcctcggagt acctgagccc cgggctggtg cagtttgccc gcgccaccga gacgtacttc 18960

agcctgaata acaagtttag aaaccccacg gtggcgccta cgcacgacgt gaccacagac 19020

cggtcccagc gtttgacgct gcggttcatc cctgtggacc gtgaggatac tgcgtactcg 19080

tacaaggcgc ggttcaccct agctgtgggt gataaccgtg tgctggacat ggcttccacg 19140

tactttgaca tccgcggcgt gctggacagg ggccctactt ttaagcccta ctctggcact 19200

gcctacaacg ccctggctcc caagggtgcc ccaaatcctt gcgaatggga tgaagctgct 19260

actgctcttg aaataaacct agaagaagag gacgatgaca acgaagacga agtagacgag 19320

caagctgagc agcaaaaaac tcacgtattt gggcaggcgc cttattctgg tataaatatt 19380

acaaaggagg gtattcaaat aggtgtcgaa ggtcaaacac ctaaatatgc cgataaaaca 19440

tttcaacctg aacctcaaat aggagaatct cagtggtacg aaacagaaat taatcatgca 19500

gctgggagag tcctaaaaaa gactacccca atgaaaccat gttacggttc atatgcaaaa 19560

cccacaaatg aaaatggagg gcaaggcatt cttgtaaagc aacaaaatgg aaagctagaa 19620

agtcaagtgg aaatgcaatt tttctcaact actgaggcag ccgcaggcaa tggtgataac 19680

ttgactccta aagtggtatt gtacagtgaa gatgtagata tagaaacccc agacactcat 19740

atttcttaca tgcccactat taaggaaggt aactcacgag aactaatggg ccaacaatct 19800

atgcccaaca ggcctaatta cattgctttt agggacaatt ttattggtct aatgtattac 19860

aacagcacgg gtaatatggg tgttctggcg ggccaagcat cgcagttgaa tgctgttgta 19920

gatttgcaag acagaaacac agagctttca taccagcttt tgcttgattc cattggtgat 19980

agaaccaggt acttttctat gtggaatcag gctgttgaca gctatgatcc agatgttaga 20040

attattgaaa atcatggaac tgaagatgaa cttccaaatt actgctttcc actgggaggt 20100

gtgattaata cagagactct taccaaggta aaacctaaaa caggtcagga aaatggatgg 20160

gaaaaagatg ctacagaatt ttcagataaa aatgaaataa gagttggaaa taattttgcc 20220

atggaaatca atctaaatgc caacctgtgg agaaatttcc tgtactccaa catagcgctg 20280

tatttgcccg acaagctaaa gtacagtcct tccaacgtaa aaatttctga taacccaaac 20340

acctacgact acatgaacaa gcgagtggtg gctcccgggc tagtggactg ctacattaac 20400

cttggagcac gctggtccct tgactatatg gacaacgtca acccatttaa ccaccaccgc 20460
aatgctggcc tgcgctaccg ctcaatgttg ctgggcaatg gtcgctatgt gcccttccac 20520
atccaggtgc ctcagaagtt ctttgccatt aaaaacctcc ttctcctgcc gggctcatac 20580
acctacgagt ggaacttcag gaaggatgtt aacatggttc tgcagagctc cctaggaaat 20640
gacctaaggg ttgacggagc cagcattaag tttgatagca tttgccttta cgccaccttc 20700
ttccccatgg cccacaacac cgcctccacg cttgaggcca tgcttagaaa cgacaccaac 20760
gaccagtcct ttaacgacta tctctccgcc gccaacatgc tctaccctat acccgccaac 20820
gctaccaacg tgcccatatc catcccctcc cgcaactggg cggctttccg cggctgggcc 20880
ttcacgcgcc ttaagactaa ggaaacccca tcactgggct cgggctacga cccttattac 20940
acctactctg gctctatacc ctacctagat ggaacctttt acctcaacca cacctttaag 21000
aaggtggcca ttacctttga ctcttctgtc agctggcctg gcaatgaccg cctgcttacc 21060
cccaacgagt ttgaaattaa gcgctcagtt gacggggagg gttacaacgt tgcccagtgt 21120
aacatgacca aagactggtt cctggtacaa atgctagcta actataacat tggctaccag 21180
ggcttctata tcccagagag ctacaaggac cgcatgtact ccttctttag aaacttccag 21240
cccatgagcc gtcaggtggt ggatgatact aaatacaagg actaccaaca ggtgggcatc 21300
ctacaccaac acaacaactc tggatttgtt ggctaccttg cccccaccat gcgcgaagga 21360
caggcctacc ctgctaactt cccctatccg cttataggca agaccgcagt tgacagcatt 21420
acccagaaaa agtttctttg cgatcgcacc ctttggcgca tcccattctc cagtaacttt 21480
atgtccatgg gcgcactcac agacctgggc caaaaccttc tctacgccaa ctccgcccac 21540
gcgctagaca tgacttttga ggtggatccc atggacgagc ccacccttct ttatgttttg 21600
tttgaagtct ttgacgtggt ccgtgtgcac cagccgcacc gcggcgtcat cgaaaccgtg 21660
tacctgcgca cgcccttctc ggccggcaac gccacaacat aaagaagcaa gcaacatcaa 21720
caacagctgc cgccatgggc tccagtgagc aggaactgaa agccattgtc aaagatcttg 21780
gttgtgggcc atattttttg ggcacctatg acaagcgctt tccaggcttt gtttctccac 21840
acaagctcgc ctgcgccata gtcaatacgg ccggtcgcga gactgggggc gtacactgga 21900
tggcctttgc ctggaacccg cactcaaaaa catgctacct ctttgagccc tttggctttt 21960
ctgaccagcg actcaagcag gtttaccagt ttgagtacga gtcactcctg cgccgtagcg 22020
ccattgcttc ttcccccgac cgctgtataa cgctggaaaa gtccacccaa agcgtacagg 22080
ggcccaactc ggccgcctgt ggactattct gctgcatgtt tctccacgcc tttgccaact 22140
ggccccaaac tcccatggat cacaacccca ccatgaacct tattaccggg gtacccaact 22200
ccatgctcaa cagtccccag gtacagccca ccctgcgtcg caaccaggaa cagctctaca 22260
gcttcctgga gcgccactcg ccctacttcc gcagccacag tgcgcagatt aggagcgcca 22320

cttctttttg tcacttgaaa aacatgtaaa aataatgtac tagagacact ttcaataaag 22380

gcaaatgctt ttatttgtac actctcgggt gattatttac ccccaccctt gccgtctgcg 22440

ccgtttaaaa atcaaagggg ttctgccgcg catcgctatg cgccactggc agggacacgt 22500

tgcgatactg gtgtttagtg ctccacttaa actcaggcac aaccatccgc ggcagctcgg 22560

tgaagttttc actccacagg ctgcgcacca tcaccaacgc gtttagcagg tcgggcgccg 22620

atatcttgaa gtcgcagttg gggcctccgc cctgcgcgcg cgagttgcga tacacagggt 22680

tgcagcactg gaacactatc agcgccgggt ggtgcacgct ggccagcacg ctcttgtcgg 22740

agatcagatc cgcgtccagg tcctccgcgt tgctcagggc gaacggagtc aactttggta 22800

gctgccttcc caaaaagggc gcgtgcccag gctttgagtt gcactcgcac cgtagtggca 22860

tcaaaaggtg accgtgcccg gtctgggcgt taggatacag cgcctgcata aaagccttga 22920

tctgcttaaa agccacctga gcctttgcgc cttcagagaa gaacatgccg caagacttgc 22980

cggaaaactg attggccgga cacgcggcgt cgtgcacgca gcaccttgcg tcggtgttgg 23040

agatctgcac cacatttcgg ccccaccggt tcttcacgat cttggccttg ctagactgct 23100

ccttcagcgc gcgctgcccg ttttcgctcg tcacatccat ttcaatcacg tgctccttat 23160

ttatcataat gcttccgtgt agacacttaa gctcgccttc gatctcagcg cagcggtgca 23220

gccacaacgc gcagcccgtg ggctcgtgat gcttgtaggt cacctctgca aacgactgca 23280

ggtacgcctg caggaatcgc cccatcatcg tcacaaaggt cttgttgctg gtgaaggtca 23340

gctgcaaccc gcggtgctcc tcgttcagcc aggtcttgca tacggccgcc agagcttcca 23400

cttggtcagg cagtagtttg aagttcgcct ttagatcgtt atccacgtgg tacttgtcca 23460

tcagcgcgcg cgcagcctcc atgcccttct cccacgcaga cacgatcggc acactcagcg 23520

ggttcatcac cgtaatttca ctttccgctt cgctgggctc ttcctcttcc tcttgcgtcc 23580

gcataccacg cgccactggg tcgtcttcat tcagccgccg cactgtgcgc ttacctcctt 23640

tgccatgctt gattagcacc ggtgggttgc tgaaacccac catttgtagc gccacatctt 23700

ctctttcttc ctcgctgtcc acgattacct ctggtgatgg cgggcgctcg ggcttgggag 23760

aagggcgctt ctttttcttc ttgggcgcaa tggccaaatc cgccgccgag gtcgatggcc 23820

gcgggctggg tgtgcgcggc accagcgcgt cttgtgatga gtcttcctcg tcctcggact 23880

cgatacgccg cctcatccgc ttttttgggg gcgcccgggg aggcggcggc gacggggacg 23940

gggacgacac gtcctccatg gttgggggac gtcgcgccgc accgcgtccg cgctcggggg 24000

tggtttcgcg ctgctcctct tcccgactgg ccatttcctt ctcctatagg cagaaaaaga 24060

tcatggagtc agtcgagaag aaggacagcc taaccgcccc ctctgagttc gccaccaccg 24120

cctccaccga tgccgccaac gcgcctacca ccttccccgt cgaggcaccc ccgcttgagg 24180

aggaggaagt gattatcgag caggacccag gttttgtaag cgaagacgac gaggaccgct 24240

cagtaccaac agaggataaa aagcaagacc aggacaacgc agaggcaaac gaggaacaag 24300

tcgggcgggg ggacgaaagg catggcgact acctagatgt gggagacgac gtgctgttga 24360

agcatctgca gcgccagtgc gccattatct gcgacgcgtt gcaagagcgc agcgatgtgc 24420

ccctcgccat agcggatgtc agccttgcct acgaacgcca cctattctca ccgcgcgtac 24480

cccccaaacg ccaagaaaac ggcacatgcg agcccaaccc gcgcctcaac ttctaccccg 24540

tatttgccgt gccagaggtg cttgccacct atcacatctt tttccaaaac tgcaagatac 24600

ccctatcctg ccgtgccaac cgcagccgag cggacaagca gctggccttg cggcagggcg 24660

ctgtcatacc tgatatcgcc tcgctcaacg aagtgccaaa aatctttgag ggtcttggac 24720

gcgacgagaa gcgcgcggca aacgctctgc aacaggaaaa cagcgaaaat gaaagtcact 24780

ctggagtgtt ggtggaactc gagggtgaca acgcgcgcct agccgtacta aaacgcagca 24840

tcgaggtcac ccactttgcc tacccggcac ttaacctacc ccccaaggtc atgagcacag 24900

tcatgagtga gctgatcgtg cgccgtgcgc agcccctgga gagggatgca aatttgcaag 24960

aacaaacaga ggagggccta cccgcagttg gcgacgagca gctagcgcgc tggcttcaaa 25020

cgcgcgagcc tgccgacttg gaggagcgac gcaaactaat gatggccgca gtgctcgtta 25080

ccgtggagct tgagtgcatg cagcggttct ttgctgaccc ggagatgcag cgcaagctag 25140

aggaaacatt gcactacacc tttcgacagg gctacgtacg ccaggcctgc aagatctcca 25200

acgtggagct ctgcaacctg gtctcctacc ttggaatttt gcacgaaaac cgccttgggc 25260

aaaacgtgct tcattccacg ctcaagggcg aggcgcgccg cgactacgtc cgcgactgcg 25320

tttacttatt tctatgctac acctggcaga cggccatggg cgtttggcag cagtgcttgg 25380

aggagtgcaa cctcaaggag ctgcagaaac tgctaaagca aaacttgaag gacctatgga 25440

cggccttcaa cgagcgctcc gtggccgcgc acctggcgga catcattttc cccgaacgcc 25500

tgcttaaaac cctgcaacag ggtctgccag acttcaccag tcaaagcatg ttgcagaact 25560

ttaggaactt tatcctagag cgctcaggaa tcttgcccgc cacctgctgt gcacttccta 25620

gcgactttgt gcccattaag taccgcgaat gccctccgcc gctttggggc cactgctacc 25680

ttctgcagct agccaactac cttgcctacc actctgacat aatggaagac gtgagcggtg 25740

acggtctact ggagtgtcac tgtcgctgca acctatgcac cccgcaccgc tccctggttt 25800

gcaattcgca gctgcttaac gaaagtcaaa ttatcggtac ctttgagctg cagggtccct 25860

cgcctgacga aaagtccgcg gctccggggt tgaaactcac tccggggctg tggacgtcgg 25920

cttaccttcg caaatttgta cctgaggact accacgccca cgagattagg ttctacgaag 25980

accaatcccg cccgcctaat gcggagctta ccgcctgcgt cattacccag ggccacattc 26040

ttggccaatt gcaagccatc aacaaagccc gccaagagtt tctgctacga aagggacggg 26100

gggtttactt ggacccccag tccggcgagg agctcaaccc aatccccccg ccgccgcagc 26160

cctatcagca gcagccgcgg gcccttgctt cccaggatgg cacccaaaaa gaagctgcag 26220

ctgccgccgc cacccacgga cgaggaggaa tactgggaca gtcaggcaga ggaggttttg 26280

gacgaggagg aggaggacat gatggaagac tgggagagcc tagacgagga agcttccgag 26340

gtcgaagagg tgtcagacga aacaccgtca ccctcggtcg cattcccctc gccggcgccc 26400

cagaaatcgg caaccggttc cagcatggct acaacctccg ctcctcaggc gccgccggca 26460

ctgcccgttc gccgacccaa ccgtagatgg gacaccactg gaaccagggc cggtaagtcc 26520

aagcagccgc cgccgttagc ccaagagcaa caacagcgcc aaggctaccg ctcatggcgc 26580

gggcacaaga acgccatagt tgcttgcttg caagactgtg ggggcaacat ctccttcgcc 26640

cgccgctttc ttctctacca tcacggcgtg gccttccccc gtaacatcct gcattactac 26700

cgtcatctct acagcccata ctgcaccggc ggcagcggca gcaacagcag cggccacaca 26760

gaagcaaagg cgaccggata gcaagactct gacaaagccc aagaaatcca cagcggcggc 26820

agcagcagga ggaggagcgc tgcgtctggc gcccaacgaa cccgtatcga cccgcgagct 26880

tagaaacagg atttttccca ctctgtatgc tatatttcaa cagagcaggg gccaagaaca 26940

agagctgaaa ataaaaaaca ggtctctgcg atccctcacc cgcagctgcc tgtatcacaa 27000

aagcgaagat cagcttcggc gcacgctgga agacgcggag gctctcttca gtaaatactg 27060

cgcgctgact cttaaggact agtttcgcgc cctttctcaa atttaagcgc gaaaactacg 27120

tcatctccag cggccacacc cggcgccagc acctgttgtc agcgccatta tgagcaagga 27180

aattcccacg ccctacatgt ggagttacca gccacaaatg ggacttgcgg ctggagctgc 27240

ccaagactac tcaacccgaa taaactacat gagcgcggga ccccacatga tatcccgggt 27300

caacggaata cgcgcccacc gaaaccgaat tctcctggaa caggcggcta ttaccaccac 27360

acctcgtaat aaccttaatc cccgtagttg gcccgctgcc ctggtgtacc aggaaagtcc 27420

cgctcccacc actgtggtac ttcccagaga cgcccaggcc gaagttcaga tgactaactc 27480

aggggcgcag cttgcgggcg gctttcgtca cagggtgcgg tcgcccgggc agggtataac 27540

tcacctgaca atcagagggc gaggtattca gctcaacgac gagtcggtga gctcctcgct 27600

tggtctccgt ccggacggga catttcagat cggcggcgcc ggccgctctt cattcacgcc 27660

tcgtcaggca atcctaactc tgcagacctc gtcctctgag ccgcgctctg gaggcattgg 27720

aactctgcaa tttattgagg agtttgtgcc atcggtctac tttaacccct tctcgggacc 27780

tcccggccac tatccggatc aatttattcc taactttgac gcggtaaagg actcggcgga 27840

cggctacgac tgaggccaat gaggccatgt taagtggaga ggcagagcaa ctgcgcctga 27900

aacacctggt ccactgtcgc cgccacaagt gctttgcccg cgactccggt gagttttgct 27960

actttgaatt gcccgaggat catatcgagg gcccggcgca cggcgtccgg cttaccgccc 28020

agggagagct tgcccgtagc ctgattcggg agtttaccca gcgccccctg ctagttgagc 28080

gggacagggg accctgtgtt ctcactgtga tttgcaactg tcctaaccct ggattacatc 28140

aagatccaga tcttctagac ccgggagcgg ccgctgtcga cctgcaggat ccgaattcga 28200

tatcactagt tattaatagt aatcaattac ggggtcatta gttcatagcc catatatgga 28260

gttccgcgtt acataactta cggtaaatgg cccgcctggc tgaccgccca acgacccccg 28320

cccattgacg tcaataatga cgtatgttcc catagtaacg ccaataggga ctttccattg 28380

acgtcaatgg gtggagtatt tacggtaaac tgcccacttg gcagtacatc aagtgtatca 28440

tatgccaagt ccgcccccta ttgacgtcaa tgacggtaaa tggcccgcct ggcattatgc 28500

ccagtacatg accttacggg actttcctac ttggcagtac atctacgtat tagtcatcgc 28560

tattaccatg gtgatgcggt tttggcagta caccaatggg cgtggatagc ggtttgactc 28620

acggggattt ccaagtctcc accccattga cgtcaatggg agtttgtttt ggcaccaaaa 28680

tcaacgggac tttccaaaat gtcgtaatac ccccgccccg ttgacgcaaa tgggcaagct 28740

tgccgggtcg aggtaggcgt gtacggtggg aggcctatat aagcaaccgg tataatcaaa 28800

cagaccagat tgtctgtttg ttaccggtgt ttagtgaacc gggcgcgcct catatcgcct 28860

ggagacgcca tccacgctgt tttgacctcc atagaagaca ccgggaccga tccagcctcc 28920

gcgggccgcc accatggcag cccgtctgct cctcctgggc atccttctcc tgctgctgcc 28980

cctgcccgtc cctgccccgt gccacacagc cgcacgctca gagtgcaagc gcagccacaa 29040

gttcgtgcct ggtgcatggc tggccgggga gggtgtggac gtgaccagcc tccgccgctc 29100

gggctccttc ccagtggaca cacaaaggtt cctgcggccc gacggcacct gcaccctctg 29160

tgaaaatgcc ctacaggagg gcaccctcca gcgcctgcct ctggcgctca ccaactggcg 29220

ggcccagggc tctggctgcc agcgccatgt aaccagggcc aaagtcagct ccactgaagc 29280

tgtggcccgg gatgcggctc gtagcatccg caacgactgg aaggtcgggc tggacgtgac 29340

tcctaagccc accagcaatg tgcatgtgtc tgtggccggc tcacactcac aggcagccaa 29400

ctttgcagcc cagaagaccc accaggacca gtacagcttc agcactgaca cggtggagtg 29460

ccgcttctac agtttccatg tggtacacac tcccccgctg caccctgact tcaagagggc 29520

cctcggggac ctgccccacc acttcaacgc ctccacccag cccgcctacc tcaggcttat 29580

ctccaactac ggcacccact tcatccgggc tgtggagctg ggtggccgca tatcggccct 29640

cactgccctg cgcacctgcg agctggccct ggaagggctc acggacaacg aggtggagga 29700

ctgcctgact gtcgaggccc aggtcaacat aggcatccac ggcagcatct ctgccgaagc 29760

caaggcctgt gaggagaaga agaagaagca caagatgacg gcctccttcc accaaaccta 29820

ccgggagcgc cattcggaag tggttggcgg ccatcacacc tccattaacg acctgctgtt 29880

cgggatccag gccgggcccg agcagtactc agcctgggta aactcgctgc ccggcagccc 29940

tggcctggtg gactacaccc tggaacccct gcacgtgctg ctggacagcc aggacccgcg 30000

gcgggaggca ctgaggaggg ccctgagtca gtacctgacg gacagggctc gctggaggga 30060

ctgcagccgg ccgtgcccac cagggcggca gaagagcccc cgagacccat gccagtgtgt 30120

gtgccatggc tcagcggtca ccacccagga ctgctgccct cggcagaggg gcctggccca 30180

gctggaggtg accttcatcc aagcatgggg cctgtggggg gactggttca ctgccacgga 30240

tgcctatgtg aagctcttct ttggtggcca ggagctgagg acgagcaccg tgtgggacaa 30300

taacaacccc atctggtcag tgcggctgga ttttggggat gtgctcctgg ccacaggggg 30360

gcccctgagg ttgcaggtct gggatcagga ctctggcagg gacgatgacc tccttggcac 30420

ctgtgatcag gctcccaagt ctggttccca tgaggtgaga tgcaacctga atcatggcca 30480

cctaaaattc cgctatcatg ccaggtgctt gccccacctg ggaggaggca cctgcctgga 30540

ctatgtcccc caaatgcttc tgggggagcc tccaggaaac cggagtgggg ccgtgtggtg 30600

acccctctcc ctcccccccc taacgttact ggccgaagcc gcttggaata aggccggtgt 30660

gcgtttgtct atatgttatt ttccaccata ttgccgtctt ttggcaatgt gagggcccgg 30720

aaacctggcc ctgtcttctt gacgagcatt cctaggggtc tttcccctct cgccaaagga 30780

atgcaaggtc tgttgaatgt cgtgaaggaa gcagttcctc tggaagcttc ttgaagacaa 30840

acaacgtctg tagcgaccct ttgcaggcag cggaaccccc cacctggcga caggtgcctc 30900

tgcggccaaa agccacgtgt ataagataca cctgcaaagg cggcacaacc ccagtgccac 30960

gttgtgagtt ggatagttgt ggaaagagtc aaatggctct cctcaagcgt attcaacaag 31020

gggctgaagg atgcccagaa ggtaccccat tgtatgggat ctgatctggg gcctcggtgc 31080

acatgcttta catgtgttta gtcgaggtta aaaaaacgtc taggcccccc gaaccacggg 31140

gacgtggttt tcctttgaaa aacacgatga taagcttgcc acaacccggg atcctctaga 31200

gtcgacatgc aaccaatcct gcttctgctg gccttcctcc tgctgcccag ggcagatgca 31260

ggggagatca tcgggggaca tgaggccaag ccccactccc gcccctacat ggcttatctt 31320

atgatctggg atcagaagtc tctgaagagg tgcggtggct tcctgataca agacgacttc 31380

gtgctgacag ctgctcactg ttggggaagc tccataaatg tcaccttggg ggcccacaat 31440

atcaaagaac aggagccgac ccagcagttt atccctgtga aaagacccat cccccatcca 31500

gcctataatc ctaagaactt ctccaacgac atcatgctac tgcagctgga gagaaaggcc 31560

aagcggacca gagctgtgca gcccctcagg ctacctagca acaaggccca ggtgaagcca 31620

gggcagacat gcagtgtggc cggctggggg cagacggccc ccctgggaaa acactcacac 31680

acactacaag aggtgaagat gacagtgcag gaagatcgaa agtgcgaatc tgacttacgc 31740

cattattacg acagtaccat tgagttgtgc gtgggggacc cagagattaa aaagacttcc 31800

tttaaggggg actctggagg ccctcttgtg tgtaacaagg tggcccaggg cattgtctcc 31860

tatggacgaa acaatggcat gcctccacga gcctgcacca aagtctcaag ctttgtacac 31920

tggataaaga aaaccatgaa acgccactaa aacttgttta ttgcagctta taatggttac 31980

aaataaagca atagcatcac aaatttcaca aataaagcat ttttttcact gcattctagt 32040

tgtggtttgt ccaaactcat caatgtatct taactagtgg taccgatctt attcccttta 32100

actaataaaa aaaaataata aagcatcact tacttaaaat cagttagcaa atttctgtcc 32160

agtttattca gcagcacctc cttgccctcc tcccagctct ggtattgcag cttcctcctg 32220

gctgcaaact ttctccacaa tctaaatgga atgtcagttt cctcctgttc ctgtccatcc 32280

gcacccacta tcttcatgtt gttgcagatg aagcgcgcaa gaccgtctga agataccttc 32340

aaccccgtgt atccatatga cacggaaacc ggtcctccaa ctgtgccttt tcttactcct 32400

ccctttgtat cccccaatgg gtttcaagag agtccccctg gggtactctc tttgcgccta 32460

tccgaacctc tagttacctc caatggcatg cttgcgctca aaatgggcaa cggcctctct 32520

ctggacgagg ccggcaacct tacctcccaa aatgtaacca ctgtgagccc acctctcaaa 32580

aaaaccaagt caaacataaa cctggaaata tctgcacccc tcacagttac ctcagaagcc 32640

ctaactgtgg ctgccgccgc acctctaatg gtcgcgggca acacactcac catgcaatca 32700

caggccccgc taaccgtgca cgactccaaa cttagcattg ccacccaagg acccctcaca 32760

gtgtcagaag gaaagctagc cctgcaaaca tcaggccccc tcaccaccac cgatagcagt 32820

acccttacta tcactgcctc acccccctca actactgcca ctggtagctt gggcattgac 32880

ttgaaagagc ccatttatac acaaaatgga aaactaggac taaagtacgg ggctcctttg 32940

catgtaacag acgacctaaa cactttgacc gtagcaactg gtccaggtgt gactattaat 33000

aatacttcct tgcaaactaa agttactgga gccttgggtt ttgattcaca aggcaatatg 33060

caacttaatg tagcaggagg actaaggatt gattctcaaa acagacgcct tatacttgat 33120

gttagttatc cgtttgatgc tcaaaaccaa ctaaatctaa gactaggaca gggccctctt 33180

tttataaact cagcccacaa cttggatatt aactacaaca aaggccttta cttgtttaca 33240

gcttcaaaca attccaaaaa gcttgaggtt aacctaagca ctgccaaggg gttgatgttt 33300

gacgctacag ccatagccat taatgcagga gatgggcttg aatttggttc acctaatgca 33360

ccaaacacaa atcccctcaa aacaaaaatt ggccatggcc tagaatttga ttcaaacaag 33420

gctatggttc ctaaactagg aactggcctt agttttgaca gcacaggtgc cattacagta 33480

ggaaacaaaa ataatgataa gctaacccta tggacaggtc caaaaccaga agccaactgc 33540

ataattgaat acgggaaaca aaacccagat agcaaactaa ctttaatcct tgtaaaaaat 33600

ggaggaattg ttaatggata tgtaacgcta atgggagcct cagactacgt taacacctta 33660

tttaaaaaca aaaatgtctc cattaatgta gaactatact ttgatgccac tggtcatata 33720

ttaccagact catcttctct taaaacagat ctagaactaa aatacaagca aaccgctgac 33780

tttagtgcaa gaggttttat gccaagtact acagcgtatc catttgtcct tcctaatgcg 33840

ggaacacata atgaaaatta tatttttggt caatgctact acaaagcaag cgatggtgcc 33900

ctttttccgt tggaagttac tgttatgctt aataaacgcc tgccagatag tcgcacatcc 33960

tatgttatga cttttttatg gtccttgaat gctggtctag ctccagaaac tactcaggca 34020

accctcataa cctccccatt taccttttcc tatattagag aagatgacta ataaagaatc 34080

gtttgtgggc caccatggcc ttatgtttca acgtgtttat ttttcaattg cagaaaattt 34140

caagtcattt ttcattcagt agtatagccc caccaccaca tagcttatac agatcaccgt 34200

accttaatca aactcacaga accctagtat tcaacctgcc acctccctcc caacacacag 34260

agtacacagt cctttctccc cggctggcct taaaaagcat catatcatgg gtaacagaca 34320

tattcttagg tgttatattc cacacggttt cctgtcgagc caaacgctca tcagtgatat 34380

taataaactc cccgggcagc tcacttaagt tcatgtcgct gtccagctgc tgagccacag 34440

gctgctgtcc aacttgcggt tgcttaacgg gcggcgaagg agaagtccac gcctacatgg 34500

gggtagagtc ataatcgtgc atcaggatag ggcggtggtg ctgcagcagc gcgcgaataa 34560

actgctgccg ccgccgctcc gtcctgcagg aatacaacat ggcagtggtc tcctcagcga 34620

tgattcgcac cgcccgcagc ataaggcgcc ttgtcctccg ggcacagcag cgcaccctga 34680

tctcacttaa atcagcacag taactgcagc acagcaccac aatattgttc aaaatcccac 34740

agtgcaaggc gctgtatcca aagctcatgg cggggaccac agaacccacg tggccatcat 34800

accacaagcg caggtagatt aagtggcgac ccctcataaa cacgctggac ataaacatta 34860

cctcttttgg catgttgtaa ttcaccacct cccggtacca tataaacctc tgattaaaca 34920

tggcgccatc caccaccatc ctaaaccagc tggccaaaac ctgcccgccg gctatacact 34980

gcagggaacc gggactggaa caatgacagt ggagagccca ggactcgtaa ccatggatca 35040

tcatgctcgt catgatatca atgttggcac aacacaggca cacgtgcata cacttcctca 35100

ggattacaag ctcctcccgc gttagaacca tatcccaggg aacaacccat tcctgaatca 35160

gcgtaaatcc cacactgcag ggaagacctc gcacgtaact cacgttgtgc attgtcaaag 35220

tgttacattc gggcagcagc ggatgatcct ccagtatggt agcgcgggtt tctgtctcaa 35280

aaggaggtag acgatcccta ctgtacggag tgcgccgaga caaccgagat cgtgttggtc 35340

gtagtgtcat gccaaatgga acgccggacg tagtcatatt tcctgaagca aaaccaggtg 35400

cgggcgtgac aaacagatct gcgtctccgg tctcgccgct tagatcgctc tgtgtagtag 35460

ttgtagtata tccactctct caaagcatcc aggcgccccc tggcttcggg ttctatgtaa 35520

actccttcat gcgccgctgc cctgataaca tccaccaccg cagaataagc cacacccagc 35580
caacctacac attcgttctg cgagtcacac acgggaggag cgggaagagc tggaagaacc 35640
atgttttttt ttttattcca aaagattatc caaaacctca aaatgaagat ctattaagtg 35700
aacgcgctcc cctccggtgg cgtggtcaaa ctctacagcc aaagaacaga taatggcatt 35760
tgtaagatgt tgcacaatgg cttccaaaag gcaaacggcc ctcacgtcca agtggacgta 35820
aaggctaaac ccttcagggt gaatctcctc tataaacatt ccagcacctt caaccatgcc 35880
caaataattc tcatctcgcc accttctcaa tatatctcta agcaaatccc gaatattaag 35940
tccggccatt gtaaaaatct gctccagagc gccctccacc ttcagcctca agcagcgaat 36000
catgattgca aaaattcagg ttcctcacag acctgtataa gattcaaaag cggaacatta 36060
acaaaaatac cgcgatcccg taggtccctt cgcagggcca gctgaacata atcgtgcagg 36120
tctgcacgga ccagcgcggc cacttccccg ccaggaacca tgacaaaaga acccacactg 36180
attatgacac gcatactcgg agctatgcta accagcgtag ccccgatgta agcttgttgc 36240
atgggcggcg atataaaatg caaggtgctg ctcaaaaaat caggcaaagc ctcgcgcaaa 36300
aaagaaagca catcgtagtc atgctcatgc agataaaggc aggtaagctc cggaaccacc 36360
acagaaaaag acaccatttt tctctcaaac atgtctgcgg gtttctgcat aaacacaaaa 36420
taaaataaca aaaaaacatt taaacattag aagcctgtct tacaacagga aaaacaaccc 36480
ttataagcat aagacggact acggccatgc cggcgtgacc gtaaaaaaac tggtcaccgt 36540
gattaaaaag caccaccgac agctcctcgg tcatgtccgg agtcataatg taagactcgg 36600
taaacacatc aggttgattc acatcggtca gtgctaaaaa gcgaccgaaa tagcccgggg 36660
gaatacatac ccgcaggcgt agagacaaca ttacagcccc cataggaggt ataacaaaat 36720
taataggaga gaaaaacaca taaacacctg aaaaaccctc ctgcctaggc aaaatagcac 36780
cctcccgctc cagaacaaca tacagcgctt ccacagcggc agccataaca gtcagcctta 36840
ccagtaaaaa agaaaaccta ttaaaaaaac accactcgac acggcaccag ctcaatcagt 36900
cacagtgtaa aaaagggcca agtgcagagc gagtatatat aggactaaaa aatgacgtaa 36960
cggttaaagt ccacaaaaaa cacccagaaa accgcacgcg aacctacgcc cagaaacgaa 37020
agccaaaaaa cccacaactt cctcaaatcg tcacttccgt tttcccacgt tacgtcactt 37080
cccattttaa gaaaactaca attcccaaca catacaagtt actccgccct aaaacctacg 37140
tcacccgccc cgttcccacg ccccgcgcca cgtcacaaac tccaccccct cattatcata 37200
ttggcttcaa tccaaaataa ggtatattat tgatgatgtt aat 37243

<210> 3
<211> 34928
<212> DNA
<213> Artificial Sequence

<220>
<223> Ad5/3-D24-Grb (TGX-01)

<400> 3
   taacatcatc aataatatac cttattttgg attgaagcca atatgataat gagggggtgg 60
   agtttgtgac gtggcgcggg gcgtgggaac ggggcgggtg acgtagtagt gtggcggaag 120
   tgtgatgttg caagtgtggc ggaacacatg taagcgacgg atgtggcaaa agtgacgttt 180
   ttggtgtgcg ccggtgtaca caggaagtga caattttcgc gcggttttag gcggatgttg 240
   tagtaaattt gggcgtaacc gagtaagatt tggccatttt cgcgggaaaa ctgaataaga 300
   ggaagtgaaa tctgaataat tttgtgttac tcatagcgcg taatatttgt ctagggccgc 360
   ggggactttg accgtttacg tggagactcg cccaggtgtt tttctcaggt gttttccgcg 420
   ttccgggtca aagttggcgt tttattatta tagtcagctg acgtgtagtg tatttatacc 480
   cggtgagttc ctcaagaggc cactcttgag tgccagcgag tagagttttc tcctccgagc 540
   cgctccgaca ccgggactga aaatgagaca tattatctgc cacggaggtg ttattaccga 600
   agaaatggcc gccagtcttt tggaccagct gatcgaagag gtactggctg ataatcttcc 660
   acctcctagc cattttgaac cacctaccct tcacgaactg tatgatttag acgtgacggc 720
   ccccgaagat cccaacgagg aggcggtttc gcagattttt cccgactctg taatgttggc 780
   ggtgcaggaa gggattgact tactcacttt tccgccggcg cccggttctc cggagccgcc 840
   tcacctttcc cggcagcccg agcagccgga gcagagagcc ttgggtccgg tttctatgcc 900
   aaaccttgta ccggaggtga tcgatccacc cagtgacgac gaggatgaag agggtgagga 960
   gtttgtgtta gattatgtgg agcaccccgg gcacggttgc aggtcttgtc attatcaccg 1020
   gaggaatacg ggggacccag atattatgtg ttcgctttgc tatatgagga cctgtggcat 1080
   gtttgtctac agtaagtgaa aattatgggc agtgggtgat agagtggtgg gtttggtgtg 1140
   gtaatttttt ttttaatttt tacagttttg tggtttaaag aattttgtat tgtgattttt 1200
   ttaaaaggtc ctgtgtctga acctgagcct gagcccgagc cagaaccgga gcctgcaaga 1260
   cctacccgcc gtcctaaaat ggcgcctgct atcctgagac gcccgacatc acctgtgtct 1320
   agagaatgca atagtagtac ggatagctgt gactccggtc cttctaacac acctcctgag 1380
   atacacccgg tggtcccgct gtgccccatt aaaccagttg ccgtgagagt tggtgggcgt 1440
   cgccaggctg tggaatgtat cgaggacttg cttaacgagc ctgggcaacc tttggacttg 1500
   agctgtaaac gccccaggcc ataaggtgta aacctgtgat tgcgtgtgtg gttaacgcct 1560
   ttgtttgctg aatgagttga tgtaagttta ataaagggtg agataatgtt taacttgcat 1620
   ggcgtgttaa atggggcggg gcttaaaggg tatataatgc gccgtgggct aatcttggtt 1680
   acatctgacc tcatggaggc ttgggagtgt ttggaagatt tttctgctgt gcgtaacttg 1740
   ctggaacaga gctctaacag tacctcttgg ttttggaggt ttctgtgggg ctcatcccag 1800
   gcaaagttag tctgcagaat taaggaggat tacaagtggg aatttgaaga gcttttgaaa 1860
   tcctgtggtg agctgtttga ttctttgaat ctgggtcacc aggcgctttt ccaagagaag 1920
   gtcatcaaga ctttggattt ttccacaccg gggcgcgctg cggctgctgt tgcttttttg 1980
   agttttataa aggataaatg gagcgaagaa acccatctga gcggggggta cctgctggat 2040
   tttctggcca tgcatctgtg gagagcggtt gtgagacaca agaatcgcct gctactgttg 2100
   tcttccgtcc gcccggcgat aataccgacg gaggagcagc agcagcagca ggaggaagcc 2160
   aggcggcggc ggcaggagca gagcccatgg aacccgagag ccggcctgga ccctcgggaa 2220
   tgaatgttgt acaggtggct gaactgtatc cagaactgag acgcattttg acaattacag 2280
   aggatgggca ggggctaaag ggggtaaaga gggagcgggg ggcttgtgag gctacagagg 2340
   aggctaggaa tctagctttt agcttaatga ccagacaccg tcctgagtgt attacttttc 2400
   aacagatcaa ggataattgc gctaatgagc ttgatctgct ggcgcagaag tattccatag 2460
   agcagctgac cacttactgg ctgcagccag gggatgattt tgaggaggct attagggtat 2520
   atgcaaaggt ggcacttagg ccagattgca agtacaagat cagcaaactt gtaaatatca 2580
   ggaattgttg ctacatttct gggaacgggg ccgaggtgga gatagatacg gaggataggg 2640
   tggcctttag atgtagcatg ataaatatgt ggccgggggt gcttggcatg gacggggtgg 2700
   ttattatgaa tgtaaggttt actggcccca attttagcgg tacggttttc ctggccaata 2760
   ccaaccttat cctacacggt gtaagcttct atgggtttaa caatacctgt gtggaagcct 2820
   ggaccgatgt aagggttcgg ggctgtgcct tttactgctg ctggaagggg gtggtgtgtc 2880
   gccccaaaag cagggcttca attaagaaat gcctctttga aaggtgtacc ttgggtatcc 2940
   tgtctgaggg taactccagg gtgcgccaca atgtggcctc cgactgtggt tgcttcatgc 3000
   tagtgaaaag cgtggctgtg attaagcata acatggtatg tggcaactgc gaggacaggg 3060
   cctctcagat gctgacctgc tcggacggca actgtcacct gctgaagacc attcacgtag 3120
   ccagccactc tcgcaaggcc tggccagtgt ttgagcataa catactgacc cgctgttcct 3180
   tgcatttggg taacaggagg ggggtgttcc taccttacca atgcaatttg agtcacacta 3240
   agatattgct tgagcccgag agcatgtcca aggtgaacct gaacggggtg tttgacatga 3300
   ccatgaagat ctggaaggtg ctgaggtacg atgagacccg caccaggtgc agaccctgcg 3360
   agtgtggcgg taaacatatt aggaaccagc ctgtgatgct ggatgtgacc gaggagctga 3420
   ggcccgatca cttggtgctg gcctgcaccc gcgctgagtt tggctctagc gatgaagata 3480
   cagattgagg tactgaaatg tgtgggcgtg gcttaagggt gggaaagaat atataaggtg 3540
   ggggtcttat gtagttttgt atctgttttg cagcagccgc cgccgccatg agcaccaact 3600
   cgtttgatgg aagcattgtg agctcatatt tgacaacgcg catgccccca tgggccgggg 3660
   tgcgtcagaa tgtgatgggc tccagcattg atggtcgccc cgtcctgccc gcaaactcta 3720
   ctaccttgac ctacgagacc gtgtctggaa cgccgttgga gactgcagcc tccgccgccg 3780
   cttcagccgc tgcagccacc gcccgcggga ttgtgactga ctttgctttc ctgagcccgc 3840
   ttgcaagcag tgcagcttcc cgttcatccg cccgcgatga caagttgacg gctcttttgg 3900
   cacaattgga ttctttgacc cgggaactta atgtcgtttc tcagcagctg ttggatctgc 3960
   gccagcaggt ttctgccctg aaggcttcct cccctcccaa tgcggtttaa aacataaata 4020
   aaaaaccaga ctctgtttgg atttggatca agcaagtgtc ttgctgtctt tatttagggg 4080
   ttttgcgcgc gcggtaggcc cgggaccagc ggtctcggtc gttgagggtc ctgtgtattt 4140
   tttccaggac gtggtaaagg tgactctgga tgttcagata catgggcata agcccgtctc 4200
   tggggtggag gtagcaccac tgcagagctt catgctgcgg ggtggtgttg tagatgatcc 4260
   agtcgtagca ggagcgctgg gcgtggtgcc taaaaatgtc tttcagtagc aagctgattg 4320
   ccaggggcag gcccttggtg taagtgttta caaagcggtt aagctgggat gggtgcatac 4380
   gtggggatat gagatgcatc ttggactgta tttttaggtt ggctatgttc ccagccatat 4440
   ccctccgggg attcatgttg tgcagaacca ccagcacagt gtatccggtg cacttgggaa 4500
   atttgtcatg tagcttagaa ggaaatgcgt ggaagaactt ggagacgccc ttgtgacctc 4560
   caagattttc catgcattcg tccataatga tggcaatggg cccacgggcg gcggcctggg 4620
   cgaagatatt tctgggatca ctaacgtcat agttgtgttc caggatgaga tcgtcatagg 4680
   ccatttttac aaagcgcggg cggagggtgc cagactgcgg tataatggtt ccatccggcc 4740
   caggggcgta gttaccctca cagatttgca tttcccacgc tttgagttca gatgggggga 4800
   tcatgtctac ctgcggggcg atgaagaaaa cggtttccgg ggtaggggag atcagctggg 4860
   aagaaagcag gttcctgagc agctgcgact taccgcagcc ggtgggcccg taaatcacac 4920
   ctattaccgg ctgcaactgg tagttaagag agctgcagct gccgtcatcc ctgagcaggg 4980
   gggccacttc gttaagcatg tccctgactc gcatgttttc cctgaccaaa tccgccagaa 5040
   ggcgctcgcc gcccagcgat agcagttctt gcaaggaagc aaagtttttc aacggtttga 5100
   gaccgtccgc cgtaggcatg cttttgagcg tttgaccaag cagttccagg cggtcccaca 5160
   gctcggtcac ctgctctacg gcatctcgat ccagcatatc tcctcgtttc gcgggttggg 5220
   gcggctttcg ctgtacggca gtagtcggtg ctcgtccaga cgggccaggg tcatgtcttt 5280
   ccacgggcgc agggtcctcg tcagcgtagt ctgggtcacg gtgaaggggt gcgctccggg 5340
   ctgcgcgctg gccagggtgc gcttgaggct ggtcctgctg gtgctgaagc gctgccggtc 5400
   ttcgccctgc gcgtcggcca ggtagcattt gaccatggtg tcatagtcca gcccctccgc 5460
   ggcgtggccc ttggcgcgca gcttgccctt ggaggaggcg ccgcacgagg ggcagtgcag 5520
   acttttgagg gcgtagagct tgggcgcgag aaataccgat tccggggagt aggcatccgc 5580
   gccgcaggcc ccgcagacgg tctcgcattc cacgagccag gtgagctctg gccgttcggg 5640
   gtcaaaaacc aggtttcccc catgcttttt gatgcgtttc ttacctctgg tttccatgag 5700
   ccggtgtcca cgctcggtga cgaaaaggct gtccgtgtcc ccgtatacag acttgagagg 5760
   cctgtcctcg agcggtgttc cgcggtcctc ctcgtataga aactcggacc actctgagac 5820
   aaaggctcgc gtccaggcca gcacgaagga ggctaagtgg gaggggtagc ggtcgttgtc 5880
   cactaggggg tccactcgct ccagggtgtg aagacacatg tcgccctctt cggcatcaag 5940
   gaaggtgatt ggtttgtagg tgtaggccac gtgaccgggt gttcctgaag gggggctata 6000
   aaagggggtg ggggcgcgtt cgtcctcact ctcttccgca tcgctgtctg cgagggccag 6060
   ctgttggggt gagtactccc tctgaaaagc gggcatgact tctgcgctaa gattgtcagt 6120
   ttccaaaaac gaggaggatt tgatattcac ctggcccgcg gtgatgcctt tgagggtggc 6180
   cgcatccatc tggtcagaaa agacaatctt tttgttgtca agcttggtgg caaacgaccc 6240
   gtagagggcg ttggacagca acttggcgat ggagcgcagg gtttggtttt tgtcgcgatc 6300
   ggcgcgctcc ttggccgcga tgtttagctg cacgtattcg cgcgcaacgc accgccattc 6360
   gggaaagacg gtggtgcgct cgtcgggcac caggtgcacg cgccaaccgc ggttgtgcag 6420
   ggtgacaagg tcaacgctgg tggctacctc tccgcgtagg cgctcgttgg tccagcagag 6480
   gcggccgccc ttgcgcgagc agaatggcgg tagggggtct agctgcgtct cgtccggggg 6540
   gtctgcgtcc acggtaaaga ccccgggcag caggcgcgcg tcgaagtagt ctatcttgca 6600
   tccttgcaag tctagcgcct gctgccatgc gcgggcggca agcgcgcgct cgtatgggtt 6660
   gagtggggga ccccatggca tggggtgggt gagcgcggag gcgtacatgc cgcaaatgtc 6720
   gtaaacgtag aggggctctc tgagtattcc aagatatgta gggtagcatc ttccaccgcg 6780
   gatgctggcg cgcacgtaat cgtatagttc gtgcgaggga gcgaggaggt cgggaccgag 6840
   gttgctacgg gcgggctgct ctgctcggaa gactatctgc ctgaagatgg catgtgagtt 6900
   ggatgatatg gttggacgct ggaagacgtt gaagctggcg tctgtgagac ctaccgcgtc 6960
   acgcacgaag gaggcgtagg agtcgcgcag cttgttgacc agctcggcgg tgacctgcac 7020
   gtctagggcg cagtagtcca gggtttcctt gatgatgtca tacttatcct gtcccttttt 7080
   tttccacagc tcgcggttga ggacaaactc ttcgcggtct ttccagtact cttggatcgg 7140
   aaacccgtcg gcctccgaac ggtaagagcc tagcatgtag aactggttga cggcctggta 7200
   ggcgcagcat cccttttcta cgggtagcgc gtatgcctgc gcggccttcc ggagcgaggt 7260
   gtgggtgagc gcaaaggtgt ccctgaccat gactttgagg tactggtatt tgaagtcagt 7320
   gtcgtcgcat ccgccctgct cccagagcaa aaagtccgtg cgctttttgg aacgcggatt 7380
   tggcagggcg aaggtgacat cgttgaagag tatctttccc gcgcgaggca taaagttgcg 7440
   tgtgatgcgg aagggtcccg gcacctcgga acggttgtta attacctggg cggcgagcac 7500
   gatctcgtca aagccgttga tgttgtggcc cacaatgtaa agttccaaga agcgcgggat 7560
   gcccttgatg gaaggcaatt ttttaagttc ctcgtaggtg agctcttcag gggagctgag 7620
   cccgtgctct gaaagggccc agtctgcaag atgagggttg gaagcgacga atgagctcca 7680
   caggtcacgg gccattagca tttgcaggtg gtcgcgaaag gtcctaaact ggcgacctat 7740
   ggccattttt tctggggtga tgcagtagaa ggtaagcggg tcttgttccc agcggtccca 7800
   tccaaggttc gcggctaggt ctcgcgcggc agtcactaga ggctcatctc cgccgaactt 7860
   catgaccagc atgaagggca cgagctgctt cccaaaggcc cccatccaag tataggtctc 7920
   tacatcgtag gtgacaaaga gacgctcggt gcgaggatgc gagccgatcg ggaagaactg 7980
   gatctcccgc caccaattgg aggagtggct attgatgtgg tgaaagtaga agtccctgcg 8040
   acgggccgaa cactcgtgct ggcttttgta aaaacgtgcg cagtactggc agcggtgcac 8100
   gggctgtaca tcctgcacga ggttgacctg acgaccgcgc acaaggaagc agagtgggaa 8160
   tttgagcccc tcgcctggcg ggtttggctg gtggtcttct acttcggctg cttgtccttg 8220
   accgtctggc tgctcgaggg gagttacggt ggatcggacc accacgccgc gcgagcccaa 8280
   agtccagatg tccgcgcgcg gcggtcggag cttgatgaca acatcgcgca gatgggagct 8340
   gtccatggtc tggagctccc gcggcgtcag gtcaggcggg agctcctgca ggtttacctc 8400
   gcatagacgg gtcagggcgc gggctagatc caggtgatac ctaatttcca ggggctggtt 8460
   ggtggcggcg tcgatggctt gcaagaggcc gcatccccgc ggcgcgacta cggtaccgcg 8520
   cggcgggcgg tgggccgcgg gggtgtcctt ggatgatgca tctaaaagcg gtgacgcggg 8580
   cgagcccccg gaggtagggg gggctccgga cccgccggga gagggggcag gggcacgtcg 8640
   gcgccgcgcg cgggcaggag ctggtgctgc gcgcgtaggt tgctggcgaa cgcgacgacg 8700
   cggcggttga tctcctgaat ctggcgcctc tgcgtgaaga cgacgggccc ggtgagcttg 8760
   aacctgaaag agagttcgac agaatcaatt tcggtgtcgt tgacggcggc ctggcgcaaa 8820
   atctcctgca cgtctcctga gttgtcttga taggcgatct cggccatgaa ctgctcgatc 8880
   tcttcctcct ggagatctcc gcgtccggct cgctccacgg tggcggcgag gtcgttggaa 8940
   atgcgggcca tgagctgcga gaaggcgttg aggcctccct cgttccagac gcggctgtag 9000
   accacgcccc cttcggcatc gcgggcgcgc atgaccacct gcgcgagatt gagctccacg 9060
   tgccgggcga agacggcgta gtttcgcagg cgctgaaaga ggtagttgag ggtggtggcg 9120
   gtgtgttctg ccacgaagaa gtacataacc cagcgtcgca acgtggattc gttgatatcc 9180
   cccaaggcct caaggcgctc catggcctcg tagaagtcca cggcgaagtt gaaaaactgg 9240
   gagttgcgcg ccgacacggt taactcctcc tccagaagac ggatgagctc ggcgacagtg 9300
   tcgcgcacct cgcgctcaaa ggctacaggg gcctcttctt cttcttcaat ctcctcttcc 9360
   ataagggcct ccccttcttc ttcttctggc ggcggtgggg gaggggggac acggcggcga 9420
   cgacggcgca ccgggaggcg gtcgacaaag cgctcgatca tctccccgcg gcgacggcgc 9480
   atggtctcgg tgacggcgcg gccgttctcg cgggggcgca gttggaagac gccgcccgtc 9540
   atgtcccggt tatgggttgg cggggggctg ccatgcggca gggatacggc gctaacgatg 9600
   catctcaaca attgttgtgt aggtactccg ccgccgaggg acctgagcga gtccgcatcg 9660
   accggatcgg aaaacctctc gagaaaggcg tctaaccagt cacagtcgca aggtaggctg 9720
   agcaccgtgg cgggcggcag cgggcggcgg tcggggttgt ttctggcgga ggtgctgctg 9780
   atgatgtaat taaagtaggc ggtcttgaga cggcggatgg tcgacagaag caccatgtcc 9840
   ttgggtccgg cctgctgaat gcgcaggcgg tcggccatgc cccaggcttc gttttgacat 9900
   cggcgcaggt ctttgtagta gtcttgcatg agcctttcta ccggcacttc ttcttctcct 9960
   tcctcttgtc ctgcatctct tgcatctatc gctgcggcgg cggcggagtt tggccgtagg 10020
   tggcgccctc ttcctcccat gcgtgtgacc ccgaagcccc tcatcggctg aagcagggct 10080
   aggtcggcga caacgcgctc ggctaatatg gcctgctgca cctgcgtgag ggtagactgg 10140
   aagtcatcca tgtccacaaa gcggtggtat gcgcccgtgt tgatggtgta agtgcagttg 10200
   gccataacgg accagttaac ggtctggtga cccggctgcg agagctcggt gtacctgaga 10260
   cgcgagtaag ccctcgagtc aaatacgtag tcgttgcaag tccgcaccag gtactggtat 10320
   cccaccaaaa agtgcggcgg cggctggcgg tagaggggcc agcgtagggt ggccggggct 10380
   ccgggggcga gatcttccaa cataaggcga tgatatccgt agatgtacct ggacatccag 10440
   gtgatgccgg cggcggtggt ggaggcgcgc ggaaagtcgc ggacgcggtt ccagatgttg 10500
   cgcagcggca aaaagtgctc catggtcggg acgctctggc cggtcaggcg cgcgcaatcg 10560
   ttgacgctct agaccgtgca aaaggagagc ctgtaagcgg gcactcttcc gtggtctggt 10620
   ggataaattc gcaagggtat catggcggac gaccggggtt cgagccccgt atccggccgt 10680
   ccgccgtgat ccatgcggtt accgcccgcg tgtcgaaccc aggtgtgcga cgtcagacaa 10740
   cgggggagtg ctccttttgg cttccttcca ggcgcggcgg ctgctgcgct agcttttttg 10800
   gccactggcc gcgcgcagcg taagcggtta ggctggaaag cgaaagcatt aagtggctcg 10860
   ctccctgtag ccggagggtt attttccaag ggttgagtcg cgggaccccc ggttcgagtc 10920
   tcggaccggc cggactgcgg cgaacggggg tttgcctccc cgtcatgcaa gaccccgctt 10980
   gcaaattcct ccggaaacag ggacgagccc cttttttgct tttcccagat gcatccggtg 11040
   ctgcggcaga tgcgcccccc tcctcagcag cggcaagagc aagagcagcg gcagacatgc 11100
   agggcaccct cccctcctcc taccgcgtca ggaggggcga catccgcggt tgacgcggca 11160
   gcagatggtg attacgaacc cccgcggcgc cgggcccggc actacctgga cttggaggag 11220
   ggcgagggcc tggcgcggct aggagcgccc tctcctgagc ggcacccaag ggtgcagctg 11280
   aagcgtgata cgcgtgaggc gtacgtgccg cggcagaacc tgtttcgcga ccgcgaggga 11340
   gaggagcccg aggagatgcg ggatcgaaag ttccacgcag ggcgcgagct gcggcatggc 11400
   ctgaatcgcg agcggttgct gcgcgaggag gactttgagc ccgacgcgcg aaccgggatt 11460
   agtcccgcgc gcgcacacgt ggcggccgcc gacctggtaa ccgcatacga gcagacggtg 11520
   aaccaggaga ttaactttca aaaaagcttt aacaaccacg tgcgtacgct tgtggcgcgc 11580
   gaggaggtgg ctataggact gatgcatctg tgggactttg taagcgcgct ggagcaaaac 11640
   ccaaatagca agccgctcat ggcgcagctg ttccttatag tgcagcacag cagggacaac 11700
   gaggcattca gggatgcgct gctaaacata gtagagcccg agggccgctg gctgctcgat 11760
   ttgataaaca tcctgcagag catagtggtg caggagcgca gcttgagcct ggctgacaag 11820
   gtggccgcca tcaactattc catgcttagc ctgggcaagt tttacgcccg caagatatac 11880
   catacccctt acgttcccat agacaaggag gtaaagatcg aggggttcta catgcgcatg 11940
   gcgctgaagg tgcttacctt gagcgacgac ctgggcgttt atcgcaacga gcgcatccac 12000
   aaggccgtga gcgtgagccg gcggcgcgag ctcagcgacc gcgagctgat gcacagcctg 12060
   caaagggccc tggctggcac gggcagcggc gatagagagg ccgagtccta ctttgacgcg 12120
   ggcgctgacc tgcgctgggc cccaagccga cgcgccctgg aggcagctgg ggccggacct 12180
   gggctggcgg tggcacccgc gcgcgctggc aacgtcggcg gcgtggagga atatgacgag 12240
   gacgatgagt acgagccaga ggacggcgag tactaagcgg tgatgtttct gatcagatga 12300
   tgcaagacgc aacggacccg gcggtgcggg cggcgctgca gagccagccg tccggcctta 12360
   actccacgga cgactggcgc caggtcatgg accgcatcat gtcgctgact gcgcgcaatc 12420
   ctgacgcgtt ccggcagcag ccgcaggcca accggctctc cgcaattctg gaagcggtgg 12480
   tcccggcgcg cgcaaacccc acgcacgaga aggtgctggc gatcgtaaac gcgctggccg 12540
   aaaacagggc catccggccc gacgaggccg gcctggtcta cgacgcgctg cttcagcgcg 12600
   tggctcgtta caacagcggc aacgtgcaga ccaacctgga ccggctggtg ggggatgtgc 12660
   gcgaggccgt ggcgcagcgt gagcgcgcgc agcagcaggg caacctgggc tccatggttg 12720
   cactaaacgc cttcctgagt acacagcccg ccaacgtgcc gcggggacag gaggactaca 12780
   ccaactttgt gagcgcactg cggctaatgg tgactgagac accgcaaagt gaggtgtacc 12840
   agtctgggcc agactatttt ttccagacca gtagacaagg cctgcagacc gtaaacctga 12900
   gccaggcttt caaaaacttg caggggctgt ggggggtgcg ggctcccaca ggcgaccgcg 12960
   cgaccgtgtc tagcttgctg acgcccaact cgcgcctgtt gctgctgcta atagcgccct 13020
   tcacggacag tggcagcgtg tcccgggaca catacctagg tcacttgctg acactgtacc 13080
   gcgaggccat aggtcaggcg catgtggacg agcatacttt ccaggagatt acaagtgtca 13140
   gccgcgcgct ggggcaggag gacacgggca gcctggaggc aaccctaaac tacctgctga 13200
   ccaaccggcg gcagaagatc ccctcgttgc acagtttaaa cagcgaggag gagcgcattt 13260
   tgcgctacgt gcagcagagc gtgagcctta acctgatgcg cgacggggta acgcccagcg 13320
   tggcgctgga catgaccgcg cgcaacatgg aaccgggcat gtatgcctca aaccggccgt 13380
   ttatcaaccg cctaatggac tacttgcatc gcgcggccgc cgtgaacccc gagtatttca 13440
   ccaatgccat cttgaacccg cactggctac cgccccctgg tttctacacc gggggattcg 13500
   aggtgcccga gggtaacgat ggattcctct gggacgacat agacgacagc gtgttttccc 13560
   cgcaaccgca gaccctgcta gagttgcaac agcgcgagca ggcagaggcg gcgctgcgaa 13620
   aggaaagctt ccgcaggcca agcagcttgt ccgatctagg cgctgcggcc ccgcggtcag 13680
   atgctagtag cccatttcca agcttgatag ggtctcttac cagcactcgc accacccgcc 13740
   cgcgcctgct gggcgaggag gagtacctaa acaactcgct gctgcagccg cagcgcgaaa 13800
   aaaacctgcc tccggcattt cccaacaacg ggatagagag cctagtggac aagatgagta 13860
   gatggaagac gtacgcgcag gagcacaggg acgtgccagg cccgcgcccg cccacccgtc 13920
   gtcaaaggca cgaccgtcag cggggtctgg tgtgggagga cgatgactcg gcagacgaca 13980
   gcagcgtcct ggatttggga gggagtggca acccgtttgc gcaccttcgc cccaggctgg 14040
   ggagaatgtt ttaaaaaaaa aaaaagcatg atgcaaaata aaaaactcac caaggccatg 14100
   gcaccgagcg ttggttttct tgtattcccc ttagtatgcg gcgcgcggcg atgtatgagg 14160
   aaggtcctcc tccctcctac gagagtgtgg tgagcgcggc gccagtggcg gcggcgctgg 14220
   gttctccctt cgatgctccc ctggacccgc cgtttgtgcc tccgcggtac ctgcggccta 14280
   ccggggggag aaacagcatc cgttactctg agttggcacc cctattcgac accacccgtg 14340
   tgtacctggt ggacaacaag tcaacggatg tggcatccct gaactaccag aacgaccaca 14400
   gcaactttct gaccacggtc attcaaaaca atgactacag cccgggggag gcaagcacac 14460
   agaccatcaa tcttgacgac cggtcgcact ggggcggcga cctgaaaacc atcctgcata 14520
   ccaacatgcc aaatgtgaac gagttcatgt ttaccaataa gtttaaggcg cgggtgatgg 14580
   tgtcgcgctt gcctactaag gacaatcagg tggagctgaa atacgagtgg gtggagttca 14640
   cgctgcccga gggcaactac tccgagacca tgaccataga ccttatgaac aacgcgatcg 14700
   tggagcacta cttgaaagtg ggcagacaga acggggttct ggaaagcgac atcggggtaa 14760
   agtttgacac ccgcaacttc agactggggt ttgaccccgt cactggtctt gtcatgcctg 14820
   gggtatatac aaacgaagcc ttccatccag acatcatttt gctgccagga tgcggggtgg 14880
   acttcaccca cagccgcctg agcaacttgt tgggcatccg caagcggcaa cccttccagg 14940
   agggctttag gatcacctac gatgatctgg agggtggtaa cattcccgca ctgttggatg 15000
   tggacgccta ccaggcgagc ttgaaagatg acaccgaaca gggcgggggt ggcgcaggcg 15060
   gcagcaacag cagtggcagc ggcgcggaag agaactccaa cgcggcagcc gcggcaatgc 15120
   agccggtgga ggacatgaac gatcatgcca ttcgcggcga cacctttgcc acacgggctg 15180
   aggagaagcg cgctgaggcc gaagcagcgg ccgaagctgc cgcccccgct gcgcaacccg 15240
   aggtcgagaa gcctcagaag aaaccggtga tcaaacccct gacagaggac agcaagaaac 15300
   gcagttacaa cctaataagc aatgacagca ccttcaccca gtaccgcagc tggtaccttg 15360
   catacaacta cggcgaccct cagaccggaa tccgctcatg gaccctgctt tgcactcctg 15420
   acgtaacctg cggctcggag caggtctact ggtcgttgcc agacatgatg caagaccccg 15480
   tgaccttccg ctccacgcgc cagatcagca actttccggt ggtgggcgcc gagctgttgc 15540
   ccgtgcactc caagagcttc tacaacgacc aggccgtcta ctcccaactc atccgccagt 15600
   ttacctctct gacccacgtg ttcaatcgct ttcccgagaa ccagattttg gcgcgcccgc 15660
   cagcccccac catcaccacc gtcagtgaaa acgttcctgc tctcacagat cacgggacgc 15720
   taccgctgcg caacagcatc ggaggagtcc agcgagtgac cattactgac gccagacgcc 15780
   gcacctgccc ctacgtttac aaggccctgg gcatagtctc gccgcgcgtc ctatcgagcc 15840
   gcactttttg agcaagcatg tccatcctta tatcgcccag caataacaca ggctggggcc 15900
   tgcgcttccc aagcaagatg tttggcgggg ccaagaagcg ctccgaccaa cacccagtgc 15960
   gcgtgcgcgg gcactaccgc gcgccctggg gcgcgcacaa acgcggccgc actgggcgca 16020
   ccaccgtcga tgacgccatc gacgcggtgg tggaggaggc gcgcaactac acgcccacgc 16080
   cgccaccagt gtccacagtg gacgcggcca ttcagaccgt ggtgcgcgga gcccggcgct 16140
   atgctaaaat gaagagacgg cggaggcgcg tagcacgtcg ccaccgccgc cgacccggca 16200
   ctgccgccca acgcgcggcg gcggccctgc ttaaccgcgc acgtcgcacc ggccgacggg 16260
   cggccatgcg ggccgctcga aggctggccg cgggtattgt cactgtgccc cccaggtcca 16320
   ggcgacgagc ggccgccgca gcagccgcgg ccattagtgc tatgactcag ggtcgcaggg 16380
   gcaacgtgta ttgggtgcgc gactcggtta gcggcctgcg cgtgcccgtg cgcacccgcc 16440
   ccccgcgcaa ctagattgca agaaaaaact acttagactc gtactgttgt atgtatccag 16500
   cggcggcggc gcgcaacgaa gctatgtcca agcgcaaaat caaagaagag atgctccagg 16560
   tcatcgcgcc ggagatctat ggccccccga agaaggaaga gcaggattac aagccccgaa 16620
   agctaaagcg ggtcaaaaag aaaaagaaag atgatgatga tgaacttgac gacgaggtgg 16680
   aactgctgca cgctaccgcg cccaggcgac gggtacagtg gaaaggtcga cgcgtaaaac 16740
   gtgttttgcg acccggcacc accgtagtct ttacgcccgg tgagcgctcc acccgcacct 16800
   acaagcgcgt gtatgatgag gtgtacggcg acgaggacct gcttgagcag gccaacgagc 16860
   gcctcgggga gtttgcctac ggaaagcggc ataaggacat gctggcgttg ccgctggacg 16920
   agggcaaccc aacacctagc ctaaagcccg taacactgca gcaggtgctg cccgcgcttg 16980
   caccgtccga agaaaagcgc ggcctaaagc gcgagtctgg tgacttggca cccaccgtgc 17040
   agctgatggt acccaagcgc cagcgactgg aagatgtctt ggaaaaaatg accgtggaac 17100
   ctgggctgga gcccgaggtc cgcgtgcggc caatcaagca ggtggcgccg ggactgggcg 17160
   tgcagaccgt ggacgttcag atacccacta ccagtagcac cagtattgcc accgccacag 17220
   agggcatgga gacacaaacg tccccggttg cctcagcggt ggcggatgcc gcggtgcagg 17280
   cggtcgctgc ggccgcgtcc aagacctcta cggaggtgca aacggacccg tggatgtttc 17340
   gcgtttcagc cccccggcgc ccgcgccgtt cgaggaagta cggcgccgcc agcgcgctac 17400
   tgcccgaata tgccctacat ccttccattg cgcctacccc cggctatcgt ggctacacct 17460
   accgccccag aagacgagca actacccgac gccgaaccac cactggaacc cgccgccgcc 17520
   gtcgccgtcg ccagcccgtg ctggccccga tttccgtgcg cagggtggct cgcgaaggag 17580
   gcaggaccct ggtgctgcca acagcgcgct accaccccag catcgtttaa aagccggtct 17640
   ttgtggttct tgcagatatg gccctcacct gccgcctccg tttcccggtg ccgggattcc 17700
   gaggaagaat gcaccgtagg aggggcatgg ccggccacgg cctgacgggc ggcatgcgtc 17760
   gtgcgcacca ccggcggcgg cgcgcgtcgc accgtcgcat gcgcggcggt atcctgcccc 17820
   tccttattcc actgatcgcc gcggcgattg gcgccgtgcc cggaattgca tccgtggcct 17880
   tgcaggcgca gagacactga ttaaaaacaa gttgcatgtg gaaaaatcaa aataaaaagt 17940
   ctggactctc acgctcgctt ggtcctgtaa ctattttgta gaatggaaga catcaacttt 18000
   gcgtctctgg ccccgcgaca cggctcgcgc ccgttcatgg gaaactggca agatatcggc 18060
   accagcaata tgagcggtgg cgccttcagc tggggctcgc tgtggagcgg cattaaaaat 18120
   ttcggttcca ccgttaagaa ctatggcagc aaggcctgga acagcagcac aggccagatg 18180
   ctgagggata agttgaaaga gcaaaatttc caacaaaagg tggtagatgg cctggcctct 18240
   ggcattagcg gggtggtgga cctggccaac caggcagtgc aaaataagat taacagtaag 18300
   cttgatcccc gccctcccgt agaggagcct ccaccggccg tggagacagt gtctccagag 18360
   gggcgtggcg aaaagcgtcc gcgccccgac agggaagaaa ctctggtgac gcaaatagac 18420
   gagcctccct cgtacgagga ggcactaaag caaggcctgc ccaccacccg tcccatcgcg 18480
   cccatggcta ccggagtgct gggccagcac acacccgtaa cgctggacct gcctcccccc 18540
   gccgacaccc agcagaaacc tgtgctgcca ggcccgaccg ccgttgttgt aacccgtcct 18600
   agccgcgcgt ccctgcgccg cgccgccagc ggtccgcgat cgttgcggcc cgtagccagt 18660
   ggcaactggc aaagcacact gaacagcatc gtgggtctgg gggtgcaatc cctgaagcgc 18720
   cgacgatgct tctgatagct aacgtgtcgt atgtgtgtca tgtatgcgtc catgtcgccg 18780
   ccagaggagc tgctgagccg ccgcgcgccc gctttccaag atggctaccc cttcgatgat 18840
   gccgcagtgg tcttacatgc acatctcggg ccaggacgcc tcggagtacc tgagccccgg 18900
   gctggtgcag tttgcccgcg ccaccgagac gtacttcagc ctgaataaca agtttagaaa 18960
   ccccacggtg gcgcctacgc acgacgtgac cacagaccgg tcccagcgtt tgacgctgcg 19020
   gttcatccct gtggaccgtg aggatactgc gtactcgtac aaggcgcggt tcaccctagc 19080
   tgtgggtgat aaccgtgtgc tggacatggc ttccacgtac tttgacatcc gcggcgtgct 19140
   ggacaggggc cctactttta agccctactc tggcactgcc tacaacgccc tggctcccaa 19200
   gggtgcccca aatccttgcg aatgggatga agctgctact gctcttgaaa taaacctaga 19260
   agaagaggac gatgacaacg aagacgaagt agacgagcaa gctgagcagc aaaaaactca 19320
   cgtatttggg caggcgcctt attctggtat aaatattaca aaggagggta ttcaaatagg 19380
   tgtcgaaggt caaacaccta aatatgccga taaaacattt caacctgaac ctcaaatagg 19440
   agaatctcag tggtacgaaa cagaaattaa tcatgcagct gggagagtcc taaaaaagac 19500
   taccccaatg aaaccatgtt acggttcata tgcaaaaccc acaaatgaaa atggagggca 19560
   aggcattctt gtaaagcaac aaaatggaaa gctagaaagt caagtggaaa tgcaattttt 19620
   ctcaactact gaggcagccg caggcaatgg tgataacttg actcctaaag tggtattgta 19680
   cagtgaagat gtagatatag aaaccccaga cactcatatt tcttacatgc ccactattaa 19740
   ggaaggtaac tcacgagaac taatgggcca acaatctatg cccaacaggc ctaattacat 19800
   tgcttttagg gacaatttta ttggtctaat gtattacaac agcacgggta atatgggtgt 19860
   tctggcgggc caagcatcgc agttgaatgc tgttgtagat ttgcaagaca gaaacacaga 19920
   gctttcatac cagcttttgc ttgattccat tggtgataga accaggtact ttctatgtgg 19980
   gaatcaggct gttgacagct atgatccaga tgttagaatt attgaaaatc atggaactga 20040
   agatgaactt ccaaattact gctttccact gggaggtgtg attaatacag agactcttac 20100
   caaggtaaaa cctaaaacag gtcaggaaaa tggatgggaa aaagatgcta cagaattttc 20160
   agataaaaat gaaataagag ttggaaataa ttttgccatg gaaatcaatc taaatgccaa 20220
   cctgtggaga aatttcctgt actccaacat agcgctgtat ttgcccgaca agctaaagta 20280
   cagtccttcc aacgtaaaaa tttctgataa cccaaacacc tacgactaca tgaacaagcg 20340
   agtggtggct cccgggctag tggactgcta cattaacctt ggagcacgct ggtcccttga 20400
   ctatatggac aacgtcaacc catttaacca ccaccgcaat gctggcctgc gctaccgctc 20460
   aatgttgctg ggcaatggtc gctatgtgcc cttccacatc caggtgcctc agaagttctt 20520
   tgccattaaa aacctccttc tcctgccggg ctcatacacc tacgagtgga acttcaggaa 20580
   ggatgttaac atggttctgc agagctccct aggaaatgac ctaagggttg acggagccag 20640
   cattaagttt gatagcattt gcctttacgc caccttcttc cccatggccc acaacaccgc 20700
   ctccacgctt gaggccatgc ttagaaacga caccaacgac cagtccttta acgactatct 20760
   ctccgccgcc aacatgctct accctatacc cgccaacgct accaacgtgc ccatatccat 20820
   cccctcccgc aactgggcgg ctttccgcgg ctgggccttc acgcgcctta agactaagga 20880
   aaccccatca ctgggctcgg gctacgaccc ttattacacc tactctggct ctatacccta 20940
   cctagatgga accttttacc tcaaccacac ctttaagaag gtggccatta cctttgactc 21000
   ttctgtcagc tggcctggca atgaccgcct gcttaccccc aacgagtttg aaattaagcg 21060
   ctcagttgac ggggagggtt acaacgttgc ccagtgtaac atgaccaaag actggttcct 21120
   ggtacaaatg ctagctaact ataacattgg ctaccagggc ttctatatcc cagagagcta 21180
   caaggaccgc atgtactcct tctttagaaa cttccagccc atgagccgtc aggtggtgga 21240
   tgatactaaa tacaaggact accaacaggt gggcatccta caccaacaca acaactctgg 21300
   atttgttggc taccttgccc ccaccatgcg cgaaggacag gcctaccctg ctaacttccc 21360
   ctatccgctt ataggcaaga ccgcagttga cagcattacc cagaaaaagt ttctttgcga 21420
   tcgcaccctt tggcgcatcc cattctccag taactttatg tccatgggcg cactcacaga 21480
   cctgggccaa aaccttctct acgccaactc cgcccacgcg ctagacatga cttttgaggt 21540
   ggatcccatg gacgagccca cccttcttta tgttttgttt gaagtctttg acgtggtccg 21600
   tgtgcaccag ccgcaccgcg gcgtcatcga aaccgtgtac ctgcgcacgc ccttctcggc 21660
   cggcaacgcc acaacataaa gaagcaagca acatcaacaa cagctgccgc catgggctcc 21720
   agtgagcagg aactgaaagc cattgtcaaa gatcttggtt gtgggccata ttttttgggc 21780
   acctatgaca agcgctttcc aggctttgtt tctccacaca agctcgcctg cgccatagtc 21840
   aatacggccg gtcgcgagac tgggggcgta cactggatgg cctttgcctg gaacccgcac 21900
   tcaaaaacat gctacctctt tgagcccttt ggcttttctg accagcgact caagcaggtt 21960
   taccagtttg agtacgagtc actcctgcgc cgtagcgcca ttgcttcttc ccccgaccgc 22020
   tgtataacgc tggaaaagtc cacccaaagc gtacaggggc ccaactcggc cgcctgtgga 22080
   ctattctgct gcatgtttct ccacgccttt gccaactggc cccaaactcc catggatcac 22140
   aaccccacca tgaaccttat taccggggta cccaactcca tgctcaacag tccccaggta 22200
   cagcccaccc tgcgtcgcaa ccaggaacag ctctacagct tcctggagcg ccactcgccc 22260
   tacttccgca gccacagtgc gcagattagg agcgccactt ctttttgtca cttgaaaaac 22320
   atgtaaaaat aatgtactag agacactttc aataaaggca aatgctttta tttgtacact 22380
   ctcgggtgat tatttacccc cacccttgcc gtctgcgccg tttaaaaatc aaaggggttc 22440
   tgccgcgcat cgctatgcgc cactggcagg gacacgttgc gatactggtg tttagtgctc 22500
   cacttaaact caggcacaac catccgcggc agctcggtga agttttcact ccacaggctg 22560
   cgcaccatca ccaacgcgtt tagcaggtcg ggcgccgata tcttgaagtc gcagttgggg 22620
   cctccgccct gcgcgcgcga gttgcgatac acagggttgc agcactggaa cactatcagc 22680
   gccgggtggt gcacgctggc cagcacgctc ttgtcggaga tcagatccgc gtccaggtcc 22740
   tccgcgttgc tcagggcgaa cggagtcaac tttggtagct gccttcccaa aaagggcgcg 22800
   tgcccaggct ttgagttgca ctcgcaccgt agtggcatca aaaggtgacc gtgcccggtc 22860
   tgggcgttag gatacagcgc ctgcataaaa gccttgatct gcttaaaagc cacctgagcc 22920
   tttgcgcctt cagagaagaa catgccgcaa gacttgccgg aaaactgatt ggccggacag 22980
   gccgcgtcgt gcacgcagca ccttgcgtcg gtgttggaga tctgcaccac atttcggccc 23040
   caccggttct tcacgatctt ggccttgcta gactgctcct tcagcgcgcg ctgcccgttt 23100
   tcgctcgtca catccatttc aatcacgtgc tccttattta tcataatgct tccgtgtaga 23160
   cacttaagct cgccttcgat ctcagcgcag cggtgcagcc acaacgcgca gcccgtgggc 23220
   tcgtgatgct tgtaggtcac ctctgcaaac gactgcaggt acgcctgcag gaatcgcccc 23280
   atcatcgtca caaaggtctt gttgctggtg aaggtcagct gcaacccgcg gtgctcctcg 23340
   ttcagccagg tcttgcatac ggccgccaga gcttccactt ggtcaggcag tagtttgaag 23400
   ttcgccttta gatcgttatc cacgtggtac ttgtccatca gcgcgcgcgc agcctccatg 23460
   cccttctccc acgcagacac gatcggcaca ctcagcgggt tcatcaccgt aatttcactt 23520
   tccgcttcgc tgggctcttc ctcttcctct tgcgtccgca taccacgcgc cactgggtcg 23580
   tcttcattca gccgccgcac tgtgcgctta cctcctttgc catgcttgat tagcaccggt 23640
   gggttgctga aacccaccat ttgtagcgcc acatcttctc tttcttcctc gctgtccacg 23700
   attacctctg gtgatggcgg gcgctcgggc ttgggagaag ggcgcttctt tttcttcttg 23760
   ggcgcaatgg ccaaatccgc cgccgaggtc gatggccgcg ggctgggtgt gcgcggcacc 23820
   agcgcgtctt gtgatgagtc ttcctcgtcc tcggactcga tacgccgcct catccgcttt 23880
   tttgggggcg cccggggagg cggcggcgac ggggacgggg acgacacgtc ctccatggtt 23940
   gggggacgtc gcgccgcacc gcgtccgcgc tcgggggtgg tttcgcgctg ctcctcttcc 24000
   cgactggcca tttccttctc ctataggcag aaaaagatca tggagtcagt cgagaagaag 24060
   gacagcctaa ccgccccctc tgagttcgcc accaccgcct ccaccgatgc cgccaacgcg 24120
   cctaccacct tccccgtcga ggcacccccg cttgaggagg aggaagtgat tatcgagcag 24180
   gacccaggtt ttgtaagcga agacgacgag gaccgctcag taccaacaga ggataaaaag 24240
   caagaccagg acaacgcaga ggcaaacgag gaacaagtcg ggcgggggga cgaaaggcat 24300
   ggcgactacc tagatgtggg agacgacgtg ctgttgaagc atctgcagcg ccagtgcgcc 24360
   attatctgcg acgcgttgca agagcgcagc gatgtgcccc tcgccatagc ggatgtcagc 24420
   cttgcctacg aacgccacct attctcaccg cgcgtacccc ccaaacgcca agaaaacggc 24480
   acatgcgagc ccaacccgcg cctcaacttc taccccgtat ttgccgtgcc agaggtgctt 24540
   gccacctatc acatcttttt ccaaaactgc aagatacccc tatcctgccg tgccaaccgc 24600
   agccgagcgg acaagcagct ggccttgcgg cagggcgctg tcatacctga tatcgcctcg 24660
   ctcaacgaag tgccaaaaat ctttgagggt cttggacgcg acgagaagcg cgcggcaaac 24720
   gctctgcaac aggaaaacag cgaaaatgaa agtcactctg gagtgttggt ggaactcgag 24780
   ggtgacaacg cgcgcctagc cgtactaaaa cgcagcatcg aggtcaccca ctttgcctac 24840
   ccggcactta acctaccccc caaggtcatg agcacagtca tgagtgagct gatcgtgcgc 24900
   cgtgcgcagc ccctggagag ggatgcaaat ttgcaagaac aaacagagga gggcctaccc 24960
   gcagttggcg acgagcagct agcgcgctgg cttcaaacgc gcgagcctgc cgacttggag 25020
   gagcgacgca aactaatgat ggccgcagtg ctcgttaccg tggagcttga gtgcatgcag 25080
   cggttctttg ctgacccgga gatgcagcgc aagctagagg aaacattgca ctacaccttt 25140
   cgacagggct acgtacgcca ggcctgcaag atctccaacg tggagctctg caacctggtc 25200
   tcctaccttg gaattttgca cgaaaaccgc cttgggcaaa acgtgcttca ttccacgctc 25260
   aagggcgagg cgcgccgcga ctacgtccgc gactgcgttt acttatttct atgctacacc 25320
   tggcagacgg ccatgggcgt ttggcagcag tgcttggagg agtgcaacct caaggagctg 25380
   cagaaactgc taaagcaaaa cttgaaggac ctatggacgg ccttcaacga gcgctccgtg 25440
   gccgcgcacc tggcggacat cattttcccc gaacgcctgc ttaaaaccct gcaacagggt 25500
   ctgccagact tcaccagtca aagcatgttg cagaacttta ggaactttat cctagagcgc 25560
   tcaggaatct tgcccgccac ctgctgtgca cttcctagcg actttgtgcc cattaagtac 25620
   cgcgaatgcc ctccgccgct ttggggccac tgctaccttc tgcagctagc caactacctt 25680
   gcctaccact ctgacataat ggaagacgtg agcggtgacg gtctactgga gtgtcactgt 25740
   cgctgcaacc tatgcacccc gcaccgctcc ctggtttgca attcgcagct gcttaacgaa 25800
   agtcaaatta tcggtacctt tgagctgcag ggtccctcgc ctgacgaaaa gtccgcggct 25860
   ccggggttga aactcactcc ggggctgtgg acgtcggctt accttcgcaa atttgtacct 25920
   gaggactacc acgcccacga gattaggttc tacgaagacc aatcccgccc gcctaatgcg 25980
   gagcttaccg cctgcgtcat tacccagggc cacattcttg gccaattgca agccatcaac 26040
   aaagcccgcc aagagtttct gctacgaaag ggacgggggg tttacttgga cccccagtcc 26100
   ggcgaggagc tcaacccaat ccccccgccg ccgcagccct atcagcagca gccgcgggcc 26160
   cttgcttccc aggatggcac ccaaaaagaa gctgcagctg ccgccgccac ccacggacga 26220
   ggaggaatac tgggacagtc aggcagagga ggttttggac gaggaggagg aggacatgat 26280
   ggaagactgg gagagcctag acgaggaagc ttccgaggtc gaagaggtgt cagacgaaac 26340
   accgtcaccc tcggtcgcat tcccctcgcc ggcgccccag aaatcggcaa ccggttccag 26400
   catggctaca acctccgctc ctcaggcgcc gccggcactg cccgttcgcc gacccaaccg 26460
   tagatgggac accactggaa ccagggccgg taagtccaag cagccgccgc cgttagccca 26520
   agagcaacaa cagcgccaag gctaccgctc atggcgcggg cacaagaacg ccatagttgc 26580
   ttgcttgcaa gactgtgggg gcaacatctc cttcgcccgc cgctttcttc tctaccatca 26640
   cggcgtggcc ttcccccgta acatcctgca ttactaccgt catctctaca gcccatactg 26700
   caccggcggc agcggcagca acagcagcgg ccacacagaa gcaaaggcga ccggatagca 26760
   agactctgac aaagcccaag aaatccacag cggcggcagc agcaggagga ggagcgctgc 26820
   gtctggcgcc caacgaaccc gtatcgaccc gcgagcttag aaacaggatt tttcccactc 26880
   tgtatgctat atttcaacag agcaggggcc aagaacaaga gctgaaaata aaaaacaggt 26940
   ctctgcgatc cctcacccgc agctgcctgt atcacaaaag cgaagatcag cttcggcgca 27000
   cgctggaaga cgcggaggct ctcttcagta aatactgcgc gctgactctt aaggactagt 27060
   ttcgcgccct ttctcaaatt taagcgcgaa aactacgtca tctccagcgg ccacacccgg 27120
   cgccagcacc tgttgtcagc gccattatga gcaaggaaat tcccacgccc tacatgtgga 27180
   gttaccagcc acaaatggga cttgcggctg gagctgccca agactactca acccgaataa 27240
   actacatgag cgcgggaccc cacatgatat cccgggtcaa cggaatacgc gcccaccgaa 27300
   accgaattct cctggaacag gcggctatta ccaccacacc tcgtaataac cttaatcccc 27360
   gtagttggcc cgctgccctg gtgtaccagg aaagtcccgc tcccaccact gtggtacttc 27420
   ccagagacgc ccaggccgaa gttcagatga ctaactcagg ggcgcagctt gcgggcggct 27480
   ttcgtcacag ggtgcggtcg cccgggcagg gtataactca cctgacaatc agagggcgag 27540
   gtattcagct caacgacgag tcggtgagct cctcgcttgg tctccgtccg gacgggacat 27600
   ttcagatcgg cggcgccggc cgctcttcat tcacgcctcg tcaggcaatc ctaactctgc 27660
   agacctcgtc ctctgagccg cgctctggag gcattggaac tctgcaattt attgaggagt 27720
   ttgtgccatc ggtctacttt aaccccttct cgggacctcc cggccactat ccggatcaat 27780
   ttattcctaa ctttgacgcg gtaaaggact cggcggacgg ctacgactga atgttaagtg 27840
   gagaggcaga gcaactgcgc ctgaaacacc tggtccactg tcgccgccac aagtgctttg 27900
   cccgcgactc cggtgagttt tgctactttg aattgcccga ggatcatatc gagggcccgg 27960
   cgcacggcgt ccggcttacc gcccagggag agcttgcccg tagcctgatt cgggagttta 28020
   cccagcgccc cctgctagtt gagcgggaca ggggaccctg tgttctcact gtgatttgca 28080
   actgtcctaa ccctggatta catcaagatc cagatcttct agacccggga gcggccgctg 28140
   tcgacctgca ggatccgaat tcgatatcac tagttattaa tagtaatcaa ttacggggtc 28200
   attagttcat agcccatata tggagttccg cgttacataa cttacggtaa atggcccgcc 28260
   tggctgaccg cccaacgacc cccgcccatt gacgtcaata atgacgtatg ttcccatagt 28320
   aacgccaata gggactttcc attgacgtca atgggtggag tatttacggt aaactgccca 28380
   cttggcagta catcaagtgt atcatatgcc aagtccgccc cctattgacg tcaatgacgg 28440
   taaatggccc gcctggcatt atgcccagta catgacctta cgggactttc ctacttggca 28500
   gtacatctac gtattagtca tcgctattac catggtgatg cggttttggc agtacaccaa 28560
   tgggcgtgga tagcggtttg actcacgggg atttccaagt ctccacccca ttgacgtcaa 28620
   tgggagtttg ttttggcacc aaaatcaacg ggactttcca aaatgtcgta atacccccgc 28680
   cccgttgacg caaatgggca agcttgccgg gtcgaggtag gcgtgtacgg tgggaggcct 28740
   atataagcaa ccggtataat caaacagacc agattgtctg tttgttaccg gtgtttagtg 28800
   aaccgggcgc gcctcatatc gcctggagac gccatccacg ctgttttgac ctccatagaa 28860
   gacaccggga ccgatccagc ctccgcgggc cgccaccatg caaccaatcc tgcttctgct 28920
   ggccttcctc ctgctgccca gggcagatgc aggggagatc atcgggggac atgaggccaa 28980
   gccccactcc cgcccctaca tggcttatct tatgatctgg gatcagaagt ctctgaagag 29040
   gtgcggtggc ttcctgatac aagacgactt cgtgctgaca gctgctcact gttggggaag 29100
   ctccataaat gtcaccttgg gggcccacaa tatcaaagaa caggagccga cccagcagtt 29160
   tatccctgtg aaaagaccca tcccccatcc agcctataat cctaagaact tctccaacga 29220
   catcatgcta ctgcagctgg agagaaaggc caagcggacc agagctgtgc agcccctcag 29280
   gctacctagc aacaaggccc aggtgaagcc agggcagaca tgcagtgtgg ccggctgggg 29340
   gcagacggcc cccctgggaa aacactcaca cacactacaa gaggtgaaga tgacagtgca 29400
   ggaagatcga aagtgcgaat ctgacttacg ccattattac gacagtacca ttgagttgtg 29460
   cgtgggggac ccagagatta aaaagacttc ctttaagggg gactctggag gccctcttgt 29520
   gtgtaacaag gtggcccagg gcattgtctc ctatggacga aacaatggca tgcctccacg 29580
   agcctgcacc aaagtctcaa gctttgtaca ctggataaag aaaaccatga aacgccacta 29640
   aaacttgttt attgcagctt ataatggtta caaataaagc aatagcatca caaatttcac 29700
   aaataaagca tttttttcac tgcattctag ttgtggtttg tccaaactca tcaatgtatc 29760
   ttaactagtg gtaccgatct tattcccttt aactaataaa aaaaaataat aaagcatcac 29820
   ttacttaaaa tcagttagca aatttctgtc cagtttattc agcagcacct ccttgccctc 29880
   ctcccagctc tggtattgca gcttcctcct ggctgcaaac tttctccaca atctaaatgg 29940
   aatgtcagtt tcctcctgtt cctgtccatc cgcacccact atcttcatgt tgttgcagat 30000
   gaagcgcgca agaccgtctg aagatacctt caaccccgtg tatccatatg acacggaaac 30060
   cggtcctcca actgtgcctt ttcttactcc tccctttgta tcccccaatg ggtttcaaga 30120
   gagtccccct ggggtactct ctttgcgcct atccgaacct ctagttacct ccaatggcat 30180
   gcttgcgctc aaaatgggca acggcctctc tctggacgag gccggcaacc ttacctccca 30240
   aaatgtaacc actgtgagcc cacctctcaa aaaaaccaag tcaaacataa acctggaaat 30300
   atctgcaccc ctcacagtta cctcagaagc cctaactgtg gctgccgccg cacctctaat 30360
   ggtcgcgggc aacacactca ccatgcaatc acaggccccg ctaaccgtgc acgactccaa 30420
   acttagcatt gccacccaag gacccctcac agtgtcagaa ggaaagctag ccctgcaaac 30480
   atcaggcccc ctcaccacca ccgatagcag tacccttact atcactgcct caccccctct 30540
   aactactgcc actggtagct tgggcattga cttgaaagag cccatttata cacaaaatgg 30600
   aaaactagga ctaaagtacg gggctccttt gcatgtaaca gacgacctaa acactttgac 30660
   cgtagcaact ggtccaggtg tgactattaa taatacttcc ttgcaaacta aagttactgg 30720
   agccttgggt tttgattcac aaggcaatat gcaacttaat gtagcaggag gactaaggat 30780
   tgattctcaa aacagacgcc ttatacttga tgttagttat ccgtttgatg ctcaaaacca 30840
   actaaatcta agactaggac agggccctct ttttataaac tcagcccaca acttggatat 30900
   taactacaac aaaggccttt acttgtttac agcttcaaac aattccaaaa agcttgaggt 30960
   taacctaagc actgccaagg ggttgatgtt tgacgctaca gccatagcca ttaatgcagg 31020
   agatgggctt gaatttggtt cacctaatgc accaaacaca aatcccctca aaacaaaaat 31080
   tggccatggc ctagaatttg attcaaacaa ggctatggtt cctaaactag gaactggcct 31140
   tagttttgac agcacaggtg ccattacagt aggaaacaaa aataatgata agctaaccct 31200
   atggacaggt ccaaaaccag aagccaactg cataattgaa tacgggaaac aaaacccaga 31260
   tagcaaacta actttaatcc ttgtaaaaaa tggaggaatt gttaatggat atgtaacgct 31320
   aatgggagcc tcagactacg ttaacacctt atttaaaaac aaaaatgtct ccattaatgt 31380
   agaactatac tttgatgcca ctggtcatat attaccagac tcatcttctc ttaaaacaga 31440
   tctagaacta aaatacaagc aaaccgctga ctttagtgca agaggtttta tgccaagtac 31500
   tacagcgtat ccatttgtcc ttcctaatgc gggaacacat aatgaaaatt atattttggg 31560
   tcaatgctac tacaaagcaa gcgatggtgc cctttttccg ttggaagtta ctgttatgct 31620
   taataaacgc ctgccagata gtcgcacatc ctatgttatg acttttttat ggtccttgaa 31680
   tgctggtcta gctccagaaa ctactcaggc aaccctcata acctccccat ttaccttttc 31740
   ctatattaga gaagatgact aataaactct aaagaatcgt ttgtgttatg tttcaacgtg 31800
   tttatttttc aattgcagaa aatttcaagt catttttcat tcagtagtat agccccacca 31860
   ccacatagct tatacagatc accgtacctt aatcaaactc acagaaccct agtattcaac 31920
   ctgccacctc cctcccaaca cacagagtac acagtccttt ctccccggct ggccttaaaa 31980
   agcatcatat catgggtaac agacatattc ttaggtgtta tattccacac ggtttcctgt 32040
   cgagccaaac gctcatcagt gatattaata aactccccgg gcagctcact taagttcatg 32100
   tcgctgtcca gctgctgagc cacaggctgc tgtccaactt gcggttgctt aacgggcggc 32160
   gaaggagaag tccacgccta catgggggta gagtcataat cgtgcatcag gatagggcgg 32220
   tggtgctgca gcagcgcgcg aataaactgc tgccgccgcc gctccgtcct gcaggaatac 32280
   aacatggcag tggtctcctc agcgatgatt cgcaccgccc gcagcataag gcgccttgtc 32340
   ctccgggcac agcagcgcac cctgatctca cttaaatcag cacagtaact gcagcacagc 32400
   accacaatat tgttcaaaat cccacagtgc aaggcgctgt atccaaagct catggcgggg 32460
   accacagaac ccacgtggcc atcataccac aagcgcaggt agattaagtg gcgacccctc 32520
   ataaacacgc tggacataaa cattacctct tttggcatgt tgtaattcac cacctcccgg 32580
   taccatataa acctctgatt aaacatggcg ccatccacca ccatcctaaa ccagctggcc 32640
   aaaacctgcc cgccggctat acactgcagg gaaccgggac tggaacaatg acagtggaga 32700
   gcccaggact cgtaaccatg gatcatcatg ctcgtcatga tatcaatgtt ggcacaacac 32760
   aggcacacgt gcatacactt cctcaggatt acaagctcct cccgcgttag aaccatatcc 32820
   cagggaacaa cccattcctg aatcagcgta aatcccacac tgcagggaag acctcgcacg 32880
   taactcacgt tgtgcattgt caaagtgtta cattcgggca gcagcggatg atcctccagt 32940
   atggtagcgc gggtttctgt ctcaaaagga ggtagacgat ccctactgta cggagtgcgc 33000
   cgagacaacc gagatcgtgt tggtcgtagt gtcatgccaa atggaacgcc ggacgtagtc 33060
   atatttcctg aagcaaaacc aggtgcgggc gtgacaaaca gatctgcgtc tccggtctcg 33120
   ccgcttagat cgctctgtgt agtagttgta gtatatccac tctctcaaag catccaggcg 33180
   ccccctggct tcgggttcta tgtaaactcc ttcatgcgcc gctgccctga taacatccac 33240
   caccgcagaa taagccacac ccagccaacc tacacattcg ttctgcgagt cacacacggg 33300
   aggagcggga agagctggaa gaaccatgtt ttttttttta ttccaaaaga ttatccaaaa 33360
   cctcaaaatg aagatctatt aagtgaacgc gctcccctcc ggtggcgtgg tcaaactcta 33420
   cagccaaaga acagataatg gcatttgtaa gatgttgcac aatggcttcc aaaaggcaaa 33480
   cggccctcac gtccaagtgg acgtaaaggc taaacccttc agggtgaatc tcctctataa 33540
   acattccagc accttcaacc atgcccaaat aattctcatc tcgccacctt ctcaatatat 33600
   ctctaagcaa atcccgaata ttaagtccgg ccattgtaaa aatctgctcc agagcgccct 33660
   ccaccttcag cctcaagcag cgaatcatga ttgcaaaaat tcaggttcct cacagacctg 33720
   tataagattc aaaagcggaa cattaacaaa aataccgcga tcccgtaggt cccttcgcag 33780
   ggccagctga acataatcgt gcaggtctgc acggaccagc gcggccactt ccccgccagg 33840
   aaccatgaca aaagaaccca cactgattat gacacgcata ctcggagcta tgctaaccag 33900
   cgtagccccg atgtaagctt gttgcatggg cggcgatata aaatgcaagg tgctgctcaa 33960
   aaaatcaggc aaagcctcgc gcaaaaaaga aagcacatcg tagtcatgct catgcagata 34020
   aaggcaggta agctccggaa ccaccacaga aaaagacacc atttttctct caaacatgtc 34080
   tgcgggtttc tgcataaaca caaaataaaa taacaaaaaa acatttaaac attagaagcc 34140
   tgtcttacaa caggaaaaac aacccttata agcataagac ggactacggc catgccggcg 34200
   tgaccgtaaa aaaactggtc accgtgatta aaaagcacca ccgacagctc ctcggtcatg 34260
   tccggagtca taatgtaaga ctcggtaaac acatcaggtt gattcacatc ggtcagtgct 34320
   aaaaagcgac cgaaatagcc cgggggaata catacccgca ggcgtagaga caacattaca 34380
   gcccccatag gaggtataac aaaattaata ggagagaaaa acacataaac acctgaaaaa 34440
   ccctcctgcc taggcaaaat agcaccctcc cgctccagaa caacatacag cgcttccaca 34500
   gcggcagcca taacagtcag ccttaccagt aaaaaagaaa acctattaaa aaaacaccac 34560
   tcgacacggc accagctcaa tcagtcacag tgtaaaaaag ggccaagtgc agagcgagta 34620
   tatataggac taaaaaatga cgtaacggtt aaagtccaca aaaaacaccc agaaaaccgc 34680
   acgcgaacct acgcccagaa acgaaagcca aaaaacccac aacttcctca aatcgtcact 34740
   tccgttttcc cacgttacgt cacttcccat tttaagaaaa ctacaattcc caacacatac 34800
   aagttactcc gccctaaaac ctacgtcacc cgccccgttc ccacgccccg cgccacgtca 34860
   caaactccac cccctcatta tcatattggc ttcaatccaa aataaggtat attattgatg 34920
   atgttaat 34928

<210> 4
<211> 35852
<212> DNA
<213> Artificial Sequence

<220>
<223> Ad5/3-D24-PERF (TGX-02)

<400> 4
taacatcatc aataatatac cttattttgg attgaagcca atatgataat gagggggtgg 60

agtttgtgac gtggcgcggg gcgtgggaac ggggcgggtg acgtagtagt gtggcggaag 120

tgtgatgttg caagtgtggc ggaacacatg taagcgacgg atgtggcaaa agtgacgttt 180

ttggtgtgcg ccggtgtaca caggaagtga caattttcgc gcggttttag gcggatgttg 240

tagtaaattt gggcgtaacc gagtaagatt tggccatttt cgcgggaaaa ctgaataaga 300

ggaagtgaaa tctgaataat tttgtgttac tcatagcgcg taatatttgt ctagggccgc 360

ggggactttg accgtttacg tggagactcg cccaggtgtt tttctcaggt gttttccgcg 420

ttccgggtca aagttggcgt tttattatta tagtcagctg acgtgtagtg tatttatacc 480

cggtgagttc ctcaagaggc cactcttgag tgccagcgag tagagttttc tcctccgagc 540

cgctccgaca ccgggactga aaatgagaca tattatctgc cacggaggtg ttattaccga 600

agaaatggcc gccagtcttt tggaccagct gatcgaagag gtactggctg ataatcttcc 660

acctcctagc cattttgaac cacctaccct tcacgaactg tatgatttag acgtgacggc 720

ccccgaagat cccaacgagg aggcggtttc gcagattttt cccgactctg taatgttggc 780

ggtgcaggaa gggattgact tactcacttt tccgccggcg cccggttctc cggagccgcc 840

tcacctttcc cggcagcccg agcagccgga gcagagagcc ttgggtccgg tttctatgcc 900

aaaccttgta ccggaggtga tcgatccacc cagtgacgac gaggatgaag agggtgagga 960

gtttgtgtta gattatgtgg agcaccccgg gcacggttgc aggtcttgtc attatcaccg 1020

gaggaatacg ggggacccag atattatgtg ttcgctttgc tatatgagga cctgtggcat 1080

gtttgtctac agtaagtgaa aattatgggc agtgggtgat agagtggtgg gtttggtgtg 1140

gtaatttttt ttttaatttt tacagttttg tggtttaaag aattttgtat tgtgattttt 1200

ttaaaaggtc ctgtgtctga acctgagcct gagcccgagc cagaaccgga gcctgcaaga 1260

cctacccgcc gtcctaaaat ggcgcctgct atcctgagac gcccgacatc acctgtgtct 1320

agagaatgca atagtagtac ggatagctgt gactccggtc cttctaacac acctcctgag 1380

atacacccgg tggtcccgct gtgccccatt aaaccagttg ccgtgagagt tggtgggcgt 1440

cgccaggctg tggaatgtat cgaggacttg cttaacgagc ctgggcaacc tttggacttg 1500

agctgtaaac gccccaggcc ataaggtgta aacctgtgat tgcgtgtgtg gttaacgcct 1560

ttgtttgctg aatgagttga tgtaagttta ataaagggtg agataatgtt taacttgcat 1620

ggcgtgttaa atggggcggg gcttaaaggg tatataatgc gccgtgggct aatcttggtt 1680

acatctgacc tcatggaggc ttgggagtgt ttggaagatt tttctgctgt gcgtaacttg 1740

ctggaacaga gctctaacag tacctcttgg ttttggaggt ttctgtgggg ctcatcccag 1800

gcaaagttag tctgcagaat taaggaggat tacaagtggg aatttgaaga gcttttgaaa 1860

tcctgtggtg agctgtttga ttctttgaat ctgggtcacc aggcgctttt ccaagagaag 1920

gtcatcaaga ctttggattt ttccacaccg gggcgcgctg cggctgctgt tgcttttttg 1980

agttttataa aggataaatg gagcgaagaa acccatctga gcggggggta cctgctggat 2040

tttctggcca tgcatctgtg gagagcggtt gtgagacaca agaatcgcct gctactgttg 2100

tcttccgtcc gcccggcgat aataccgacg gaggagcagc agcagcagca ggaggaagcc 2160

aggcggcggc ggcaggagca gagcccatgg aacccgagag ccggcctgga ccctcgggaa 2220

tgaatgttgt acaggtggct gaactgtatc cagaactgag acgcattttg acaattacag 2280

aggatgggca ggggctaaag ggggtaaaga gggagcgggg ggcttgtgag gctacagagg 2340

aggctaggaa tctagctttt agcttaatga ccagacaccg tcctgagtgt attacttttc 2400

aacagatcaa ggataattgc gctaatgagc ttgatctgct ggcgcagaag tattccatag 2460

agcagctgac cacttactgg ctgcagccag gggatgattt tgaggaggct attagggtat 2520

atgcaaaggt ggcacttagg ccagattgca agtacaagat cagcaaactt gtaaatatca 2580

ggaattgttg ctacatttct gggaacgggg ccgaggtgga gatagatacg gaggataggg 2640

tggcctttag atgtagcatg ataaatatgt ggccgggggt gcttggcatg gacggggtgg 2700

ttattatgaa tgtaaggttt actggcccca attttagcgg tacggttttc ctggccaata 2760

ccaaccttat cctacacggt gtaagcttct atgggtttaa caatacctgt gtggaagcct 2820

ggaccgatgt aagggttcgg ggctgtgcct tttactgctg ctggaagggg gtggtgtgtc 2880

gccccaaaag cagggcttca attaagaaat gcctctttga aaggtgtacc ttgggtatcc 2940

tgtctgaggg taactccagg gtgcgccaca atgtggcctc cgactgtggt tgcttcatgc 3000

tagtgaaaag cgtggctgtg attaagcata acatggtatg tggcaactgc gaggacaggg 3060

cctctcagat gctgacctgc tcggacggca actgtcacct gctgaagacc attcacgtag 3120

ccagccactc tcgcaaggcc tggccagtgt ttgagcataa catactgacc cgctgttcct 3180

tgcatttggg taacaggagg ggggtgttcc taccttacca atgcaatttg agtcacacta 3240

agatattgct tgagcccgag agcatgtcca aggtgaacct gaacggggtg tttgacatga 3300

ccatgaagat ctggaaggtg ctgaggtacg atgagacccg caccaggtgc agaccctgcg 3360

agtgtggcgg taaacatatt aggaaccagc ctgtgatgct ggatgtgacc gaggagctga 3420

ggcccgatca cttggtgctg gcctgcaccc gcgctgagtt tggctctagc gatgaagata 3480

cagattgagg tactgaaatg tgtgggcgtg gcttaagggt gggaaagaat atataaggtg 3540

ggggtcttat gtagttttgt atctgttttg cagcagccgc cgccgccatg agcaccaact 3600

cgtttgatgg aagcattgtg agctcatatt tgacaacgcg catgccccca tgggccgggg 3660

tgcgtcagaa tgtgatgggc tccagcattg atggtcgccc cgtcctgccc gcaaactcta 3720

ctaccttgac ctacgagacc gtgtctggaa cgccgttgga gactgcagcc tccgccgccg 3780

cttcagccgc tgcagccacc gcccgcggga ttgtgactga ctttgctttc ctgagcccgc 3840

ttgcaagcag tgcagcttcc cgttcatccg cccgcgatga caagttgacg gctcttttgg 3900

cacaattgga ttctttgacc cgggaactta atgtcgtttc tcagcagctg ttggatctgc 3960

gccagcaggt ttctgccctg aaggcttcct cccctcccaa tgcggtttaa aacataaata 4020

aaaaaccaga ctctgtttgg atttggatca agcaagtgtc ttgctgtctt tatttagggg 4080

ttttgcgcgc gcggtaggcc cgggaccagc ggtctcggtc gttgagggtc ctgtgtattt 4140

tttccaggac gtggtaaagg tgactctgga tgttcagata catgggcata agcccgtctc 4200

tggggtggag gtagcaccac tgcagagctt catgctgcgg ggtggtgttg tagatgatcc 4260

agtcgtagca ggagcgctgg gcgtggtgcc taaaaatgtc tttcagtagc aagctgattg 4320

ccaggggcag gcccttggtg taagtgttta caaagcggtt aagctgggat gggtgcatac 4380

gtggggatat gagatgcatc ttggactgta tttttaggtt ggctatgttc ccagccatat 4440

ccctccgggg attcatgttg tgcagaacca ccagcacagt gtatccggtg cacttgggaa 4500

atttgtcatg tagcttagaa ggaaatgcgt ggaagaactt ggagacgccc ttgtgacctc 4560

caagattttc catgcattcg tccataatga tggcaatggg cccacgggcg gcggcctggg 4620

cgaagatatt tctgggatca ctaacgtcat agttgtgttc caggatgaga tcgtcatagg 4680

ccatttttac aaagcgcggg cggagggtgc cagactgcgg tataatggtt ccatccggcc 4740

caggggcgta gttaccctca cagatttgca tttcccacgc tttgagttca gatgggggga 4800

tcatgtctac ctgcggggcg atgaagaaaa cggtttccgg ggtaggggag atcagctggg 4860

aagaaagcag gttcctgagc agctgcgact taccgcagcc ggtgggcccg taaatcacac 4920

ctattaccgg ctgcaactgg tagttaagag agctgcagct gccgtcatcc ctgagcaggg 4980

gggccacttc gttaagcatg tccctgactc gcatgttttc cctgaccaaa tccgccagaa 5040

ggcgctcgcc gcccagcgat agcagttctt gcaaggaagc aaagtttttc aacggtttga 5100

gaccgtccgc cgtaggcatg cttttgagcg tttgaccaag cagttccagg cggtcccaca 5160

gctcggtcac ctgctctacg gcatctcgat ccagcatatc tcctcgtttc gcgggttggg 5220

gcggctttcg ctgtacggca gtagtcggtg ctcgtccaga cgggccaggg tcatgtcttt 5280

ccacgggcgc agggtcctcg tcagcgtagt ctgggtcacg gtgaaggggt gcgctccggg 5340

ctgcgcgctg gccagggtgc gcttgaggct ggtcctgctg gtgctgaagc gctgccggtc 5400

ttcgccctgc gcgtcggcca ggtagcattt gaccatggtg tcatagtcca gcccctccgc 5460

ggcgtggccc ttggcgcgca gcttgccctt ggaggaggcg ccgcacgagg ggcagtgcag 5520

acttttgagg gcgtagagct tgggcgcgag aaataccgat tccggggagt aggcatccgc 5580

gccgcaggcc ccgcagacgg tctcgcattc cacgagccag gtgagctctg gccgttcggg 5640

gtcaaaaacc aggtttcccc catgcttttt gatgcgtttc ttacctctgg tttccatgag 5700

ccggtgtcca cgctcggtga cgaaaaggct gtccgtgtcc ccgtatacag acttgagagg 5760

cctgtcctcg agcggtgttc cgcggtcctc ctcgtataga aactcggacc actctgagac 5820

aaaggctcgc gtccaggcca gcacgaagga ggctaagtgg gaggggtagc ggtcgttgtc 5880

cactaggggg tccactcgct ccagggtgtg aagacacatg tcgccctctt cggcatcaag 5940

gaaggtgatt ggtttgtagg tgtaggccac gtgaccgggt gttcctgaag gggggctata 6000

aaagggggtg ggggcgcgtt cgtcctcact ctcttccgca tcgctgtctg cgagggccag 6060

ctgttggggt gagtactccc tctgaaaagc gggcatgact tctgcgctaa gattgtcagt 6120

ttccaaaaac gaggaggatt tgatattcac ctggcccgcg gtgatgcctt tgagggtggc 6180

cgcatccatc tggtcagaaa agacaatctt tttgttgtca agcttggtgg caaacgaccc 6240

gtagagggcg ttggacagca acttggcgat ggagcgcagg gtttggtttt tgtcgcgatc 6300

ggcgcgctcc ttggccgcga tgtttagctg cacgtattcg cgcgcaacgc accgccattc 6360

gggaaagacg gtggtgcgct cgtcgggcac caggtgcacg cgccaaccgc ggttgtgcag 6420

ggtgacaagg tcaacgctgg tggctacctc tccgcgtagg cgctcgttgg tccagcagag 6480

gcggccgccc ttgcgcgagc agaatggcgg tagggggtct agctgcgtct cgtccggggg 6540

gtctgcgtcc acggtaaaga ccccgggcag caggcgcgcg tcgaagtagt ctatcttgca 6600

tccttgcaag tctagcgcct gctgccatgc gcgggcggca agcgcgcgct cgtatgggtt 6660

gagtggggga ccccatggca tggggtgggt gagcgcggag gcgtacatgc cgcaaatgtc 6720

gtaaacgtag aggggctctc tgagtattcc aagatatgta gggtagcatc ttccaccgcg 6780

gatgctggcg cgcacgtaat cgtatagttc gtgcgaggga gcgaggaggt cgggaccgag 6840

gttgctacgg gcgggctgct ctgctcggaa gactatctgc ctgaagatgg catgtgagtt 6900

ggatgatatg gttggacgct ggaagacgtt gaagctggcg tctgtgagac ctaccgcgtc 6960

acgcacgaag gaggcgtagg agtcgcgcag cttgttgacc agctcggcgg tgacctgcac 7020

gtctagggcg cagtagtcca gggtttcctt gatgatgtca tacttatcct gtcccttttt 7080

tttccacagc tcgcggttga ggacaaactc ttcgcggtct ttccagtact cttggatcgg 7140

aaacccgtcg gcctccgaac ggtaagagcc tagcatgtag aactggttga cggcctggta 7200

ggcgcagcat cccttttcta cgggtagcgc gtatgcctgc gcggccttcc ggagcgaggt 7260

gtgggtgagc gcaaaggtgt ccctgaccat gactttgagg tactggtatt tgaagtcagt 7320

gtcgtcgcat ccgccctgct cccagagcaa aaagtccgtg cgctttttgg aacgcggatt 7380

tggcagggcg aaggtgacat cgttgaagag tatctttccc gcgcgaggca taaagttgcg 7440

tgtgatgcgg aagggtcccg gcacctcgga acggttgtta attacctggg cggcgagcac 7500

gatctcgtca aagccgttga tgttgtggcc cacaatgtaa agttccaaga agcgcgggat 7560

gcccttgatg gaaggcaatt ttttaagttc ctcgtaggtg agctcttcag gggagctgag 7620

cccgtgctct gaaagggccc agtctgcaag atgagggttg gaagcgacga atgagctcca 7680

caggtcacgg gccattagca tttgcaggtg gtcgcgaaag gtcctaaact ggcgacctat 7740

ggccattttt tctggggtga tgcagtagaa ggtaagcggg tcttgttccc agcggtccca 7800

tccaaggttc gcggctaggt ctcgcgcggc agtcactaga ggctcatctc cgccgaactt 7860

catgaccagc atgaaggcca cgagctgctt cccaaaggcc cccatccaag tataggtctc 7920

tacatcgtag gtgacaaaga gacgctcggt gcgaggatgc gagccgatcg ggaagaactg 7980

gatctcccgc caccaattgg aggagtggct attgatgtgg tgaaagtaga agtccctgcg 8040

acgggccgaa cactcgtgct ggcttttgta aaaacgtgcg cagtactggc agcggtgcac 8100

gggctgtaca tcctgcacga ggttgacctg acgaccgcgc acaaggaagc agagtgggaa 8160

tttgagcccc tcgcctggcg ggtttggctg gtggtcttct acttcggctg cttgtccttg 8220

accgtctggc tgctcgaggg gagttacggt ggatcggacc accacgccgc gcgagcccaa 8280

agtccagatg tccgcgcgcg gcggtcggag cttgatgaca acatcgcgca gatgggagct 8340

gtccatggtc tggagctccc gcggcgtcag gtcaggcggg agctcctgca ggtttacctc 8400

gcatagacgg gtcagggcgc gggctagatc caggtgatac ctaatttcca ggggctggtt 8460

ggtggcggcg tcgatggctt gcaagaggcc gcatccccgc ggcgcgacta cggtaccgcg 8520

cggcgggcgg tgggccgcgg gggtgtcctt ggatgatgca tctaaaagcg gtgacgcggg 8580

cgagcccccg gaggtagggg gggctccgga cccgccggga gagggggcag gggcacgtcg 8640

gcgccgcgcg cgggcaggag ctggtgctgc gcgcgtaggt tgctggcgaa cgcgacgacg 8700

cggcggttga tctcctgaat ctggcgcctc tgcgtgaaga cgacgggccc ggtgagcttg 8760

aacctgaaag agagttcgac agaatcaatt tcggtgtcgt tgacggcggc ctggcgcaaa 8820

atctcctgca cgtctcctga gttgtcttga taggcgatct cggccatgaa ctgctcgatc 8880

tcttcctcct ggagatctcc gcgtccggct cgctccacgg tggcggcgag gtcgttggaa 8940

atgcgggcca tgagctgcga gaaggcgttg aggcctccct cgttccagac gcggctgtag 9000

accacgcccc cttcggcatc gcgggcgcgc atgaccacct gcgcgagatt gagctccacg 9060

tgccgggcga agacggcgta gtttcgcagg cgctgaaaga ggtagttgag ggtggtggcg 9120

gtgtgttctg ccacgaagaa gtacataacc cagcgtcgca acgtggattc gttgatatcc 9180

cccaaggcct caaggcgctc catggcctcg tagaagtcca cggcgaagtt gaaaaactgg 9240

gagttgcgcg ccgacacggt taactcctcc tccagaagac ggatgagctc ggcgacagtg 9300

tcgcgcacct cgcgctcaaa ggctacaggg gcctcttctt cttcttcaat ctcctcttcc 9360

ataagggcct ccccttcttc ttcttctggc ggcggtgggg gaggggggac acggcggcga 9420

cgacggcgca ccgggaggcg gtcgacaaag cgctcgatca tctccccgcg gcgacggcgc 9480

atggtctcgg tgacggcgcg gccgttctcg cgggggcgca gttggaagac gccgcccgtc 9540

atgtcccggt tatgggttgg cggggggctg ccatgcggca gggatacggc gctaacgatg 9600

catctcaaca attgttgtgt aggtactccg ccgccgaggg acctgagcga gtccgcatcg 9660

accggatcgg aaaacctctc gagaaaggcg tctaaccagt cacagtcgca aggtaggctg 9720

agcaccgtgg cgggcggcag cgggcggcgg tcggggttgt ttctggcgga ggtgctgctg 9780

atgatgtaat taaagtaggc ggtcttgaga cggcggatgg tcgacagaag caccatgtcc 9840

ttgggtccgg cctgctgaat gcgcaggcgg tcggccatgc cccaggcttc gttttgacat 9900

cggcgcaggt ctttgtagta gtcttgcatg agcctttcta ccggcacttc ttcttctcct 9960

tcctcttgtc ctgcatctct tgcatctatc gctgcggcgg cggcggagtt tggccgtagg 10020

tggcgccctc ttcctcccat gcgtgtgacc ccgaagcccc tcatcggctg aagcagggct 10080

aggtcggcga caacgcgctc ggctaatatg gcctgctgca cctgcgtgag ggtagactgg 10140

aagtcatcca tgtccacaaa gcggtggtat gcgcccgtgt tgatggtgta agtgcagttg 10200

gccataacgg accagttaac ggtctggtga cccggctgcg agagctcggt gtacctgaga 10260

cgcgagtaag ccctcgagtc aaatacgtag tcgttgcaag tccgcaccag gtactggtat 10320

cccaccaaaa agtgcggcgg cggctggcgg tagaggggcc agcgtagggt ggccggggct 10380

ccgggggcga gatcttccaa cataaggcga tgatatccgt agatgtacct ggacatccag 10440

gtgatgccgg cggcggtggt ggaggcgcgc ggaaagtcgc ggacgcggtt ccagatgttg 10500

cgcagcggca aaaagtgctc catggtcggg acgctctggc cggtcaggcg cgcgcaatcg 10560

ttgacgctct agaccgtgca aaaggagagc ctgtaagcgg gcactcttcc gtggtctggt 10620

ggataaattc gcaagggtat catggcggac gaccggggtt cgagccccgt atccggccgt 10680

ccgccgtgat ccatgcggtt accgcccgcg tgtcgaaccc aggtgtgcga cgtcagacaa 10740

cgggggagtg ctccttttgg cttccttcca ggcgcggcgg ctgctgcgct agcttttttg 10800

gccactggcc gcgcgcagcg taagcggtta ggctggaaag cgaaagcatt aagtggctcg 10860

ctccctgtag ccggagggtt attttccaag ggttgagtcg cgggaccccc ggttcgagtc 10920

tcggaccggc cggactgcgg cgaacggggg tttgcctccc cgtcatgcaa gaccccgctt 10980

gcaaattcct ccggaaacag ggacgagccc cttttttgct tttcccagat gcatccggtg 11040

ctgcggcaga tgcgcccccc tcctcagcag cggcaagagc aagagcagcg gcagacatgc 11100

agggcaccct cccctcctcc taccgcgtca ggaggggcga catccgcggt tgacgcggca 11160

gcagatggtg attacgaacc cccgcggcgc cgggcccggc actacctgga cttggaggag 11220

ggcgagggcc tggcgcggct aggagcgccc tctcctgagc ggcacccaag ggtgcagctg 11280

aagcgtgata cgcgtgaggc gtacgtgccg cggcagaacc tgtttcgcga ccgcgaggga 11340

gaggagcccg aggagatgcg ggatcgaaag ttccacgcag ggcgcgagct gcggcatggc 11400

ctgaatcgcg agcggttgct gcgcgaggag gactttgagc ccgacgcgcg aaccgggatt 11460

agtcccgcgc gcgcacacgt ggcggccgcc gacctggtaa ccgcatacga gcagacggtg 11520

aaccaggaga ttaactttca aaaaagcttt aacaaccacg tgcgtacgct tgtggcgcgc 11580

gaggaggtgg ctataggact gatgcatctg tgggactttg taagcgcgct ggagcaaaac 11640

ccaaatagca agccgctcat ggcgcagctg ttccttatag tgcagcacag cagggacaac 11700

gaggcattca gggatgcgct gctaaacata gtagagcccg agggccgctg gctgctcgat 11760

ttgataaaca tcctgcagag catagtggtg caggagcgca gcttgagcct ggctgacaag 11820

gtggccgcca tcaactattc catgcttagc ctgggcaagt tttacgcccg caagatatac 11880

catacccctt acgttcccat agacaaggag gtaaagatcg aggggttcta catgcgcatg 11940

gcgctgaagg tgcttacctt gagcgacgac ctgggcgttt atcgcaacga gcgcatccac 12000

aaggccgtga gcgtgagccg gcggcgcgag ctcagcgacc gcgagctgat gcacagcctg 12060

caaagggccc tggctggcac gggcagcggc gatagagagg ccgagtccta ctttgacgcg 12120

ggcgctgacc tgcgctgggc cccaagccga cgcgccctgg aggcagctgg ggccggacct 12180

gggctggcgg tggcacccgc gcgcgctggc aacgtcggcg gcgtggagga atatgacgag 12240

gacgatgagt acgagccaga ggacggcgag tactaagcgg tgatgtttct gatcagatga 12300

tgcaagacgc aacggacccg gcggtgcggg cggcgctgca gagccagccg tccggcctta 12360

actccacgga cgactggcgc caggtcatgg accgcatcat gtcgctgact gcgcgcaatc 12420

ctgacgcgtt ccggcagcag ccgcaggcca accggctctc cgcaattctg gaagcggtgg 12480

tcccggcgcg cgcaaacccc acgcacgaga aggtgctggc gatcgtaaac gcgctggccg 12540

aaaacagggc catccggccc gacgaggccg gcctggtcta cgacgcgctg cttcagcgcg 12600

tggctcgtta caacagcggc aacgtgcaga ccaacctgga ccggctggtg ggggatgtgc 12660

gcgaggccgt ggcgcagcgt gagcgcgcgc agcagcaggg caacctgggc tccatggttg 12720

cactaaacgc cttcctgagt acacagcccg ccaacgtgcc gcggggacag gaggactaca 12780

ccaactttgt gagcgcactg cggctaatgg tgactgagac accgcaaagt gaggtgtacc 12840

agtctgggcc agactatttt ttccagacca gtagacaagg cctgcagacc gtaaacctga 12900

gccaggcttt caaaaacttg caggggctgt ggggggtgcg ggctcccaca ggcgaccgcg 12960

cgaccgtgtc tagcttgctg acgcccaact cgcgcctgtt gctgctgcta atagcgccct 13020

tcacggacag tggcagcgtg tcccgggaca catacctagg tcacttgctg acactgtacc 13080

gcgaggccat aggtcaggcg catgtggacg agcatacttt ccaggagatt acaagtgtca 13140

gccgcgcgct ggggcaggag gacacgggca gcctggaggc aaccctaaac tacctgctga 13200

ccaaccggcg gcagaagatc ccctcgttgc acagtttaaa cagcgaggag gagcgcattt 13260

tgcgctacgt gcagcagagc gtgagcctta acctgatgcg cgacggggta acgcccagcg 13320

tggcgctgga catgaccgcg cgcaacatgg aaccgggcat gtatgcctca aaccggccgt 13380

ttatcaaccg cctaatggac tacttgcatc gcgcggccgc cgtgaacccc gagtatttca 13440

ccaatgccat cttgaacccg cactggctac cgccccctgg tttctacacc gggggattcg 13500

aggtgcccga gggtaacgat ggattcctct gggacgacat agacgacagc gtgttttccc 13560

cgcaaccgca gaccctgcta gagttgcaac agcgcgagca ggcagaggcg gcgctgcgaa 13620

aggaaagctt ccgcaggcca agcagcttgt ccgatctagg cgctgcggcc ccgcggtcag 13680

atgctagtag cccatttcca agcttgatag ggtctcttac cagcactcgc accacccgcc 13740

cgcgcctgct gggcgaggag gagtacctaa acaactcgct gctgcagccg cagcgcgaaa 13800

aaaacctgcc tccggcattt cccaacaacg ggatagagag cctagtggac aagatgagta 13860

gatggaagac gtacgcgcag gagcacaggg acgtgccagg cccgcgcccg cccacccgtc 13920

gtcaaaggca cgaccgtcag cggggtctgg tgtgggagga cgatgactcg gcagacgaca 13980

gcagcgtcct ggatttggga gggagtggca acccgtttgc gcaccttcgc cccaggctgg 14040

ggagaatgtt ttaaaaaaaa aaaaagcatg atgcaaaata aaaaactcac caaggccatg 14100

gcaccgagcg ttggttttct tgtattcccc ttagtatgcg gcgcgcggcg atgtatgagg 14160

aaggtcctcc tccctcctac gagagtgtgg tgagcgcggc gccagtggcg gcggcgctgg 14220

gttctccctt cgatgctccc ctggacccgc cgtttgtgcc tccgcggtac ctgcggccta 14280

ccggggggag aaacagcatc cgttactctg agttggcacc cctattcgac accacccgtg 14340

tgtacctggt ggacaacaag tcaacggatg tggcatccct gaactaccag aacgaccaca 14400

gcaactttct gaccacggtc attcaaaaca atgactacag cccgggggag gcaagcacac 14460

agaccatcaa tcttgacgac cggtcgcact ggggcggcga cctgaaaacc atcctgcata 14520

ccaacatgcc aaatgtgaac gagttcatgt ttaccaataa gtttaaggcg cgggtgatgg 14580

tgtcgcgctt gcctactaag gacaatcagg tggagctgaa atacgagtgg gtggagttca 14640

cgctgcccga gggcaactac tccgagacca tgaccataga ccttatgaac aacgcgatcg 14700

tggagcacta cttgaaagtg ggcagacaga acggggttct ggaaagcgac atcggggtaa 14760

agtttgacac ccgcaacttc agactggggt ttgaccccgt cactggtctt gtcatgcctg 14820

gggtatatac aaacgaagcc ttccatccag acatcatttt gctgccagga tgcggggtgg 14880

acttcaccca cagccgcctg agcaacttgt tgggcatccg caagcggcaa cccttccagg 14940

agggctttag gatcacctac gatgatctgg agggtggtaa cattcccgca ctgttggatg 15000

tggacgccta ccaggcgagc ttgaaagatg acaccgaaca gggcgggggt ggcgcaggcg 15060

gcagcaacag cagtggcagc ggcgcggaag agaactccaa cgcggcagcc gcggcaatgc 15120

agccggtgga ggacatgaac gatcatgcca ttcgcggcga cacctttgcc acacgggctg 15180

aggagaagcg cgctgaggcc gaagcagcgg ccgaagctgc cgcccccgct gcgcaacccg 15240

aggtcgagaa gcctcagaag aaaccggtga tcaaacccct gacagaggac agcaagaaac 15300

gcagttacaa cctaataagc aatgacagca ccttcaccca gtaccgcagc tggtaccttg 15360

catacaacta cggcgaccct cagaccggaa tccgctcatg gaccctgctt tgcactcctg 15420

acgtaacctg cggctcggag caggtctact ggtcgttgcc agacatgatg caagaccccg 15480

tgaccttccg ctccacgcgc cagatcagca actttccggt ggtgggcgcc gagctgttgc 15540

ccgtgcactc caagagcttc tacaacgacc aggccgtcta ctcccaactc atccgccagt 15600

ttacctctct gacccacgtg ttcaatcgct ttcccgagaa ccagattttg gcgcgcccgc 15660

cagcccccac catcaccacc gtcagtgaaa acgttcctgc tctcacagat cacgggacgc 15720

taccgctgcg caacagcatc ggaggagtcc agcgagtgac cattactgac gccagacgcc 15780

gcacctgccc ctacgtttac aaggccctgg gcatagtctc gccgcgcgtc ctatcgagcc 15840

gcactttttg agcaagcatg tccatcctta tatcgcccag caataacaca ggctggggcc 15900

tgcgcttccc aagcaagatg tttggcgggg ccaagaagcg ctccgaccaa cacccagtgc 15960

gcgtgcgcgg gcactaccgc gcgccctggg gcgcgcacaa acgcggccgc actgggcgca 16020

ccaccgtcga tgacgccatc gacgcggtgg tggaggaggc gcgcaactac acgcccacgc 16080

cgccaccagt gtccacagtg gacgcggcca ttcagaccgt ggtgcgcgga gcccggcgct 16140

atgctaaaat gaagagacgg cggaggcgcg tagcacgtcg ccaccgccgc cgacccggca 16200

ctgccgccca acgcgcggcg gcggccctgc ttaaccgcgc acgtcgcacc ggccgacggg 16260

cggccatgcg ggccgctcga aggctggccg cgggtattgt cactgtgccc cccaggtcca 16320

ggcgacgagc ggccgccgca gcagccgcgg ccattagtgc tatgactcag ggtcgcaggg 16380

gcaacgtgta ttgggtgcgc gactcggtta gcggcctgcg cgtgcccgtg cgcacccgcc 16440

ccccgcgcaa ctagattgca agaaaaaact acttagactc gtactgttgt atgtatccag 16500

cggcggcggc gcgcaacgaa gctatgtcca agcgcaaaat caaagaagag atgctccagg 16560

tcatcgcgcc ggagatctat ggccccccga agaaggaaga gcaggattac aagccccgaa 16620

agctaaagcg ggtcaaaaag aaaaagaaag atgatgatga tgaacttgac gacgaggtgg 16680

aactgctgca cgctaccgcg cccaggcgac gggtacagtg gaaaggtcga cgcgtaaaac 16740

gtgttttgcg acccggcacc accgtagtct ttacgcccgg tgagcgctcc acccgcacct 16800

acaagcgcgt gtatgatgag gtgtacggcg acgaggacct gcttgagcag gccaacgagc 16860

gcctcgggga gtttgcctac ggaaagcggc ataaggacat gctggcgttg ccgctggacg 16920

agggcaaccc aacacctagc ctaaagcccg taacactgca gcaggtgctg cccgcgcttg 16980

caccgtccga agaaaagcgc ggcctaaagc gcgagtctgg tgacttggca cccaccgtgc 17040

agctgatggt acccaagcgc cagcgactgg aagatgtctt ggaaaaaatg accgtggaac 17100

ctgggctgga gcccgaggtc cgcgtgcggc caatcaagca ggtggcgccg ggactgggcg 17160

tgcagaccgt ggacgttcag atacccacta ccagtagcac cagtattgcc accgccacag 17220

agggcatgga gacacaaacg tccccggttg cctcagcggt ggcggatgcc gcggtgcagg 17280

cggtcgctgc ggccgcgtcc aagacctcta cggaggtgca aacggacccg tggatgtttc 17340

gcgtttcagc cccccggcgc ccgcgccgtt cgaggaagta cggcgccgcc agcgcgctac 17400

tgcccgaata tgccctacat ccttccattg cgcctacccc cggctatcgt ggctacacct 17460

accgccccag aagacgagca actacccgac gccgaaccac cactggaacc cgccgccgcc 17520

gtcgccgtcg ccagcccgtg ctggccccga tttccgtgcg cagggtggct cgcgaaggag 17580

gcaggaccct ggtgctgcca acagcgcgct accaccccag catcgtttaa aagccggtct 17640

ttgtggttct tgcagatatg gccctcacct gccgcctccg tttcccggtg ccgggattcc 17700

gaggaagaat gcaccgtagg aggggcatgg ccggccacgg cctgacgggc ggcatgcgtc 17760

gtgcgcacca ccggcggcgg cgcgcgtcgc accgtcgcat gcgcggcggt atcctgcccc 17820

tccttattcc actgatcgcc gcggcgattg gcgccgtgcc cggaattgca tccgtggcct 17880

tgcaggcgca gagacactga ttaaaaacaa gttgcatgtg gaaaaatcaa aataaaaagt 17940

ctggactctc acgctcgctt ggtcctgtaa ctattttgta gaatggaaga catcaacttt 18000

gcgtctctgg ccccgcgaca cggctcgcgc ccgttcatgg gaaactggca agatatcggc 18060

accagcaata tgagcggtgg cgccttcagc tggggctcgc tgtggagcgg cattaaaaat 18120

ttcggttcca ccgttaagaa ctatggcagc aaggcctgga acagcagcac aggccagatg 18180

ctgagggata agttgaaaga gcaaaatttc caacaaaagg tggtagatgg cctggcctct 18240

ggcattagcg gggtggtgga cctggccaac caggcagtgc aaaataagat taacagtaag 18300

cttgatcccc gccctcccgt agaggagcct ccaccggccg tggagacagt gtctccagag 18360

gggcgtggcg aaaagcgtcc gcgccccgac agggaagaaa ctctggtgac gcaaatagac 18420

gagcctccct cgtacgagga ggcactaaag caaggcctgc ccaccacccg tcccatcgcg 18480

cccatggcta ccggagtgct gggccagcac acacccgtaa cgctggacct gcctcccccc 18540

gccgacaccc agcagaaacc tgtgctgcca ggcccgaccg ccgttgttgt aacccgtcct 18600

agccgcgcgt ccctgcgccg cgccgccagc ggtccgcgat cgttgcggcc cgtagccagt 18660

ggcaactggc aaagcacact gaacagcatc gtgggtctgg gggtgcaatc cctgaagcgc 18720

cgacgatgct tctgatagct aacgtgtcgt atgtgtgtca tgtatgcgtc catgtcgccg 18780

ccagaggagc tgctgagccg ccgcgcgccc gctttccaag atggctaccc cttcgatgat 18840

gccgcagtgg tcttacatgc acatctcggg ccaggacgcc tcggagtacc tgagccccgg 18900

gctggtgcag tttgcccgcg ccaccgagac gtacttcagc ctgaataaca agtttagaaa 18960

ccccacggtg gcgcctacgc acgacgtgac cacagaccgg tcccagcgtt tgacgctgcg 19020

gttcatccct gtggaccgtg aggatactgc gtactcgtac aaggcgcggt tcaccctagc 19080

tgtgggtgat aaccgtgtgc tggacatggc ttccacgtac tttgacatcc gcggcgtgct 19140

ggacaggggc cctactttta agccctactc tggcactgcc tacaacgccc tggctcccaa 19200

gggtgcccca aatccttgcg aatgggatga agctgctact gctcttgaaa taaacctaga 19260

agaagaggac gatgacaacg aagacgaagt agacgagcaa gctgagcagc aaaaaactca 19320

cgtatttggg caggcgcctt attctggtat aaatattaca aaggagggta ttcaaatagg 19380

tgtcgaaggt caaacaccta aatatgccga taaaacattt caacctgaac ctcaaatagg 19440

agaatctcag tggtacgaaa cagaaattaa tcatgcagct gggagagtcc taaaaaagac 19500

taccccaatg aaaccatgtt acggttcata tgcaaaaccc acaaatgaaa atggagggca 19560

aggcattctt gtaaagcaac aaaatggaaa gctagaaagt caagtggaaa tgcaattttt 19620

ctcaactact gaggcagccg caggcaatgg tgataacttg actcctaaag tggtattgta 19680

cagtgaagat gtagatatag aaaccccaga cactcatatt tcttacatgc ccactattaa 19740

ggaaggtaac tcacgagaac taatgggcca acaatctatg cccaacaggc ctaattacat 19800

tgcttttagg gacaatttta ttggtctaat gtattacaac agcacgggta atatgggtgt 19860

tctggcgggc caagcatcgc agttgaatgc tgttgtagat ttgcaagaca gaaacacaga 19920

gctttcatac cagcttttgc ttgattccat tggtgataga accaggtact tttctatgtg 19980

gaatcaggct gttgacagct atgatccaga tgttagaatt attgaaaatc atggaactga 20040

agatgaactt ccaaattact gctttccact gggaggtgtg attaatacag agactcttac 20100

caaggtaaaa cctaaaacag gtcaggaaaa tggatgggaa aaagatgcta cagaattttc 20160

agataaaaat gaaataagag ttggaaataa ttttgccatg gaaatcaatc taaatgccaa 20220

cctgtggaga aatttcctgt actccaacat agcgctgtat ttgcccgaca agctaaagta 20280

cagtccttcc aacgtaaaaa tttctgataa cccaaacacc tacgactaca tgaacaagcg 20340

agtggtggct cccgggctag tggactgcta cattaacctt ggagcacgct ggtcccttga 20400

ctatatggac aacgtcaacc catttaacca ccaccgcaat gctggcctgc gctaccgctc 20460

aatgttgctg ggcaatggtc gctatgtgcc cttccacatc caggtgcctc agaagttctt 20520

tgccattaaa aacctccttc tcctgccggg ctcatacacc tacgagtgga acttcaggaa 20580

ggatgttaac atggttctgc agagctccct aggaaatgac ctaagggttg acggagccag 20640

cattaagttt gatagcattt gcctttacgc caccttcttc cccatggccc acaacaccgc 20700

ctccacgctt gaggccatgc ttagaaacga caccaacgac cagtccttta acgactatct 20760

ctccgccgcc aacatgctct accctatacc cgccaacgct accaacgtgc ccatatccat 20820

cccctcccgc aactgggcgg ctttccgcgg ctgggccttc acgcgcctta agactaagga 20880

aaccccatca ctgggctcgg gctacgaccc ttattacacc tactctggct ctatacccta 20940

cctagatgga accttttacc tcaaccacac ctttaagaag gtggccatta cctttgactc 21000

ttctgtcagc tggcctggca atgaccgcct gcttaccccc aacgagtttg aaattaagcg 21060

ctcagttgac ggggagggtt acaacgttgc ccagtgtaac atgaccaaag actggttcct 21120

ggtacaaatg ctagctaact ataacattgg ctaccagggc ttctatatcc cagagagcta 21180

caaggaccgc atgtactcct tctttagaaa cttccagccc atgagccgtc aggtggtgga 21240

tgatactaaa tacaaggact accaacaggt gggcatccta caccaacaca acaactctgg 21300

atttgttggc taccttgccc ccaccatgcg cgaaggacag gcctaccctg ctaacttccc 21360

ctatccgctt ataggcaaga ccgcagttga cagcattacc cagaaaaagt ttctttgcga 21420

tcgcaccctt tggcgcatcc cattctccag taactttatg tccatgggcg cactcacaga 21480

cctgggccaa aaccttctct acgccaactc cgcccacgcg ctagacatga cttttgaggt 21540

ggatcccatg gacgagccca cccttcttta tgttttgttt gaagtctttg acgtggtccg 21600

tgtgcaccag ccgcaccgcg gcgtcatcga aaccgtgtac ctgcgcacgc ccttctcggc 21660

cggcaacgcc acaacataaa gaagcaagca acatcaacaa cagctgccgc catgggctcc 21720

agtgagcagg aactgaaagc cattgtcaaa gatcttggtt gtgggccata ttttttgggc 21780

acctatgaca agcgctttcc aggctttgtt tctccacaca agctcgcctg cgccatagtc 21840

aatacggccg gtcgcgagac tgggggcgta cactggatgg cctttgcctg gaacccgcac 21900

tcaaaaacat gctacctctt tgagcccttt ggcttttctg accagcgact caagcaggtt 21960

taccagtttg agtacgagtc actcctgcgc cgtagcgcca ttgcttcttc ccccgaccgc 22020

tgtataacgc tggaaaagtc cacccaaagc gtacaggggc ccaactcggc cgcctgtgga 22080

ctattctgct gcatgtttct ccacgccttt gccaactggc cccaaactcc catggatcac 22140

aaccccacca tgaaccttat taccggggta cccaactcca tgctcaacag tccccaggta 22200

cagcccaccc tgcgtcgcaa ccaggaacag ctctacagct tcctggagcg ccactcgccc 22260

tacttccgca gccacagtgc gcagattagg agcgccactt ctttttgtca cttgaaaaac 22320

atgtaaaaat aatgtactag agacactttc aataaaggca aatgctttta tttgtacact 22380

ctcgggtgat tatttacccc cacccttgcc gtctgcgccg tttaaaaatc aaaggggttc 22440

tgccgcgcat cgctatgcgc cactggcagg gacacgttgc gatactggtg tttagtgctc 22500

cacttaaact caggcacaac catccgcggc agctcggtga agttttcact ccacaggctg 22560

cgcaccatca ccaacgcgtt tagcaggtcg ggcgccgata tcttgaagtc gcagttgggg 22620

cctccgccct gcgcgcgcga gttgcgatac acagggttgc agcactggaa cactatcagc 22680

gccgggtggt gcacgctggc cagcacgctc ttgtcggaga tcagatccgc gtccaggtcc 22740

tccgcgttgc tcagggcgaa cggagtcaac tttggtagct gccttcccaa aaagggcgcg 22800

tgcccaggct ttgagttgca ctcgcaccgt agtggcatca aaaggtgacc gtgcccggtc 22860

tgggcgttag gatacagcgc ctgcataaaa gccttgatct gcttaaaagc cacctgagcc 22920

tttgcgcctt cagagaagaa catgccgcaa gacttgccgg aaaactgatt ggccggacag 22980

gccgcgtcgt gcacgcagca ccttgcgtcg gtgttggaga tctgcaccac atttcggccc 23040

caccggttct tcacgatctt ggccttgcta gactgctcct tcagcgcgcg ctgcccgttt 23100

tcgctcgtca catccatttc aatcacgtgc tccttattta tcataatgct tccgtgtaga 23160

cacttaagct cgccttcgat ctcagcgcag cggtgcagcc acaacgcgca gcccgtgggc 23220

tcgtgatgct tgtaggtcac ctctgcaaac gactgcaggt acgcctgcag gaatcgcccc 23280

atcatcgtca caaaggtctt gttgctggtg aaggtcagct gcaacccgcg gtgctcctcg 23340

ttcagccagg tcttgcatac ggccgccaga gcttccactt ggtcaggcag tagtttgaag 23400

ttcgccttta gatcgttatc cacgtggtac ttgtccatca gcgcgcgcgc agcctccatg 23460

cccttctccc acgcagacac gatcggcaca ctcagcgggt tcatcaccgt aatttcactt 23520

tccgcttcgc tgggctcttc ctcttcctct tgcgtccgca taccacgcgc cactgggtcg 23580

tcttcattca gccgccgcac tgtgcgctta cctcctttgc catgcttgat tagcaccggt 23640

gggttgctga aacccaccat ttgtagcgcc acatcttctc tttcttcctc gctgtccacg 23700

attacctctg gtgatggcgg gcgctcgggc ttgggagaag ggcgcttctt tttcttcttg 23760

ggcgcaatgg ccaaatccgc cgccgaggtc gatggccgcg ggctgggtgt gcgcggcacc 23820

agcgcgtctt gtgatgagtc ttcctcgtcc tcggactcga tacgccgcct catccgcttt 23880

tttgggggcg cccggggagg cggcggcgac ggggacgggg acgacacgtc ctccatggtt 23940

gggggacgtc gcgccgcacc gcgtccgcgc tcgggggtgg tttcgcgctg ctcctcttcc 24000

cgactggcca tttccttctc ctataggcag aaaaagatca tggagtcagt cgagaagaag 24060

gacagcctaa ccgccccctc tgagttcgcc accaccgcct ccaccgatgc cgccaacgcg 24120

cctaccacct tccccgtcga ggcacccccg cttgaggagg aggaagtgat tatcgagcag 24180

gacccaggtt ttgtaagcga agacgacgag gaccgctcag taccaacaga ggataaaaag 24240

caagaccagg acaacgcaga ggcaaacgag gaacaagtcg ggcgggggga cgaaaggcat 24300

ggcgactacc tagatgtggg agacgacgtg ctgttgaagc atctgcagcg ccagtgcgcc 24360

attatctgcg acgcgttgca agagcgcagc gatgtgcccc tcgccatagc ggatgtcagc 24420

cttgcctacg aacgccacct attctcaccg cgcgtacccc ccaaacgcca agaaaacggc 24480

acatgcgagc ccaacccgcg cctcaacttc taccccgtat ttgccgtgcc agaggtgctt 24540

gccacctatc acatcttttt ccaaaactgc aagatacccc tatcctgccg tgccaaccgc 24600

agccgagcgg acaagcagct ggccttgcgg cagggcgctg tcatacctga tatcgcctcg 24660

ctcaacgaag tgccaaaaat ctttgagggt cttggacgcg acgagaagcg cgcggcaaac 24720

gctctgcaac aggaaaacag cgaaaatgaa agtcactctg gagtgttggt ggaactcgag 24780

ggtgacaacg cgcgcctagc cgtactaaaa cgcagcatcg aggtcaccca ctttgcctac 24840

ccggcactta acctaccccc caaggtcatg agcacagtca tgagtgagct gatcgtgcgc 24900

cgtgcgcagc ccctggagag ggatgcaaat ttgcaagaac aaacagagga gggcctaccc 24960

gcagttggcg acgagcagct agcgcgctgg cttcaaacgc gcgagcctgc cgacttggag 25020

gagcgacgca aactaatgat ggccgcagtg ctcgttaccg tggagcttga gtgcatgcag 25080

cggttctttg ctgacccgga gatgcagcgc aagctagagg aaacattgca ctacaccttt 25140

cgacagggct acgtacgcca ggcctgcaag atctccaacg tggagctctg caacctggtc 25200

tcctaccttg gaattttgca cgaaaaccgc cttgggcaaa acgtgcttca ttccacgctc 25260

aagggcgagg cgcgccgcga ctacgtccgc gactgcgttt acttatttct atgctacacc 25320

tggcagacgg ccatgggcgt ttggcagcag tgcttggagg agtgcaacct caaggagctg 25380

cagaaactgc taaagcaaaa cttgaaggac ctatggacgg ccttcaacga gcgctccgtg 25440

gccgcgcacc tggcggacat cattttcccc gaacgcctgc ttaaaaccct gcaacagggt 25500

ctgccagact tcaccagtca aagcatgttg cagaacttta ggaactttat cctagagcgc 25560

tcaggaatct tgcccgccac ctgctgtgca cttcctagcg actttgtgcc cattaagtac 25620

cgcgaatgcc ctccgccgct ttggggccac tgctaccttc tgcagctagc caactacctt 25680

gcctaccact ctgacataat ggaagacgtg agcggtgacg gtctactgga gtgtcactgt 25740

cgctgcaacc tatgcacccc gcaccgctcc ctggtttgca attcgcagct gcttaacgaa 25800

agtcaaatta tcggtacctt tgagctgcag ggtccctcgc ctgacgaaaa gtccgcggct 25860

ccggggttga aactcactcc ggggctgtgg acgtcggctt accttcgcaa atttgtacct 25920

gaggactacc acgcccacga gattaggttc tacgaagacc aatcccgccc gcctaatgcg 25980

gagcttaccg cctgcgtcat tacccagggc cacattcttg gccaattgca agccatcaac 26040

aaagcccgcc aagagtttct gctacgaaag ggacgggggg tttacttgga cccccagtcc 26100

ggcgaggagc tcaacccaat ccccccgccg ccgcagccct atcagcagca gccgcgggcc 26160

cttgcttccc aggatggcac ccaaaaagaa gctgcagctg ccgccgccac ccacggacga 26220

ggaggaatac tgggacagtc aggcagagga ggttttggac gaggaggagg aggacatgat 26280

ggaagactgg gagagcctag acgaggaagc ttccgaggtc gaagaggtgt cagacgaaac 26340

accgtcaccc tcggtcgcat tcccctcgcc ggcgccccag aaatcggcaa ccggttccag 26400

catggctaca acctccgctc ctcaggcgcc gccggcactg cccgttcgcc gacccaaccg 26460

tagatgggac accactggaa ccagggccgg taagtccaag cagccgccgc cgttagccca 26520

agagcaacaa cagcgccaag gctaccgctc atggcgcggg cacaagaacg ccatagttgc 26580

ttgcttgcaa gactgtgggg gcaacatctc cttcgcccgc cgctttcttc tctaccatca 26640

cggcgtggcc ttcccccgta acatcctgca ttactaccgt catctctaca gcccatactg 26700

caccggcggc agcggcagca acagcagcgg ccacacagaa gcaaaggcga ccggatagca 26760

agactctgac aaagcccaag aaatccacag cggcggcagc agcaggagga ggagcgctgc 26820

gtctggcgcc caacgaaccc gtatcgaccc gcgagcttag aaacaggatt tttcccactc 26880

tgtatgctat atttcaacag agcaggggcc aagaacaaga gctgaaaata aaaaacaggt 26940

ctctgcgatc cctcacccgc agctgcctgt atcacaaaag cgaagatcag cttcggcgca 27000

cgctggaaga cgcggaggct ctcttcagta aatactgcgc gctgactctt aaggactagt 27060

ttcgcgccct ttctcaaatt taagcgcgaa aactacgtca tctccagcgg ccacacccgg 27120

cgccagcacc tgttgtcagc gccattatga gcaaggaaat tcccacgccc tacatgtgga 27180

gttaccagcc acaaatggga cttgcggctg gagctgccca agactactca acccgaataa 27240

actacatgag cgcgggaccc cacatgatat cccgggtcaa cggaatacgc gcccaccgaa 27300

accgaattct cctggaacag gcggctatta ccaccacacc tcgtaataac cttaatcccc 27360

gtagttggcc cgctgccctg gtgtaccagg aaagtcccgc tcccaccact gtggtacttc 27420

ccagagacgc ccaggccgaa gttcagatga ctaactcagg ggcgcagctt gcgggcggct 27480

ttcgtcacag ggtgcggtcg cccgggcagg gtataactca cctgacaatc agagggcgag 27540

gtattcagct caacgacgag tcggtgagct cctcgcttgg tctccgtccg gacgggacat 27600

ttcagatcgg cggcgccggc cgctcttcat tcacgcctcg tcaggcaatc ctaactctgc 27660

agacctcgtc ctctgagccg cgctctggag gcattggaac tctgcaattt attgaggagt 27720

ttgtgccatc ggtctacttt aaccccttct cgggacctcc cggccactat ccggatcaat 27780

ttattcctaa ctttgacgcg gtaaaggact cggcggacgg ctacgactga atgttaagtg 27840

gagaggcaga gcaactgcgc ctgaaacacc tggtccactg tcgccgccac aagtgctttg 27900

cccgcgactc cggtgagttt tgctactttg aattgcccga ggatcatatc gagggcccgg 27960

cgcacggcgt ccggcttacc gcccagggag agcttgcccg tagcctgatt cgggagttta 28020

cccagcgccc cctgctagtt gagcgggaca ggggaccctg tgttctcact gtgatttgca 28080

actgtcctaa ccctggatta catcaagatc cagatcttct agacccggga gcggccgctg 28140

tcgacctgca ggatccgaat tcgatatcac tagttattaa tagtaatcaa ttacggggtc 28200

attagttcat agcccatata tggagttccg cgttacataa cttacggtaa atggcccgcc 28260

tggctgaccg cccaacgacc cccgcccatt gacgtcaata atgacgtatg ttcccatagt 28320

aacgccaata gggactttcc attgacgtca atgggtggag tatttacggt aaactgccca 28380

cttggcagta catcaagtgt atcatatgcc aagtccgccc cctattgacg tcaatgacgg 28440

taaatggccc gcctggcatt atgcccagta catgacctta cgggactttc ctacttggca 28500

gtacatctac gtattagtca tcgctattac catggtgatg cggttttggc agtacaccaa 28560

tgggcgtgga tagcggtttg actcacgggg atttccaagt ctccacccca ttgacgtcaa 28620

tgggagtttg ttttggcacc aaaatcaacg ggactttcca aaatgtcgta atacccccgc 28680

cccgttgacg caaatgggca agcttgccgg gtcgaggtag gcgtgtacgg tgggaggcct 28740

atataagcaa ccggtataat caaacagacc agattgtctg tttgttaccg gtgtttagtg 28800

aaccgggcgc gcctcatatc gcctggagac gccatccacg ctgttttgac ctccatagaa 28860

gacaccggga ccgatccagc ctccgcgggc cgccaccatg gcagcccgtc tgctcctcct 28920

gggcatcctt ctcctgctgc tgcccctgcc cgtccctgcc ccgtgccaca cagccgcacg 28980

ctcagagtgc aagcgcagcc acaagttcgt gcctggtgca tggctggccg gggagggtgt 29040

ggacgtgacc agcctccgcc gctcgggctc cttcccagtg gacacacaaa ggttcctgcg 29100

gcccgacggc acctgcaccc tctgtgaaaa tgccctacag gagggcaccc tccagcgcct 29160

gcctctggcg ctcaccaact ggcgggccca gggctctggc tgccagcgcc atgtaaccag 29220

ggccaaagtc agctccactg aagctgtggc ccgggatgcg gctcgtagca tccgcaacga 29280

ctggaaggtc gggctggacg tgactcctaa gcccaccagc aatgtgcatg tgtctgtggc 29340

cggctcacac tcacaggcag ccaactttgc agcccagaag acccaccagg accagtacag 29400

cttcagcact gacacggtgg agtgccgctt ctacagtttc catgtggtac acactccccc 29460

gctgcaccct gacttcaaga gggccctcgg ggacctgccc caccacttca acgcctccac 29520

ccagcccgcc tacctcaggc ttatctccaa ctacggcacc cacttcatcc gggctgtgga 29580

gctgggtggc cgcatatcgg ccctcactgc cctgcgcacc tgcgagctgg ccctggaagg 29640

gctcacggac aacgaggtgg aggactgcct gactgtcgag gcccaggtca acataggcat 29700

ccacggcagc atctctgccg aagccaaggc ctgtgaggag aagaagaaga agcacaagat 29760

gacggcctcc ttccaccaaa cctaccggga gcgccattcg gaagtggttg gcggccatca 29820

cacctccatt aacgacctgc tgttcgggat ccaggccggg cccgagcagt actcagcctg 29880

ggtaaactcg ctgcccggca gccctggcct ggtggactac accctggaac ccctgcacgt 29940

gctgctggac agccaggacc cgcggcggga ggcactgagg agggccctga gtcagtacct 30000

gacggacagg gctcgctgga gggactgcag ccggccgtgc ccaccagggc ggcagaagag 30060

cccccgagac ccatgccagt gtgtgtgcca tggctcagcg gtcaccaccc aggactgctg 30120

ccctcggcag aggggcctgg cccagctgga ggtgaccttc atccaagcat ggggcctgtg 30180

gggggactgg ttcactgcca cggatgccta tgtgaagctc ttctttggtg gccaggagct 30240

gaggacgagc accgtgtggg acaataacaa ccccatctgg tcagtgcggc tggattttgg 30300

ggatgtgctc ctggccacag gggggcccct gaggttgcag gtctgggatc aggactctgg 30360

cagggacgat gacctccttg gcacctgtga tcaggctccc aagtctggtt cccatgaggt 30420

gagatgcaac ctgaatcatg gccacctaaa attccgctat catgccaggt gcttgcccca 30480

cctgggagga ggcacctgcc tggactatgt cccccaaatg cttctggggg agcctccagg 30540

aaaccggagt ggggccgtgt ggtgaaactt gtttattgca gcttataatg gttacaaata 30600

aagcaatagc atcacaaatt tcacaaataa agcatttttt tcactgcatt ctagttgtgg 30660

tttgtccaaa ctcatcaatg tatcttaact agtggtaccg atcttattcc ctttaactaa 30720
taaaaaaaaa taataaagca tcacttactt aaaatcagtt agcaaatttc tgtccagttt 30780
attcagcagc acctccttgc cctcctccca gctctggtat tgcagcttcc tcctggctgc 30840
aaactttctc cacaatctaa atggaatgtc agtttcctcc tgttcctgtc catccgcacc 30900
cactatcttc atgttgttgc agatgaagcg cgcaagaccg tctgaagata ccttcaaccc 30960
cgtgtatcca tatgacacgg aaaccggtcc tccaactgtg ccttttctta ctcctccctt 31020
tgtatccccc aatgggtttc aagagagtcc ccctggggta ctctctttgc gcctatccga 31080
acctctagtt acctccaatg gcatgcttgc gctcaaaatg ggcaacggcc tctctctgga 31140
cgaggccggc aaccttacct cccaaaatgt aaccactgtg agcccacctc tcaaaaaaac 31200
caagtcaaac ataaacctgg aaatatctgc acccctcaca gttacctcag aagccctaac 31260
tgtggctgcc gccgcacctc taatggtcgc gggcaacaca ctcaccatgc aatcacaggc 31320
cccgctaacc gtgcacgact ccaaacttag cattgccacc caaggacccc tcacagtgtc 31380
agaaggaaag ctagccctgc aaacatcagg ccccctcacc accaccgata gcagtaccct 31440
tactatcact gcctcacccc ctctaactac tgccactggt agcttgggca ttgacttgaa 31500
agagcccatt tatacacaaa atggaaaact aggactaaag tacggggctc ctttgcatgt 31560
aacagacgac ctaaacactt tgaccgtagc aactggtcca ggtgtgacta ttaataatac 31620
ttccttgcaa actaaagtta ctggagcctt gggttttgat tcacaaggca atatgcaact 31680
taatgtagca ggaggactaa ggattgattc tcaaaacaga cgccttatac ttgatgttag 31740
ttatccgttt gatgctcaaa accaactaaa tctaagacta ggacagggcc ctctttttat 31800
aaactcagcc cacaacttgg atattaacta caacaaaggc ctttacttgt ttacagcttc 31860
aaacaattcc aaaaagcttg aggttaacct aagcactgcc aaggggttga tgtttgacgc 31920
tacagccata gccattaatg caggagatgg gcttgaattt ggttcaccta atgcaccaaa 31980
cacaaatccc ctcaaaacaa aaattggcca tggcctagaa tttgattcaa acaaggctat 32040
ggttcctaaa ctaggaactg gccttagttt tgacagcaca ggtgccatta cagtaggaaa 32100
caaaaataat gataagctaa ccctatggac aggtccaaaa ccagaagcca actgcataat 32160
tgaatacggg aaacaaaacc cagatagcaa actaacttta atccttgtaa aaaatggagg 32220
aattgttaat ggatatgtaa cgctaatggg agcctcagac tacgttaaca ccttatttaa 32280
aaacaaaaat gtctccatta atgtagaact atactttgat gccactggtc atatattacc 32340
agactcatct tctcttaaaa cagatctaga actaaaatac aagcaaaccg ctgactttag 32400
tgcaagaggt tttatgccaa gtactacagc gtatccattt gtccttccta atgcgggaac 32460
acataatgaa aattatattt ttggtcaatg ctactacaaa gcaagcgatg gtgccctttt 32520
tccgttggaa gttactgtta tgcttaataa acgcctgcca gatagtcgca catcctatgt 32580

tatgactttt ttatggtcct tgaatgctgg tctagctcca gaaactactc aggcaaccct 32640

cataacctcc ccatttacct tttcctatat tagagaagat gactaataaa ctctaaagaa 32700

tcgtttgtgt tatgtttcaa cgtgtttatt tttcaattgc agaaaatttc aagtcatttt 32760

tcattcagta gtatagcccc accaccacat agcttataca gatcaccgta ccttaatcaa 32820

actcacagaa ccctagtatt caacctgcca cctccctccc aacacacaga gtacacagtc 32880

ctttctcccc ggctggcctt aaaaagcatc atatcatggg taacagacat attcttaggt 32940

gttatattcc acacggtttc ctgtcgagcc aaacgctcat cagtgatatt aataaactcc 33000

ccgggcagct cacttaagtt catgtcgctg tccagctgct gagccacagg ctgctgtcca 33060

acttgcggtt gcttaacggg cggcgaagga gaagtccacg cctacatggg ggtagagtca 33120

taatcgtgca tcaggatagg gcggtggtgc tgcagcagcg cgcgaataaa ctgctgccgc 33180

cgccgctccg tcctgcagga atacaacatg gcagtggtct cctcagcgat gattcgcacc 33240

gcccgcagca taaggcgcct tgtcctccgg gcacagcagc gcaccctgat ctcacttaaa 33300

tcagcacagt aactgcagca cagcaccaca atattgttca aaatcccaca gtgcaaggcg 33360

ctgtatccaa agctcatggc ggggaccaca gaacccacgt ggccatcata ccacaagcgc 33420

aggtagatta agtggcgacc cctcataaac acgctggaca taaacattac ctcttttggc 33480

atgttgtaat tcaccacctc ccggtaccat ataaacctct gattaaacat ggcgccatcc 33540

accaccatcc taaaccagct ggccaaaacc tgcccgccgg ctatacactg cagggaaccg 33600

ggactggaac aatgacagtg gagagcccag gactcgtaac catggatcat catgctcgtc 33660

atgatatcaa tgttggcaca acacaggcac acgtgcatac acttcctcag gattacaagc 33720

tcctcccgcg ttagaaccat atcccaggga acaacccatt cctgaatcag cgtaaatccc 33780

acactgcagg gaagacctcg cacgtaactc acgttgtgca ttgtcaaagt gttacattcg 33840

ggcagcagcg gatgatcctc cagtatggta gcgcgggttt ctgtctcaaa aggaggtaga 33900

cgatccctac tgtacggagt gcgccgagac aaccgagatc gtgttggtcg tagtgtcatg 33960

ccaaatggaa cgccggacgt agtcatattt cctgaagcaa aaccaggtgc gggcgtgaca 34020

aacagatctg cgtctccggt ctcgccgctt agatcgctct gtgtagtagt tgtagtatat 34080

ccactctctc aaagcatcca ggcgccccct ggcttcgggt tctatgtaaa ctccttcatg 34140

cgccgctgcc ctgataacat ccaccaccgc agaataagcc acacccagcc aacctacaca 34200

ttcgttctgc gagtcacaca cgggaggagc gggaagagct ggaagaacca tgtttttttt 34260

tttattccaa aagattatcc aaaacctcaa aatgaagatc tattaagtga acgcgctccc 34320

ctccggtggc gtggtcaaac tctacagcca aagaacagat aatggcattt gtaagatgtt 34380

gcacaatggc ttccaaaagg caaacggccc tcacgtccaa gtggacgtaa aggctaaacc 34440

cttcagggtg aatctcctct ataaacattc cagcaccttc aaccatgccc aaataattct 34500

catctcgcca ccttctcaat atatctctaa gcaaatcccg aatattaagt ccggccattg 34560

taaaaatctg ctccagagcg ccctccacct tcagcctcaa gcagcgaatc atgattgcaa 34620

aaattcaggt tcctcacaga cctgtataag attcaaaagc ggaacattaa caaaaatacc 34680

gcgatcccgt aggtcccttc gcagggccag ctgaacataa tcgtgcaggt ctgcacggac 34740

cagcgcggcc acttccccgc caggaaccat gacaaaagaa cccacactga ttatgacacg 34800

catactcgga gctatgctaa ccagcgtagc cccgatgtaa gcttgttgca tgggcggcga 34860

tataaaatgc aaggtgctgc tcaaaaaatc aggcaaagcc tcgcgcaaaa aagaaagcac 34920

atcgtagtca tgctcatgca gataaaggca ggtaagctcc ggaaccacca cagaaaaaga 34980

caccattttt ctctcaaaca tgtctgcggg tttctgcata aacacaaaat aaaataacaa 35040

aaaaacattt aaacattaga agcctgtctt acaacaggaa aaacaaccct tataagcata 35100

agacggacta cggccatgcc ggcgtgaccg taaaaaaact ggtcaccgtg attaaaaagc 35160

accaccgaca gctcctcggt catgtccgga gtcataatgt aagactcggt aaacacatca 35220

ggttgattca catcggtcag tgctaaaaag cgaccgaaat agcccggggg aatacatacc 35280

cgcaggcgta gagacaacat tacagccccc ataggaggta taacaaaatt aataggagag 35340

aaaaacacat aaacacctga aaaaccctcc tgcctaggca aaatagcacc ctcccgctcc 35400

agaacaacat acagcgcttc cacagcggca gccataacag tcagccttac cagtaaaaaa 35460

gaaaacctat taaaaaaaca ccactcgaca cggcaccagc tcaatcagtc acagtgtaaa 35520

aaagggccaa gtgcagagcg agtatatata ggactaaaaa atgacgtaac ggttaaagtc 35580

cacaaaaaac acccagaaaa ccgcacgcga acctacgccc agaaacgaaa gccaaaaaac 35640

ccacaacttc ctcaaatcgt cacttccgtt ttcccacgtt acgtcacttc ccattttaag 35700

aaaactacaa ttcccaacac atacaagtta ctccgcccta aaacctacgt cacccgcccc 35760

gttcccacgc cccgcgccac gtcacaaact ccaccccctc attatcatat tggcttcaat 35820

ccaaaataag gtatattatt gatgatgtta at 35852

<210> 5
<211> 8
<212> PRT
<213> Artificial Sequence

<220>
<223> Binding site for Rb protein in E1A

<400> 5
Leu Thr Cys His Glu Ala Gly Phe
1               5

<210> 6

<211> 8
<212> PRT
<213> Artificial Sequence

<220>
<223> Deleted sequence of the CR2 region

<400> 6
Leu Thr Cys His Glu Ala Gly Phe
1               5

<210> 7
<211> 45
<212> DNA
<213> Artificial Sequence

<220>
<223> CR2-D24 (TGX-01)

<400> 7
cttgtaccgg aggtgatcga tccacccagt gacgacgagg atgaa                    45

<210> 8
<211> 1764
<212> DNA
<213> Artificial Sequence

<220>
<223> Ad5/3 fiber (TGX-01)

<400> 8
atgaagcgcg caagaccgtc tgaagatacc ttcaaccccg tgtatccata tgacacggaa      60

accggtcctc caactgtgcc ttttcttact cctccctttg tatcccccaa tgggtttcaa     120

gagagtcccc ctggggtact ctctttgcgc ctatccgaac ctctagttac ctccaatggc     180

atgcttgcgc tcaaaatggg caacggcctc tctctggacg aggccggcaa ccttacctcc     240

caaaatgtaa ccactgtgag cccacctctc aaaaaaacca agtcaaacat aaacctggaa     300

atatctgcac ccctcacagt tacctcagaa gccctaactg tggctgccgc cgcacctcta     360

atggtcgcgg gcaacacact caccatgcaa tcacaggccc cgctaaccgt gcacgactcc     420

aaacttagca ttgccaccca aggacccctc acagtgtcag aaggaaagct agccctgcaa     480

acatcaggcc ccctcaccac caccgatagc agtaccctta ctatcactgc ctcaccccct     540

ctaactactg ccactggtag cttgggcatt gacttgaaag agcccattta tacacaaaat     600

ggaaaactag gactaaagta cggggctcct ttgcatgtaa cagacgacct aaacactttg     660

accgtagcaa ctggtccagg tgtgactatt aataatactt ccttgcaaac taaagttact     720

ggagccttgg gttttgattc acaaggcaat atgcaactta atgtagcagg aggactaagg     780

attgattctc aaaacagacg ccttatactt gatgttagtt atccgtttga tgctcaaaac     840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 9
<211> 530
<212> DNA
<213> Artificial Sequence

<220>
<223> CMV promoter/enhancer (TGX-01)

<400> 9
ctagttatta atagtaatca attacggggt cattagttca tagcccatat atggagttcc 60

gcgttacata acttacggta aatggcccgc ctggctgacc gcccaacgac ccccgcccat 120

tgacgtcaat aatgacgtat gttcccatag taacgccaat agggactttc cattgacgtc 180

aatgggtgga gtatttacgg taaactgccc acttggcagt acatcaagtg tatcatatgc 240

caagtccgcc ccctattgac gtcaatgacg gtaaatggcc cgcctggcat tatgcccagt 300

acatgacctt acgggacttt cctacttggc agtacatcta cgtattagtc atcgctatta 360

ccatggtgat gcggttttgg cagtacacca atgggcgtgg atagcggttt gactcacggg 420

gatttccaag tctccacccc attgacgtca atgggagttt gttttggcac caaaatcaac 480

gggactttcc aaaatgtcgt aatacccccg ccccgttgac gcaaatgggc 530

<210> 10

<211> 26
<212> DNA
<213> Artificial Sequence

<220>
<223> CUO (TGX-01)

<400> 10
caaacagacc agattgtctg tttgtt                                            26

<210> 11
<211> 75
<212> DNA
<213> Artificial Sequence

<220>
<223> CMV 5\222UTR (TGX-01)

<400> 11
tcatatcgcc tggagacgcc atccacgctg ttttgacctc catagaagac accgggaccg      60

atccagcctc cgcgg                                                        75

<210> 12
<211> 744
<212> DNA
<213> Artificial Sequence

<220>
<223> GRB (TGX-01)

<400> 12
atgcaaccaa tcctgcttct gctggccttc ctcctgctgc ccagggcaga tgcaggggag      60

atcatcgggg gacatgaggc caagccccac tcccgcccct acatggctta tcttatgatc     120

tgggatcaga agtctctgaa gaggtgcggt ggcttcctga tacaagacga cttcgtgctg     180

acagctgctc actgttgggg aagctccata aatgtcacct tgggggccca caatatcaaa     240

gaacaggagc cgacccagca gtttatccct gtgaaaagac ccatccccca tccagcctat     300

aatcctaaga acttctccaa cgacatcatg ctactgcagc tggagagaaa ggccaagcgg     360

accagagctg tgcagcccct caggctacct agcaacaagg cccaggtgaa gccagggcag     420

acatgcagtg tggccggctg ggggcagacg gcccccctgg gaaaacactc acacacacta     480

caagaggtga agatgacagt gcaggaagat cgaaagtgcg aatctgactt acgccattat     540

tacgacagta ccattgagtt gtgcgtgggg gacccagaga ttaaaaagac ttcctttaag     600

ggggactctg gaggccctct tgtgtgtaac aaggtggccc agggcattgt ctcctatgga     660

cgaaacaatg gcatgcctcc acgagcctgc accaaagtct caagctttgt acactggata     720

aagaaaacca tgaaacgcca ctaa                                              744

<210> 13
<211> 122
<212> DNA
<213> Artificial Sequence

<220>
<223> SV40 poly(A) signal (TGX-01)

<400> 13
aacttgttta ttgcagctta taatggttac aaataaagca atagcatcac aaatttcaca 60

aataaagcat ttttttcact gcattctagt tgtggtttgt ccaaactcat caatgtatct 120

ta 122

<210> 14
<211> 45
<212> DNA
<213> Artificial Sequence

<220>
<223> CR2-D24 (TGX-02)

<400> 14
cttgtaccgg aggtgatcga tccacccagt gacgacgagg atgaa 45

<210> 15
<211> 1764
<212> DNA
<213> Artificial Sequence

<220>
<223> Ad5/3 fiber (TGX-02)

<400> 15
atgaagcgcg caagaccgtc tgaagatacc ttcaaccccg tgtatccata tgacacggaa 60

accggtcctc caactgtgcc ttttcttact cctccctttg tatcccccaa tgggtttcaa 120

gagagtcccc ctggggtact ctctttgcgc ctatccgaac ctctagttac ctccaatggc 180

atgcttgcgc tcaaaatggg caacggcctc tctctggacg aggccggcaa ccttacctcc 240

caaaatgtaa ccactgtgag cccacctctc aaaaaaacca agtcaaacat aaacctggaa 300

atatctgcac ccctcacagt tacctcagaa gccctaactg tggctgccgc cgcacctcta 360

atggtcgcgg gcaacacact caccatgcaa tcacaggccc cgctaaccgt gcacgactcc 420

aaacttagca ttgccaccca aggacccctc acagtgtcag aaggaaagct agccctgcaa 480

acatcaggcc ccctcaccac caccgatagc agtaccctta ctatcactgc ctcaccccct 540

ctaactactg ccactggtag cttgggcatt gacttgaaag agcccattta tacacaaaat 600

ggaaaactag gactaaagta cggggctcct ttgcatgtaa cagacgacct aaacactttg 660

accgtagcaa ctggtccagg tgtgactatt aataatactt ccttgcaaac taaagttact 720

ggagccttgg gttttgattc acaaggcaat atgcaactta atgtagcagg aggactaagg 780

attgattctc aaaacagacg ccttatactt gatgttagtt atccgtttga tgctcaaaac 840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 16
<211> 530
<212> DNA
<213> Artificial Sequence

<220>
<223> CMV promoter/enhancer (TGX-02)

<400> 16
ctagttatta atagtaatca attacggggt cattagttca tagcccatat atggagttcc 60

gcgttacata acttacggta aatggcccgc ctggctgacc gcccaacgac ccccgcccat 120

tgacgtcaat aatgacgtat gttcccatag taacgccaat agggactttc cattgacgtc 180

aatgggtgga gtatttacgg taaactgccc acttggcagt acatcaagtg tatcatatgc 240

caagtccgcc ccctattgac gtcaatgacg gtaaatggcc cgcctggcat tatgcccagt 300

acatgacctt acgggacttt cctacttggc agtacatcta cgtattagtc atcgctatta 360

ccatggtgat gcggttttgg cagtacacca atgggcgtgg atagcggttt gactcacggg 420

gatttccaag tctccacccc attgacgtca atgggagttt gttttggcac caaaatcaac 480

gggactttcc aaaatgtcgt aataaccccg ccccgttgac gcaaatgggc 530

<210> 17
<211> 26
<212> DNA
<213> Artificial Sequence

<220>
<223> CuO (TGX-02)

<400> 17
caaacagacc agattgtctg tttgtt 26

<210> 18
<211> 75
<212> DNA
<213> Artificial Sequence

<220>
<223> CMV 5\222UTR (TGX-02)

<400> 18
tcatatcgcc tggagacgcc atccacgctg ttttgacctc catagaagac accgggaccg 60

atccagcctc cgcgg 75

<210> 19
<211> 1668
<212> DNA
<213> Artificial Sequence

<220>
<223> PERF1 (TGX-02)

<400> 19
atggcagccc gtctgctcct cctgggcatc cttctcctgc tgctgcccct gcccgtccct 60

gccccgtgcc acacagccgc acgctcagag tgcaagcgca gccacaagtt cgtgcctggt 120

gcatggctgg ccggggaggg tgtggacgtg accagcctcc gccgctcggg ctccttccca 180

gtggacacac aaaggttcct gcggcccgac ggcacctgca ccctctgtga aaatgcccta 240

caggagggca ccctccagcg cctgcctctg gcgctcacca actggcgggc ccagggctct 300

ggctgccagc gccatgtaac cagggccaaa gtcagctcca ctgaagctgt ggcccgggat 360

gcggctcgta gcatccgcaa cgactggaag gtcgggctgg acgtgactcc taagcccacc 420

agcaatgtgc atgtgtctgt ggccggctca cactcacagg cagccaactt tgcagcccag 480

aagacccacc aggaccagta cagcttcagc actgacacgg tggagtgccg cttctacagt 540

ttccatgtgg tacacactcc cccgctgcac cctgacttca agagggccct cggggacctg 600

ccccaccact tcaacgcctc cacccagccc gcctacctca ggcttatctc caactacggc 660

acccacttca tccgggctgt ggagctgggt ggccgcatat cggccctcac tgccctgcgc 720

acctgcgagc tggccctgga agggctcacg gacaacgagg tggaggactg cctgactgtc 780

gaggcccagg tcaacatagg catccacggc agcatctctg ccgaagccaa ggcctgtgag 840

gagaagaaga agaagcacaa gatgacggcc tccttccacc aaacctaccg ggagcgccat 900

tcggaagtgg ttggcggcca tcacacctcc attaacgacc tgctgttcgg gatccaggcc 960

gggcccgagc agtactcagc ctgggtaaac tcgctgcccg gcagccctgg cctggtggac 1020

tacaccctgg aacccctgca cgtgctgctg gacagccagg acccgcggcg ggaggcactg 1080

aggagggccc tgagtcagta cctgacggac agggctcgct ggagggactg cagccggccg 1140

tgcccaccag ggcggcagaa gagcccccga gacccatgcc agtgtgtgtg ccatggctca 1200

gcggtcacca cccaggactg ctgccctcgg cagaggggcc tggcccagct ggaggtgacc 1260

ttcatccaag catggggcct gtggggggac tggttcactg ccacggatgc ctatgtgaag 1320

ctcttctttg gtggccagga gctgaggacg agcaccgtgt gggacaataa caaccccatc 1380

tggtcagtgc ggctggattt tggggatgtg ctcctggcca caggggggcc cctgaggttg 1440

caggtctggg atcaggactc tggcagggac gatgacctcc ttggcacctg tgatcaggct 1500

cccaagtctg gttcccatga ggtgagatgc aacctgaatc atggccacct aaaattccgc 1560

tatcatgcca ggtgcttgcc ccacctggga ggaggcacct gcctggacta tgtcccccaa 1620

atgcttctgg gggagcctcc aggaaaccgg agtggggccg tgtggtga 1668

<210> 20
<211> 122
<212> DNA
<213> Artificial Sequence

<220>
<223> SV40 poly(A) signal (TGX-02)

<400> 20
aacttgttta ttgcagctta taatggttac aaataaagca atagcatcac aaatttcaca 60

aataaagcat ttttttcact gcattctagt tgtggtttgt ccaaactcat caatgtatct 120

ta 122

<210> 21
<211> 45
<212> DNA
<213> Artificial Sequence

<220>
<223> CR2-D24 (TGX-13)

<400> 21
cttgtaccgg aggtgatcga tccacccagt gacgacgagg atgaa                45

<210> 22
<211> 1764
<212> DNA
<213> Artificial Sequence

<220>
<223> Ad5/3 fiber (TGX-13)

<400> 22
atgaagcgcg caagaccgtc tgaagatacc ttcaaccccg tgtatccata tgacacggaa      60
accggtcctc caactgtgcc ttttcttact cctccctttg tatcccccaa tgggtttcaa     120
gagagtcccc ctggggtact ctctttgcgc ctatccgaac ctctagttac ctccaatggc     180
atgcttgcgc tcaaaatggg caacggcctc tctctggacg aggccggcaa ccttacctcc     240
caaaatgtaa ccactgtgag cccacctctc aaaaaaacca agtcaaacat aaacctggaa     300
atatctgcac ccctcacagt tacctcagaa gccctaactg tggctgccgc cgcacctcta     360
atggtcgcgg gcaacacact caccatgcaa tcacaggccc cgctaaccgt gcacgactcc     420
aaacttagca ttgccaccca aggacccctc acagtgtcag aaggaaagct agccctgcaa     480
acatcaggcc ccctcaccac caccgatagc agtaccctta ctatcactgc ctcaccccct     540
ctaactactg ccactggtag cttgggcatt gacttgaaag agcccattta tacacaaaat     600
ggaaaactag gactaaagta cggggctcct ttgcatgtaa cagacgacct aaacactttg     660
accgtagcaa ctggtccagg tgtgactatt aataatactt ccttgcaaac taaagttact     720
ggagccttgg gttttgattc acaaggcaat atgcaactta atgtagcagg aggactaagg     780
attgattctc aaaacagacg ccttatactt gatgttagtt atccgtttga tgctcaaaac     840
caactaaatc taagactagg acagggccct ctttttataa actcagccca caacttggat     900
attaactaca acaaaggcct ttacttgttt acagcttcaa acaattccaa aaagcttgag     960
gttaacctaa gcactgccaa ggggttgatg tttgacgcta cagccatagc cattaatgca    1020
ggagatgggc ttgaatttgg ttcacctaat gcaccaaaca caaatcccct caaaacaaaa    1080
attggccatg gcctagaatt tgattcaaac aaggctatgg ttcctaaact aggaactggc    1140
cttagttttg acagcacagg tgccattaca gtaggaaaca aaaataatga taagctaacc    1200
ctatggacag gtccaaaacc agaagccaac tgcataattg aatacgggaa acaaaaccca    1260
gatagcaaac taactttaat ccttgtaaaa aatggaggaa ttgttaatgg atatgtaacg    1320
ctaatgggag cctcagacta cgttaacacc ttatttaaaa acaaaaatgt ctccattaat    1380
gtagaactat actttgatgc cactggtcat atattaccag actcatcttc tcttaaaaca    1440
gatctagaac taaaatacaa gcaaaccgct gactttagtg caagaggttt tatgccaagt    1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 23
<211> 530
<212> DNA
<213> Artificial Sequence

<220>
<223> CMV promoter/enhancer (TGX-13)

<400> 23
ctagttatta atagtaatca attacggggt cattagttca tagcccatat atggagttcc 60

gcgttacata acttacggta aatggcccgc ctggctgacc gcccaacgac ccccgcccat 120

tgacgtcaat aatgacgtat gttcccatag taacgccaat agggactttc cattgacgtc 180

aatgggtgga gtatttacgg taaactgccc acttggcagt acatcaagtg tatcatatgc 240

caagtccgcc ccctattgac gtcaatgacg gtaaatggcc cgcctggcat tatgcccagt 300

acatgacctt acgggacttt cctacttggc agtacatcta cgtattagtc atcgctatta 360

ccatggtgat gcggttttgg cagtacacca atgggcgtgg atagcggttt gactcacggg 420

gatttccaag tctccacccc attgacgtca atgggagttt gttttggcac caaaatcaac 480

gggactttcc aaaatgtcgt aatacccccg ccccgttgac gcaaatgggc 530

<210> 24
<211> 26
<212> DNA
<213> Artificial Sequence

<220>
<223> CUO (TGX-13)

<400> 24
caaacagacc agattgtctg tttgtt 26

<210> 25
<211> 75
<212> DNA
<213> Artificial Sequence

<220>
<223> CMV 5\222 UTR (TGX-13)

<400> 25
tcatatcgcc tggagacgcc atccacgctg ttttgacctc catagaagac accgggaccg      60

atccagcctc cgcgg                                                      75


<210> 26
<211> 1668
<212> DNA
<213> Artificial Sequence


<220>
<223> PERF1 (TGX-13)

<400> 26
atggcagccc gtctgctcct cctgggcatc cttctcctgc tgctgcccct gcccgtccct      60

gccccgtgcc acacagccgc acgctcagag tgcaagcgca gccacaagtt cgtgcctggt     120

gcatggctgg ccggggaggg tgtggacgtg accagcctcc gccgctcggg ctccttccca     180

gtggacacac aaaggttcct gcggcccgac ggcacctgca ccctctgtga aaatgcccta     240

caggagggca ccctccagcg cctgcctctg gcgctcacca actggcgggc ccagggctct     300

ggctgccagc gccatgtaac cagggccaaa gtcagctcca ctgaagctgt ggcccgggat     360

gcggctcgta gcatccgcaa cgactggaag gtcgggctgg acgtgactcc taagcccacc     420

agcaatgtgc atgtgtctgt ggccggctca cactcacagg cagccaactt tgcagcccag     480

aagacccacc aggaccagta cagcttcagc actgacacgg tggagtgccg cttctacagt     540

ttccatgtgg tacacactcc cccgctgcac cctgacttca agagggccct cggggacctg     600

ccccaccact tcaacgcctc cacccagccc gcctacctca ggcttatctc caactacggc     660

acccacttca tccgggctgt ggagctgggt ggccgcatat cggccctcac tgccctgcgc     720

acctgcgagc tggccctgga agggctcacg gacaacgagg tggaggactg cctgactgtc     780

gaggcccagg tcaacatagg catccacggc agcatctctg ccgaagccaa ggcctgtgag     840

gagaagaaga agaagcacaa gatgacggcc tccttccacc aaacctaccg ggagcgccat     900

tcggaagtgg ttggcggcca tcacacctcc attaacgacc tgctgttcgg gatccaggcc     960

gggcccgagc agtactcagc ctgggtaaac tcgctgcccg gcagccctgg cctggtggac    1020

tacaccctgg aacccctgca cgtgctgctg gacagccagg acccgcggcg ggaggcactg    1080

aggagggccc tgagtcagta cctgacggac agggctcgct ggagggactg cagccggccg    1140

tgcccaccag ggcggcagaa gagcccccga gacccatgcc agtgtgtgtg ccatggctca    1200

gcggtcacca cccaggactg ctgccctcgg cagaggggcc tggcccagct ggaggtgacc    1260

ttcatccaag catggggcct gtggggggac tggttcactg ccacggatgc ctatgtgaag    1320

ctcttctttg gtggccagga gctgaggacg agcaccgtgt gggacaataa caaccccatc    1380

tggtcagtgc ggctggattt tggggatgtg ctcctggcca caggggggcc cctgaggttg    1440

caggtctggg atcaggactc tggcagggac gatgacctcc ttggcacctg tgatcaggct 1500

cccaagtctg gttcccatga ggtgagatgc aacctgaatc atggccacct aaaattccgc 1560

tatcatgcca ggtgcttgcc ccacctggga ggaggcacct gcctggacta tgtcccccaa 1620

atgcttctgg gggagcctcc aggaaaccgg agtggggccg tgtggtga 1668

<210> 27
<211> 572
<212> DNA
<213> Artificial Sequence

<220>
<223> IRES (TGX-13)

<400> 27
cccctctccc tcccccccct aacgttactg gccgaagccg cttggaataa ggccggtgtg 60

cgtttgtcta tatgttattt tccaccatat tgccgtcttt tggcaatgtg agggcccgga 120

aacctggccc tgtcttcttg acgagcattc ctaggggtct ttcccctctc gccaaaggaa 180

tgcaaggtct gttgaatgtc gtgaaggaag cagttcctct ggaagcttct tgaagacaaa 240

caacgtctgt agcgaccctt tgcaggcagc ggaacccccc acctggcgac aggtgcctct 300

gcggccaaaa gccacgtgta taagatacac ctgcaaaggc ggcacaaccc cagtgccacg 360

ttgtgagttg gatagttgtg gaaagagtca aatggctctc ctcaagcgta ttcaacaagg 420

ggctgaagga tgcccagaag gtaccccatt gtatgggatc tgatctgggg cctcggtgca 480

catgctttac atgtgtttag tcgaggttaa aaaaacgtct aggccccccg aaccacgggg 540

acgtggtttt cctttgaaaa acacgatgat aa 572

<210> 28
<211> 744
<212> DNA
<213> Artificial Sequence

<220>
<223> GRB (TGX-13)

<400> 28
atgcaaccaa tcctgcttct gctggccttc ctcctgctgc ccagggcaga tgcaggggag 60

atcatcgggg gacatgaggc caagccccac tcccgcccct acatggctta tcttatgatc 120

tgggatcaga agtctctgaa gaggtgcggt ggcttcctga tacaagacga cttcgtgctg 180

acagctgctc actgttgggg aagctccata aatgtcacct tgggggccca caatatcaaa 240

gaacaggagc cgacccagca gtttatccct gtgaaaagac ccatccccca tccagcctat 300

aatcctaaga acttctccaa cgacatcatg ctactgcagc tggagagaaa ggccaagcgg 360

accagagctg tgcagcccct caggctacct agcaacaagg cccaggtgaa gccagggcag 420

acatgcagtg tggccggctg ggggcagacg gcccccctgg gaaaacactc acacacacta 480

caagaggtga agatgacagt gcaggaagat cgaaagtgcg aatctgactt acgccattat 540

tacgacagta ccattgagtt gtgcgtgggg gacccagaga ttaaaaagac ttcctttaag 600

ggggactctg gaggccctct tgtgtgtaac aaggtggccc agggcattgt ctcctatgga 660

cgaaacaatg gcatgcctcc acgagcctgc accaaagtct caagctttgt acactggata 720

aagaaaacca tgaaacgcca ctaa 744

<210> 29
<211> 122
<212> DNA
<213> Artificial Sequence

<220>
<223> SV40 poly(A) signal (TGX-13)

<400> 29
aacttgttta ttgcagctta taatggttac aaataaagca atagcatcac aaatttcaca 60

aataaagcat ttttttcact gcattctagt tgtggtttgt ccaaactcat caatgtatct 120

ta 122

<210> 30
<211> 45
<212> DNA
<213> Artificial Sequence

<220>
<223> CR2-D24 (TGX-14)

<400> 30
cttgtaccgg aggtgatcga tccacccagt gacgacgagg atgaa 45

<210> 31
<211> 1764
<212> DNA
<213> Artificial Sequence

<220>
<223> Ad5/3 fiber (TGX-14)

<400> 31
atgaagcgcg caagaccgtc tgaagatacc ttcaaccccg tgtatccata tgacacggaa 60

accggtcctc caactgtgcc ttttcttact cctccctttg tatcccccaa tgggtttcaa 120

gagagtcccc ctggggtact ctctttgcgc ctatccgaac ctctagttac ctccaatggc 180

atgcttgcgc tcaaaatggg caacggcctc tctctggacg aggccggcaa ccttacctcc 240

caaaatgtaa ccactgtgag cccacctctc aaaaaaacca agtcaaacat aaacctggaa 300

atatctgcac ccctcacagt tacctcagaa gccctaactg tggctgccgc cgcacctcta 360

atggtcgcgg gcaacacact caccatgcaa tcacaggccc cgctaaccgt gcacgactcc 420

aaacttagca ttgccaccca aggacccctc acagtgtcag aaggaaagct agccctgcaa 480

acatcaggcc ccctcaccac caccgatagc agtaccctta ctatcactgc ctcaccccct 540

ctaactactg ccactggtag cttgggcatt gacttgaaag agcccattta tacacaaaat 600

ggaaaactag gactaaagta cggggctcct ttgcatgtaa cagacgacct aaacactttg 660

accgtagcaa ctggtccagg tgtgactatt aataatactt ccttgcaaac taaagttact 720

ggagccttgg gttttgattc acaaggcaat atgcaactta atgtagcagg aggactaagg 780

attgattctc aaaacagacg ccttatactt gatgttagtt atccgtttga tgctcaaaac 840

caactaaatc taagactagg acagggccct ctttttataa actcagccca caacttggat 900

attaactaca acaaaggcct ttacttgttt acagcttcaa acaattccaa aaagcttgag 960

gttaacctaa gcactgccaa ggggttgatg tttgacgcta cagccatagc cattaatgca 1020

ggagatgggc ttgaatttgg ttcacctaat gcaccaaaca caaatcccct caaaacaaaa 1080

attggccatg gcctagaatt tgattcaaac aaggctatgg ttcctaaact aggaactggc 1140

cttagttttg acagcacagg tgccattaca gtaggaaaca aaaataatga taagctaacc 1200

ctatggacag gtccaaaacc agaagccaac tgcataattg aatacgggaa acaaaaccca 1260

gatagcaaac taactttaat ccttgtaaaa aatggaggaa ttgttaatgg atatgtaacg 1320

ctaatgggag cctcagacta cgttaacacc ttatttaaaa acaaaaatgt ctccattaat 1380

gtagaactat actttgatgc cactggtcat atattaccag actcatcttc tcttaaaaca 1440

gatctagaac taaaatacaa gcaaaccgct gactttagtg caagaggttt tatgccaagt 1500

actacagcgt atccatttgt ccttcctaat gcgggaacac ataatgaaaa ttatattttt 1560

ggtcaatgct actacaaagc aagcgatggt gccctttttc cgttggaagt tactgttatg 1620

cttaataaac gcctgccaga tagtcgcaca tcctatgtta tgactttttt atggtccttg 1680

aatgctggtc tagctccaga aactactcag gcaaccctca taacctcccc atttaccttt 1740

tcctatatta gagaagatga ctaa 1764

<210> 32
<211> 529
<212> DNA
<213> Artificial Sequence

<220>
<223> CMV promoter/enhancer (TGX-14)

<400> 32

ctagttatta atagtaatca attacggggt cattagttca tagcccatat atggagttcc 60

gcgttacata acttacggta aatggcccgc ctggctgacc gcccaacgac ccccgcccat 120

tgacgtcaat aatgacgtat gttcccatag taacgccaat agggactttc cattgacgtc 180

aatgggtgga gtatttacgg taaactgccc acttggcagt acatcaagtg tatcatatgc 240

caagtccgcc ccctattgac gtcaatgacg gtaaatggcc cgcctggcat tatgcccagt 300

acatgacctt acgggacttt cctacttggc agtacatcta cgtattagtc atcgctatta 360

ccatggtgat gcggttttgg cagtacacca atgggcgtgg atagcggttt gactcacggg 420

gatttccaag tctccacccc attgacgtca atgggagttt gttttggcac caaaatcaac 480

gggactttcc aaaatgtcgt aataaccccg ccccgttgac gcaaatggg 529

<210> 33
<211> 26
<212> DNA
<213> Artificial Sequence

<220>
<223> CUO (TGX-14)

<400> 33
caaacagacc agattgtctg tttgtt 26

<210> 34
<211> 75
<212> DNA
<213> Artificial Sequence

<220>
<223> CMC 5\222UTR (TGX-14)

<400> 34
tcatatcgcc tggagacgcc atccacgctg ttttgacctc catagaagac accgggaccg 60

atccagcctc cgcgg 75

<210> 35
<211> 744
<212> DNA
<213> Artificial Sequence

<220>
<223> GRB (TGX-14)

<400> 35
atgcaaccaa tcctgcttct gctggccttc ctcctgctgc ccagggcaga tgcaggggag 60

atcatcgggg gacatgaggc caagccccac tcccgcccct acatggctta tcttatgatc 120

tgggatcaga agtctctgaa gaggtgcggt ggcttcctga tacaagacga cttcgtgctg 180

acagctgctc actgttgggg aagctccata aatgtcacct tgggggccca caatatcaaa 240

gaacaggagc cgacccagca gtttatccct gtgaaaagac ccatccccca tccagcctat 300

aatcctaaga acttctccaa cgacatcatg ctactgcagc tggagagaaa ggccaagcgg 360

accagagctg tgcagcccct caggctacct agcaacaagg cccaggtgaa gccagggcag 420

acatgcagtg tggccggctg ggggcagacg gcccccctgg gaaaacactc acacacacta 480

caagaggtga agatgacagt gcaggaagat cgaaagtgcg aatctgactt acgccattat 540

tacgacagta ccattgagtt gtgcgtgggg gacccagaga ttaaaaagac ttcctttaag 600

ggggactctg gaggccctct tgtgtgtaac aaggtggccc agggcattgt ctcctatgga 660

cgaaacaatg gcatgcctcc acgagcctgc accaaagtct caagctttgt acactggata 720

aagaaaacca tgaaacgcca ctaa 744

<210> 36
<211> 122
<212> DNA
<213> Artificial Sequence

<220>
<223> SV40 poly(A) signal (TGX-14)

<400> 36
aacttgttta ttgcagctta taatggttac aaataaagca atagcatcac aaatttcaca 60

aataaagcat ttttttcact gcattctagt tgtggtttgt ccaaactcat caatgtatct 120

ta 122

<210> 37
<211> 258
<212> DNA
<213> Artificial Sequence

<220>
<223> RSV promoter (TGX-14)

<400> 37
atgtagtctt atgcaatact cttgtagtct tgcaacatgg taacgatgag ttagcaacat 60

gccttacaag gagagaaaaa gcaccgtgca tgccgattgg tggaagtaag gtggtacgat 120

cgtgccttat taggaaggca acagacgggt ctgacatgga ttggacgaac cactgaattc 180

cgcattgcag agatattgta tttaagtgcc tagctcgata caataaacgc catttgacca 240

ttcaccacat tggtgtgc 258

<210> 38
<211> 1668
<212> DNA
<213> Artificial Sequence

<220>
<223> PERF1 (TGX-14)

<400> 38
atggcagccc gtctgctcct cctgggcatc cttctcctgc tgctgcccct gcccgtccct 60

gccccgtgcc acacagccgc acgctcagag tgcaagcgca gccacaagtt cgtgcctggt 120

gcatggctgg ccggggaggg tgtggacgtg accagcctcc gccgctcggg ctccttccca 180

gtggacacac aaaggttcct gcggcccgac ggcacctgca ccctctgtga aaatgcccta 240

caggagggca ccctccagcg cctgcctctg gcgctcacca actggcgggc ccagggctct 300

ggctgccagc gccatgtaac cagggccaaa gtcagctcca ctgaagctgt ggcccgggat 360

gcggctcgta gcatccgcaa cgactggaag gtcgggctgg acgtgactcc taagcccacc 420

agcaatgtgc atgtgtctgt ggccggctca cactcacagg cagccaactt tgcagcccag 480

aagacccacc aggaccagta cagcttcagc actgacacgg tggagtgccg cttctacagt 540

ttccatgtgg tacacactcc cccgctgcac cctgacttca agagggccct cggggacctg 600

ccccaccact tcaacgcctc cacccagccc gcctacctca ggcttatctc caactacggc 660

acccacttca tccgggctgt ggagctgggt ggccgcatat cggccctcac tgccctgcgc 720

acctgcgagc tggccctgga agggctcacg gacaacgagg tggaggactg cctgactgtc 780

gaggcccagg tcaacatagg catccacggc agcatctctg ccgaagccaa ggcctgtgag 840

gagaagaaga agaagcacaa gatgacggcc tccttccacc aaacctaccg ggagcgccat 900

tcggaagtgg ttggcggcca tcacacctcc attaacgacc tgctgttcgg gatccaggcc 960

gggcccgagc agtactcagc ctgggtaaac tcgctgcccg gcagccctgg cctggtggac 1020

tacaccctgg aacccctgca cgtgctgctg gacagccagg acccgcggcg ggaggcactg 1080

aggagggccc tgagtcagta cctgacggac agggctcgct ggagggactg cagccggccg 1140

tgcccaccag ggcggcagaa gagcccccga gacccatgcc agtgtgtgtg ccatggctca 1200

gcggtcacca cccaggactg ctgccctcgg cagaggggcc tggcccagct ggaggtgacc 1260

ttcatccaag catggggcct gtggggggac tggttcactg ccacggatgc ctatgtgaag 1320

ctcttctttg gtggccagga gctgaggacg agcaccgtgt gggacaataa caaccccatc 1380

tggtcagtgc ggctggattt tggggatgtg ctcctggcca caggggggcc cctgaggttg 1440

caggtctggg atcaggactc tggcagggac gatgacctcc ttggcacctg tgatcaggct 1500

cccaagtctg gttcccatga ggtgagatgc aacctgaatc atggccacct aaaattccgc 1560

tatcatgcca ggtgcttgcc ccacctggga ggaggcacct gcctggacta tgtcccccaa 1620

atgcttctgg gggagcctcc aggaaaccgg agtggggccg tgtggtga 1668

<210> 39
<211> 225
<212> DNA
<213> Artificial Sequence

<220>
<223> bGH poly(A) signal (TGX-14)

<400> 39
ctgtgccttc tagttgccag ccatctgttg tttgcccctc ccccgtgcct tccttgaccc 60

tggaaggtgc cactcccact gtcctttcct aataaaatga ggaaattgca tcgcattgtc 120

tgagtaggtg tcattctatt ctggggggtg gggtggggca ggacagcaag ggggaggatt 180

gggaagacaa tagcaggcat gctggggatg cggtgggctc tatgg 225

<210> 40
<211> 23
<212> DNA
<213> Artificial Sequence

<220>
<223> Primer 282-1

<400> 40
ttgacattga ttattgacta gtt 23

<210> 41
<211> 38
<212> DNA
<213> Artificial Sequence

<220>
<223> Primer 282-2

<400> 41
gctcatggtc tctcggcccg cggaggctgg atcggtcc 38

<210> 42
<211> 35
<212> DNA
<213> Artificial Sequence

<220>
<223> Primer 282-3

<400> 42
atgatcggtc tcagccgcca ccatgcaacc aatcc 35

<210> 43
<211> 39
<212> DNA
<213> Artificial Sequence

<220>
<223> Primer 282-4

<400> 43
cgtgacggtc tctagtttta gtggcgtttc atggttttc                              39


<210> 44
<211> 39
<212> DNA
<213> Artificial Sequence


<220>
<223> Primer 282-5

<400> 44
agtgacggtc tcaaacttgt ttattgcagc ttataatgg                              39


<210> 45
<211> 40
<212> DNA
<213> Artificial Sequence


<220>
<223> Primer 282-6

<400> 45
gctcatacta gttaagatac attgatgagt ttggacaaac                             40


<210> 46
<211> 40
<212> DNA
<213> Artificial Sequence


<220>
<223> Primer 282-7

<400> 46
atgatccgtc tcagccgcca ccatggcagc ccgtctgctc                             40


<210> 47
<211> 39
<212> DNA
<213> Artificial Sequence


<220>
<223> Primer 282-8

<400> 47
cgtgaccgtc tctagtttca ccacacggcc ccactccgg                              39

## Description

### FIELD OF THE DISCLOSURE

The present invention relates to cancer therapies. More specifically, the present invention relates to oncolytic adenoviral vectors and cells and pharmaceutical compositions comprising said vectors. The present invention also relates to a use of said vectors in the manufacture of a medicament for treating cancer in a subject and a method of treating cancer in a subject. Furthermore, the present invention relates to methods of producing granzyme and perforin in a cell and increasing anti-tumor effect and induction of specific immune response in a subject, as well as uses of the oncolytic adenoviral vector of the invention for producing transgenes in a cell and increasing anti-tumor effect and generation of specific immune response in a subject.

### BACKGROUND OF THE DISCLOSURE

During the last twenty years gene transfer technology has been under intensive examination. The aim of cancer gene therapies is to introduce a therapeutic gene into a tumor cell. These therapeutic genes introduced to a target cell may for example correct mutated genes, suppress active oncogenes or generate additional properties to the cell. Suitable exogenous therapeutic genes include but are not limited to immunotherapeutic, anti-angiogenic, chemoprotective and "suicide" genes, and they can be introduced to a cell by utilizing modified virus vectors or non-viral methods including electroporation, gene gun and lipid or polymer coatings.

Requirements of optimal viral vectors include an efficient capability to find specific target cells and express the viral genome in the target cells. Furthermore, optimal vectors have to stay active in the target tissues or cells. All these properties of viral vectors have been developed during the last decades and for example retroviral, adenoviral and adeno-associated viral vectors have been widely studied in biomedicine.

To further improve tumor penetration and local amplification of the anti-tumor effect, selectively oncolytic agents, e.g. conditionally replicating adenoviruses, have been constructed. Oncolytic adenoviruses are a promising tool for treatment of cancers. Tumor cells are killed by oncolytic adenoviruses due to a replication of the virus in a tumor cell, the last phase of the replication resulting in a release of thousands of virions into the surrounding tumor tissues for effective tumor penetration and vascular re-infection. Tumor cells allow replication of the virus while normal cells are spared due to engineered changes in the virus genome, which prevent replication in non-tumor cells. In addition to replication mediated cell killing, oncolytic adenoviruses can also be armed with different therapeutic transgenes. This approach combines the advantages of conventional gene delivery with the potency of replication competent agents. One goal of arming viruses is induction of an immune reaction towards the cells that allow virus replication. Virus replication alone, although immunogenic, is normally not enough to induce effective anti-tumor immunity. To strengthen induction of therapeutic immunity, viruses can be armed with stimulatory proteins such as cytokines for facilitation of the introduction of tumor antigens to antigen presenting cells such as dendritic cells, and their stimulation and/or maturation. Introduction of immunotherapeutic genes into tumor cells and furthermore, translation of the proteins, leads to activation of the immune response and efficient destruction of tumor cells. The most relevant immune cells in this regard are natural killer cells (NK) and cytotoxic CD8+ T-cells.

More than 50 different serotypes of adenoviruses have been found in humans. Adenovirus serotype 5 (Ad5) is known to cause respiratory diseases and it is the most common serotype studied in the field of gene therapy. In the first Ad5 vectors E1 and/or E3 regions were deleted enabling insertion of foreign DNA to the vectors. Furthermore, deletions of other regions as well as further mutations have provided extra properties to viral vectors, indeed, various modifications of adenoviruses have been suggested for achieving efficient antitumor effects.

Granzymes are serine proteases released by cytoplasmic granules within cytotoxic T cells and natural killer (NK) cells. They induce programmed cell death (apoptosis) in the target cell, thus eliminating cells that have become cancerous or are infected with viruses or bacteria. The family of granzymes (a family of lymphocyte granule serine proteases) comprises five known human protein members: A, B, H, M and tryptase-2, which is also known as granzyme 3. Granzyme B (GrB) is a serine protease most commonly found in the granules of cytotoxic lymphocytes (CTLs), natural killer cells (NK cells) and cytotoxic T cells (Rousalova and Krepela, 2010). It is secreted by these cells along with the pore forming protein perforin to mediate apoptosis in target cells.

Perforin (PERF, PERF1) is a pore forming cytolytic protein found in the granules of cytotoxic T lymphocytes (CTLs) and NK cells (Osinska et al., 2014). Upon degranulation, perforin binds to the target cell's plasma membrane, and oligomerises in a Ca2+ dependent manner to form pores on the target cell (Krzewski and Coligan, 2012). The pore formed allows for the passive diffusion of a family of pro-apoptotic proteases, known as the granzymes, into the target cell.

Interactions between granzyme and perforin is crucial to initiate apoptosis of target cell. The cytotoxic lymphocytes (CTLs) are able to recognize their target cells and form an immunological synapse. The microtubule-organizing centre (MTOC) of the cytotoxic lymphocyte polarizes and secretory granule traffic towards the presynaptic membrane. The granules then fuse with the presynaptic membrane and release perforin and granzymes. Subsequently, perforin forms large transmembrane pores that enable transfer of granzymes into the target cell cytosol. Granzymes then initiate apoptosis of the target cell, and the CTLs move from the dying cell and can interact with another target cell to carry out killing effect (Voskoboinik et al., 2015).

GrB is reported to be the most potent of all the human granzymes produced by CTLs. Its biological activity during a CTL or NK cell-mediated immune response is dependent upon: (i) co-release with perforin towards target cells at intercellular spaces called immunological synapses (ii) successful entry into the cytosol of the cell (iii) activation of several pro-apoptotic pathways by proteolytically attacking several intracellular protein substrates (Hlongwane et al., 2018).

EP2379586B1 discloses an oncolytic adenoviral vector comprising an adenovirus serotype 5 (Ad5) nucleic acid backbone, a 24 bp deletion (D24) in the Rb binding constant region 2 of adenoviral E1 and a nucleic acid sequence encoding a granulocyte-macrophage colony-stimulating factor (GM-CSF) in the place of the deleted gp19k/6.7K in the adenoviral E3 region. EP2415783B1 discloses a recombinant vaccinia virus that can express one or more therapeutic genes, whose products cause cell death or whose products cause an anti-tumor immune response. US20150037297A1 discloses an insertion of a potent tumoricidal transgene into a lenti-viral vector. Document further describes recombinant nucleic acid vector comprising a gene(s) encoding one or a plurality of tumoricidal transgenes encoding tumoricidal proteins or nucleotides.

Still, more efficient and accurate gene transfer as well as increased specificity and sufficient tumor killing ability of gene therapies are warranted. The present invention provides a cancer therapeutic tool with these aforementioned properties by utilizing both oncolytic and immunotherapeutic properties of adenoviruses in a novel and inventive way.

### BRIEF DESCRIPTION OF THE DISCLOSURE

The object of the invention is to provide novel methods and means for achieving the above-mentioned properties of adenoviruses and thus, solving the problems of conventional cancer therapies. More specifically, the invention provides novel methods and means for gene therapy.

The present inventors hypothesized that the proteins of granzyme and perforin families could contribute in cancer treatment and enhance properties of an oncolytic virus. The double transgene construct (TGX-14) coding for Granzyme B and Perforin utilize the properties of oncolytic viruses. The virus is capable of selectively lysing tumors due to the fact that it incorporates a 24bp deletion in E1A region for cancer cell restricted replication (Kuryk et al., 2016) and enhanced tropism due to fiber knob modifications (chimeric virus5/3 knob) (Kuryk et al., 2018). In addition to that, due to the presence of transgenes, the construct was surprisingly found to exhibit enhanced anti-tumor effects by increasing the oncolytic potency through apoptosis of both infected and non-infected tumour cells leading to an increase in the release of tumour antigens and thereby enhancing cross presentation of tumour antigens and priming of cancer specific T cells. The incorporated transgenes enhance along with the oncolytic properties of the construct and can be deduced expected to result in synergistic anti-cancer effects and improved clinical outcomes.

The double transgene viruses were found to be more efficient compared to the case where both transgenes were encoded by separate viruses but used together. The double transgene simplifies the production process especially in clinical studies over combined viruses expressing transgenes separately.

The present application describes construction of recombinant viral vectors, methods related to the vectors, and their use in tumor cells lines and animal models.

The present invention relates to an oncolytic adenoviral vector comprising an adenovirus serotype 5 (Ad5) nucleic acid backbone, a 24 bp deletion (D24) in the Rb binding constant region 2 of adenoviral E1 and a nucleic acid sequence encoding granzyme and Perforin transgenes.

In a preferred embodiment, the oncolytic adenoviral vector comprises an adenovirus serotype 5 (Ad5) nucleic acid backbone, a 24 bp deletion (D24) in the Rb binding constant region 2 of adenoviral E1 and a nucleic acid sequence encoding Granzyme B (GrB) and Perforin transgenes (PERF1, PERF). The transgenes can be inserted with or without IRES in the place of the in E3 with 2.7 kb deletion in E3 and/or between the L5 and E4 polyA signals in the place of deleted gp19k/6.7K in the adenoviral E3 region. The vector can contain an adenovirus 3 serotype knob replacing the wild type serotype 5 knob (5/3 chimeric).

The present invention further relates to a cell comprising the adenoviral vector of the invention.

The present invention also relates to a pharmaceutical composition comprising the adenoviral vector of the invention.

The present invention also relates to a use of the adenoviral vector of the invention in the manufacture of a medicament for treating cancer in a subject.

The present invention also relates to a method of treating cancer in a subject, wherein the method comprises administration of the vector or pharmaceutical composition of the invention to a subject.

Furthermore, the present invention also relates to a method of producing granzyme and perforin in a cell, wherein the method comprises: a) infecting a cell with an oncolytic virus of the invention, and b) expressing granzyme and perforin of said vector in the cell.

Furthermore, the present invention also relates to a method of increasing tumor specific immune response in a subject, wherein the method comprises: a) carrying a vehicle comprising an oncolytic adenoviral vector of the invention to a target cell or tissue, b) expressing granzyme and perforin of said vector in the cell, and c) increasing amount of cytotoxic T cells and/or natural killer cells in said target cell or tissue.

Still, the present invention also relates to a use of the oncolytic adenoviral vector of the invention for producing granzyme and perforin in a cell.

Still, the present invention also relates to a use of the oncolytic adenoviral vector of the invention for increasing tumor specific immune response in a subject.

The present invention provides a tool for treatment of cancers, some of which are refractory to current approaches. Also, restrictions regarding tumor types suitable for treatment remain few compared to many other treatments. In fact, all solid tumors may be treated with the proposed invention. Larger tumors by mass and more complex tumors can be treated by using the present invention. The treatment can be given intratumorally, intracavitary, intravenously and in a combination of these. The approach can give systemic efficacy despite local injection. The approach can also eradicate cells proposed as tumor initiating ("cancer stem cells").

### BRIEF DESCRIPTION OF THE DRAWINGS

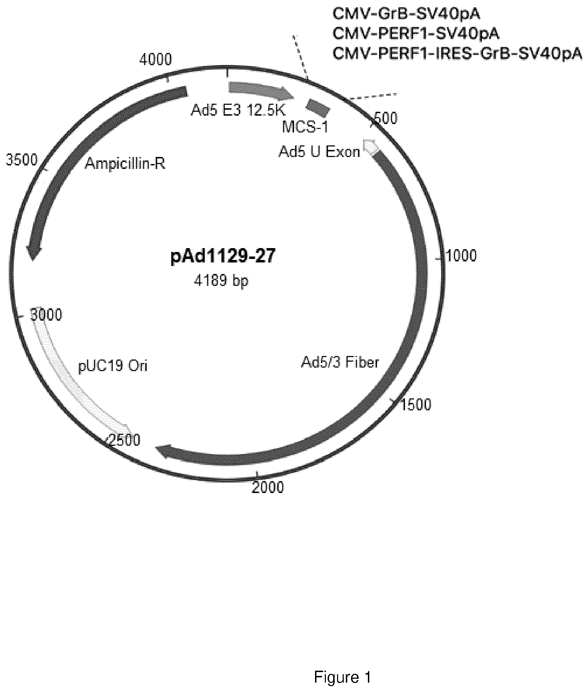

Figure 1 illustrates the shuttle plasmid constructions for the shuttle plasmids for pTGX-01 (Grb), TGX-02 (PERF) and TGX-13 (CMV-PERF-IRES-GrB).

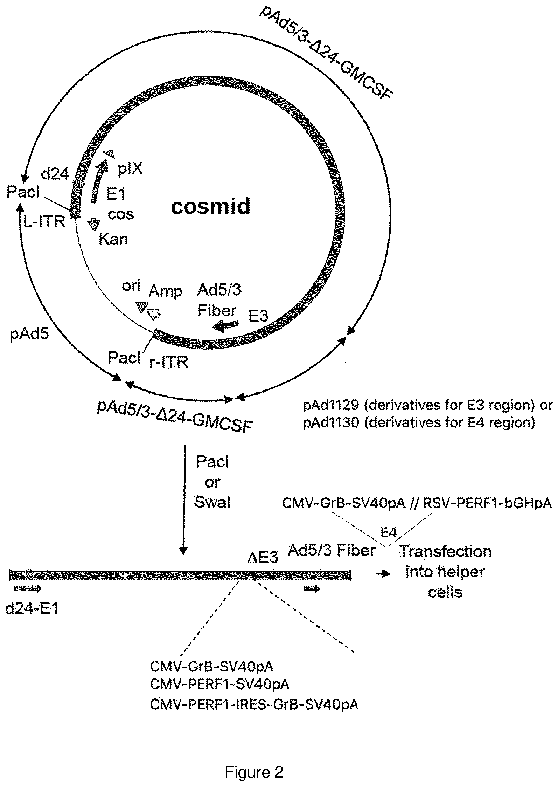

Figure 2 illustrates the cosmid constructions for TGX-01, TGX-02, TGX-13 and TGX-14 (CMV-GrB and RSV-PERF).

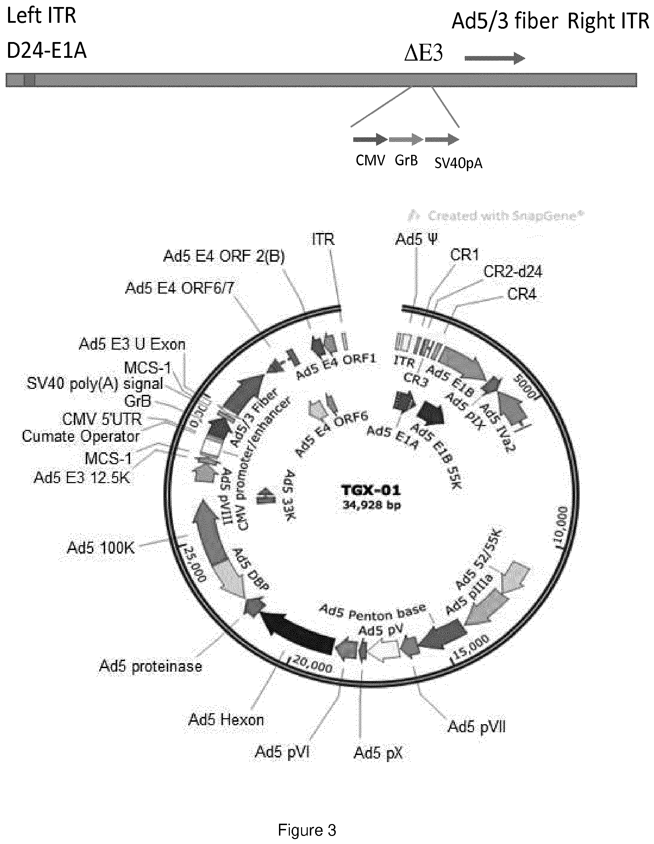

Figure 3 shows TGX-01 virus structure. GrB (functional expression cassette, location: 28,898 - 29,641bp) and SV40pA expression under the control of CMV promoter (28,170 - 28,699) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 905-949bp), expresses the Ad5/3 hybrid fiber (location: 29,999 - 31,762bp).

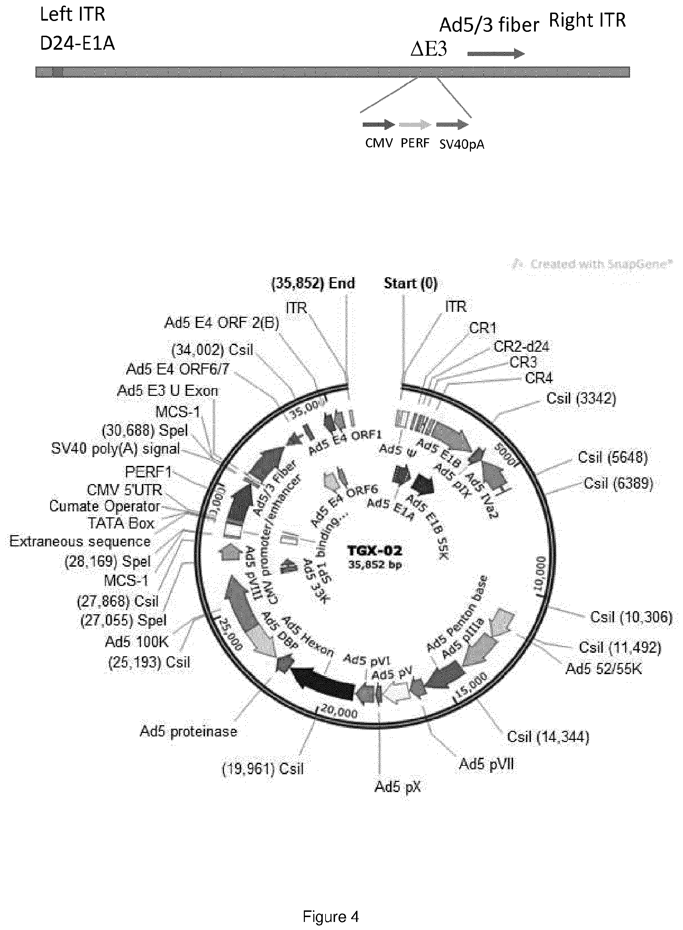

Figure 4 shows TGX-02 virus structure. PERF1 (functional expression cassette, location: 28,898 - 30,565bp) and SV40pA expression under the control of CMV promoter (28,170 - 28,699bp) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 905-949bp), expresses the Ad5/3 hybrid fiber (location: 30,923 - 32,686bp).

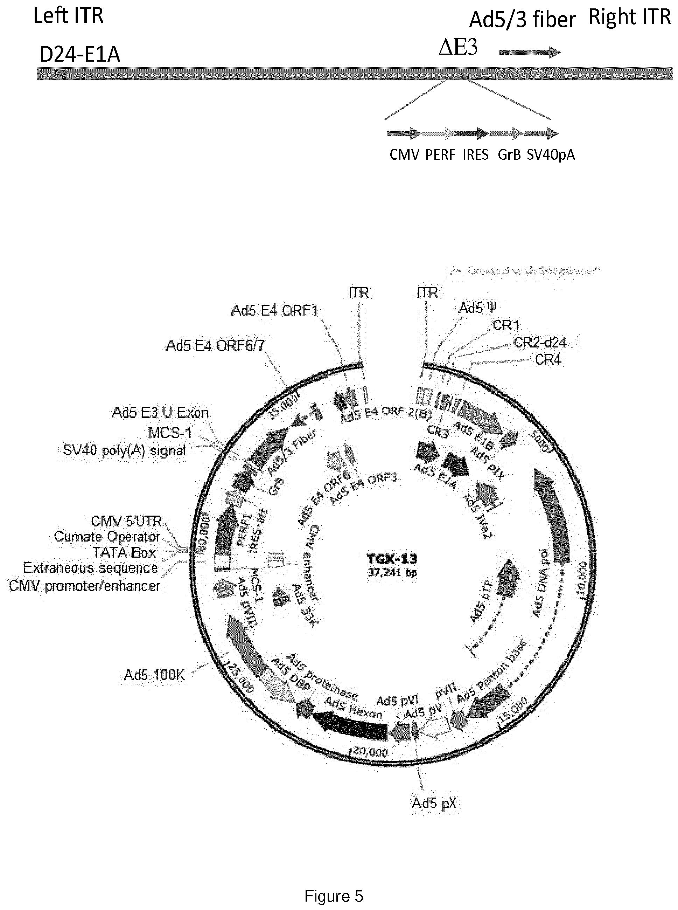

Figure 5 shows TGX-13 virus structure. PERF1 (functional expression cassette, location: 28,934 - 30,601bp) -IRES (30,602 - 31,173bp) -GrB (functional expression cassette, location: 31,207 - 31,950) and SV40pA expression under the control of CMV promoter (28,206 - 28,735bp) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,308 - 34,071 bp).

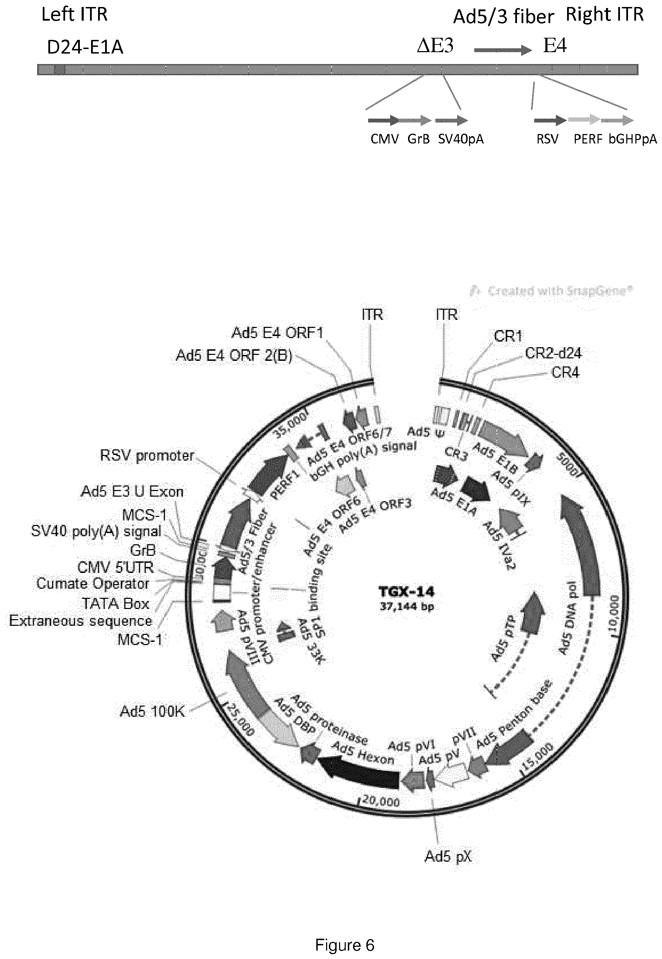

Figure 6 shows TGX-14 virus structure. GrB (functional expression cassette, location: 28,934 - 29,677bp) and SV40pA expression under the control of CMV promoter (28,206 - 28,735bp) inserted into E3 region. PERF1 (functional expression cassette, location: 32,092 33,759bp)- bGH pA expression under the control of RSV promoter (31,821 - 32,081bp) inserted between the L5 and E4 polyA signals. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 30,035 - 31,798bp).

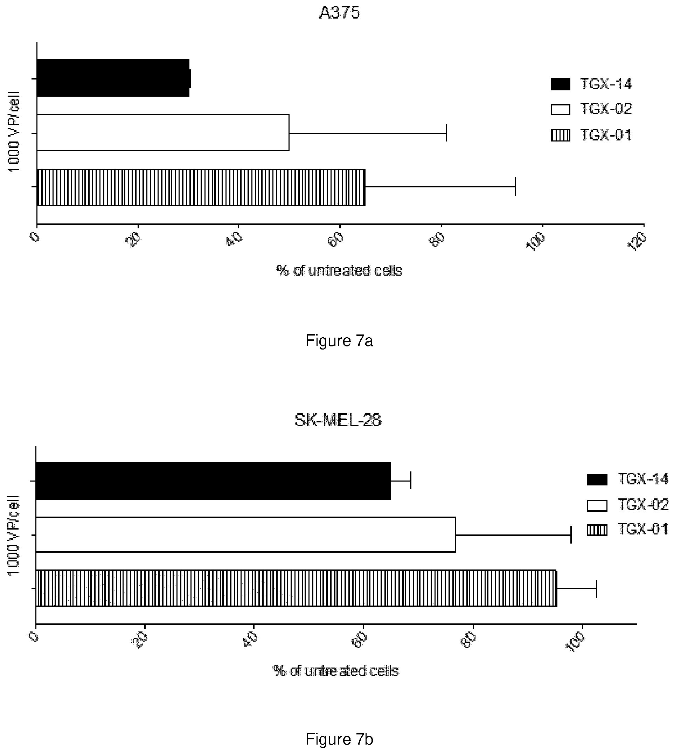

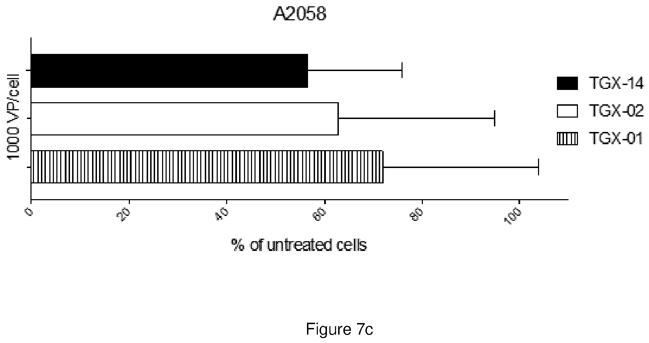

Figure 7. Figure 7 a) shows the effects of tested oncolytic adenoviruses on viability of human melanoma cell line A375 at the concentration of 1000 VP/cell after 72 hours post infection. Absorbance of untreated cells is expressed as 100% and absorbance of treated cells is expressed as a percentage of absorbance of untreated cells. Results are expressed as mean +/- SEM. Figure 7 b) illustrates the effects of tested oncolytic adenoviruses on viability of human melanoma cell line SK-MEL-28 at the concentration of 1000 VP/cell after 72 hours post infection. Absorbance of untreated cells is expressed as 100% and absorbance of treated cells is expressed as a percentage of absorbance of untreated cells. Results are expressed as mean +/- SEM. Figure 7 c) shows the effects of tested oncolytic adenoviruses on viability of human melanoma cell line A2058 at the concentration of 1000 VP/cell after 72 hours post infection. Absorbance of untreated cells is expressed as 100% and absorbance of treated cells is expressed as a percentage of absorbance of untreated cells. Results are expressed as mean +/- SEM.

### SEQUENCE LISTING

Sequence ID NO: 1. A nucleic acid sequence encoding an oncolytic adenoviral vector Ad5/3-D24-GrB-PERF (TGX-14). GrB (functional expression cassette, location: 28,934 - 29,677bp) and SV40pA expression under the control of CMV promoter (28,206 - 28,735bp) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 30,035 - 31,798bp). PERF1 (functional expression cassette, location: 32,092 33,759bp)- bGH pA expression under the control of RSV promoter (31,821 - 32,081bp) inserted into E3 region. Nucleotide sequence enclosed in attachment (TGX-14).
Sequence ID NO: 2. A nucleic acid sequence encoding an oncolytic adenoviral vector Ad5/3-D24-PERF-GrB (TGX-13). PERF1 (functional expression cassette, location: 28,934 - 30,601bp) -IRES (30,602 - 31,173bp) -GrB (functional expression cassette, location: 31,207 - 31,950) -SV40pA expression under the control of CMV promoter (28,206 - 28,735bp) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 907-951bp), expresses the Ad5/3 hybrid fiber (location: 32,308 - 34,071bp).
Sequence ID NO: 3. A nucleic acid sequence encoding an oncolytic adenoviral vector Ad5/3-D24-Grb (TGX-01) virus structure. GrB (functional expression cassette, location: 28,898 - 29,641bp) and SV40pA expression under the control of CMV promoter (28,170 - 28,699) inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 905-949bp), expresses the Ad5/3 hybrid fiber (location: 29,999 - 31,762bp).
Sequence ID NO: 4. A nucleic acid sequence encoding an oncolytic adenoviral vector Ad5/3-D24-PERF (TGX-02). PERF1 (functional expression cassette, location: 28,898 - 30,565bp) and SV40pA expression under the control of CMV promoter (28,170 - 28,699bp), inserted into E3 region. The virus contains the 24-bp deletion in E1A CR2 (E1A Conserved Region 2 (CR2) with a 24-bp deletion that removed the strongest of two binding sites in E1A for Rb protein (LTCHEAGF), location: 905-949bp), expresses the Ad5/3 hybrid fiber (location: 30,923 - 32,686bp).
Sequence ID NO: 5. A binding site for Rb protein in E1A.
Sequence ID NO: 6. Deleted sequence of CR2.
Sequence ID NO: 7. CR2-D24 (TGX-01).
Sequence ID NO: 8. Ad5/3 fiber (TGX-01).
Sequence ID NO: 9. CMV promoter/enhancer (TGX-01).
Sequence ID NO: 10. CUO (TGX-01).
Sequence ID NO: 11. CMV 5'UTR (TGX-01).
Sequence ID NO: 12. GRB (TGX-01).
Sequence ID NO: 13. SV40 poly(A) signal (TGX-01).
Sequence ID NO: 14. CR2-D24 (TGX-02).
Sequence ID NO: 15. Ad5/3 fiber (TGX-02).
Sequence ID NO: 16. CMV promoter/enhancer (TGX-02).
Sequence ID NO: 17. CUO (TGX-02).
Sequence ID NO: 18. CMV 5'UTR (TGX-02).
Sequence ID NO: 19. PERF1 (TGX-02).
Sequence ID NO: 20. SV40 poly(A) signal (TGX-02).
Sequence ID NO: 21. CR2-D24 (907-951bp) (TGX-13).
Sequence ID NO: 22. Ad5/3 fiber (TGX-13).
Sequence ID NO: 23. CMV promoter/enhancer (TGX-13).
Sequence ID NO: 24. CUO (TGX-13).
Sequence ID NO: 25. CMV 5' UTR (TGX-13).
Sequence ID NO: 26. PERF1 (TGX-13).
Sequence ID NO: 27. IRES (TGX-13).
Sequence ID NO: 28. GRB (TGX-13).
Sequence ID NO: 29. SV40 poly(A) signal (TGX-13).
Sequence ID NO: 30. CR2-D24 (TGX-14).
Sequence ID NO: 31. Ad5/3 fiber (TGX-14).
Sequence ID NO: 32. CMV promoter/enhancer (TGX-14).
Sequence ID NO: 33. CUO (TGX-14).
Sequence ID NO: 34. CMC 5'UTR (TGX-14).
Sequence ID NO: 35. GRB (TGX-14).
Sequence ID NO: 36. SV40 poly(A) signal (TGX-14).
Sequence ID NO: 37. RSV promoter (TGX-14).
Sequence ID NO: 38. PERF1 (TGX-14).
Sequence ID NO: 39. bGH poly(A) signal (TGX-14).
Sequence ID NO: 40. Primer sequence 282-1.
Sequence ID NO: 41. Primer sequence 282-2.
Sequence ID NO: 42. Primer sequence 282-3.
Sequence ID NO: 43. Primer sequence 282-4.
Sequence ID NO: 44. Primer sequence 282-5.
Sequence ID NO: 45. Primer sequence 282-6.
Sequence ID NO: 46. Primer sequence 282-7.
Sequence ID NO: 47. Primer sequence 282-8.

### DETAILED DESCRIPTION OF THE DISCLOSURE

The double transgene oncolytic adenoviral vector of the present invention is based on an adenovirus serotype 5 (Ad5) nucleic acid backbone with a 24 bp deletion (D24) in the Rb binding constant region 2 (CR2) of adenoviral E1 and a nucleic acid sequence encoding a granzyme and perforin transgenes. In a preferred embodiment of the invention, the adenoviral vector backbone is ONCOS-102 as disclosed in EP2379586B1, which comprises of granzyme and perforin transgenes that can be connected with IRES sequence in the place of GM-CSF/E3/between the L5 and E4 polyA signals.

The double transgene viruses according to the invention showed surprising effects. The double transgene viruse coding for GrB and PERF1 (TGX-14) showed anti-cancer superiority over singe transgene viruses TGX-01 (coding for GrB) and TGX-02 (coding for PERF1) *in vitro* against A375 cell line (Figure 7a). Double transgene vector (TGX-14) showed also anti-cancer superiority over single transgene vectors (TGX-01 and TGX-02) in SK-MEL-28 cell lines (Figure 7b). Improved oncolytic properties of TGX-14 were also observed in A2058 cell line (Figure 7c).

Compared to a wild type adenovirus genome, the adenoviral vector of the invention lacks 24 base pairs from CR2 in E1 region, specifically in E1A region, and has a 2,7 kbp deletion in E3 region and/or modification between the L5 and E4 polyA signals. 24 base pair deletion (D24) of E1 affects CR2 domain, which is responsible for binding the Rb tumor suppressor/cell cycle regulator protein and thus, allows the induction of the synthesis (S) phase i.e. DNA synthesis or replication phase. pRb and E1A interaction requires eight amino acids 121 to 127 of the E1A protein conserved region, which are deleted in the present invention. Viruses with the D24 are known to have a reduced ability to overcome the G1-S checkpoint and replicate efficiently in cells where this interaction is not necessary, e.g. in tumor cells defective in the Rb-p16 pathway.

The E3 region is nonessential for viral replication *in vitro,* but the E3 proteins have an important role in the regulation of host immune response i.e. in the inhibition of both innate and specific immune responses. The deletion in E3 refers to a deletion of 2.7kbp base pairs from the adenoviral E3A region. In a resulting adenoviral construct, both gp19k and 6.7K genes are deleted. The gp19k gene product is known to bind and sequester major histocompatibility complex I (MHC1) molecules in the endoplasmic reticulum, and to prevent the recognition of infected cells by cytotoxic T-lymphocytes. Since many tumors are deficient in MHC1, deletion of gp19k increases tumor selectivity of viruses (virus is cleared faster than wild type virus from normal cells but there is no difference in tumor cells). 6.7K proteins are expressed on cellular surfaces and they take part in downregulating TNF-related apoptosis inducing ligand (TRAIL) receptor 2.

Proteins from the E4 genes can modulate transcription, the cell-cycle, cell signalling and DNA repair. the early region E4 has been shown to affect transgene persistence, vector toxicity and immunogenicity. In addition, some of these proteins also cause oncogenic transformation. E4 region

The fiber polypeptide is a late product and is encoded in transcription unit L5 of the adenovirus genome. L5 is the last of five cassettes of transcripts (L1-L5) in a large mRNA transcript with a tripartite leader sequence, processed into five individual mRNA families via alternative splicing and usage of different poly(A) signals.

An internal ribosome entry site, abbreviated IRES, is an RNA element that allows for translation initiation in a cap-independent manner, as part of the greater process of protein synthesis. In eukaryotic translation, initiation typically occurs at the 5' end of mRNA molecules, since 5' cap recognition is required for the assembly of the initiation complex.

Mammalian expression plasmids are primarily used to create mRNA and the commonly used mammalian terminators (like SV40, BGH/BGHP,) include the sequence motif which promotes both polyadenylation and termination.

Preferably, Perforin is Perforin1. More preferably, Perforin 1 is human Perforin1. Granzyme can be Granzyme A, B, H or M or tryptase-2. Preferably, granzyme is Granzyme B. More preferably, Granzyme B is human Granzyme B.

In the present invention, the granzyme, perforin and IRES can be placed into 2.7kbp deletion in E3 or between the L5 and E4 polyA signals be under the E3 promoter (for E3 region). Granzyme, can be for example under CMV(Cuo) promoter and perforin for example under RSV promoter. This restricts transgene expression to tumor cells that allow replication of the virus and subsequent activation of the E3 promoter. E3 promoter may be any exogenous or endogenous promoter known in the art, preferably endogenous promoter. In a preferred embodiment of the invention, a nucleic acid sequence encoding granzyme and perforin are under the control of the viral E3 promoter, CMV or RSV promoter. CMV(CuO) promoter contains a copy of the cumate operator (CuO). In "regular" 293 cells and other cell lines, the CuO operator is inactive and transcription takes place from the CMV promoter. In 293-CymR cells, which express the CymR repressor, the CymR repressor binds onto the CuO operator, thereby preventing the transcription of the transgene.

The vectors of the invention may also comprise other modifications than partial deletions of CR2 and E3 and insertion of granzyme and perforin sequences as mentioned above. In a preferred embodiment of the invention, all the other regions of the Ad5 vector are of a wild type. In another preferred embodiment of the invention, the E4 region is of a wild type. In still another embodiment a transgene is inserted into E4 region. In a preferred embodiment of the invention, a wild type region is located upstream of the E1 region. "Upstream" refers to immediately before the E1 region in the direction of expression. E1 B region may also be modified in the vector of the invention.

As used herein, "Ad5/3 chimerism" of the capsid refers to a chimerism, wherein the knob part of the fiber is from Ad serotype 3, and the rest of the fiber is from Ad serotype 5.

Expression cassettes are used for expressing transgenes in a target, such as a cell, by utilizing vectors. As used herein, the expression "expression cassette" refers to a DNA vector or a part thereof comprising nucleotide sequences, which encode cDNAs or genes, and nucleotide sequences, which control and/or regulate the expression of said cDNAs or genes. Similar or different expression cassettes may be inserted to one vector or to several different vectors. Ad5 vectors of the present invention may comprise either several or one expression cassettes. However, only one expression cassette is adequate. In a preferred embodiment of the invention, the oncolytic adenoviral vector comprises at least one expression cassette. In a preferred embodiment of the invention, the oncolytic adenoviral vector comprises only one expression cassette.

A cell comprising the adenoviral vector of the invention may be any cell such as a eukaryotic ceil, bacterial cell, animal cell, human cell, mouse cell etc. A cell may be an *in vitro, ex vivo* or in vivo cell. For example, the cell may be used for producing the adenoviral vector *in vitro, ex vivo* or in vivo, or the cell may be a target, such as a tumor cell, which has been infected with the adenoviral vector.

In a method of producing granzyme and perforin transgenes in a cell, a vehicle comprising the vector of the invention is carried into a cell and furthermore, granzyme and perforin transgenes are expressed, and the protein is translated and secreted in a paracrine manner. "A vehicle" may be any viral vector, plasmid or other tool, such as a particle, which is able to deliver the vector of the invention to a target cell. Any conventional method known in the art can be used for delivering the vector to the cell.

Tumor specific immune response may be increased in a subject by the present invention. Cytotoxic T cells and/or natural killer cells are stimulated, produced and targeted as a consequence of the construct properties.

The recombinant Ad5 vectors of the invention have been constructed for replication competence in cells, which have defects in the Rb-pathway, specifically Rb-p16 pathway. These defective cells include all tumor cells in animals and humans. In a preferred embodiment of the invention, the vector is capable of selectively replicating in cells having defects in the Rb-pathway. As used herein "defects in the Rb-pathway" refers to mutations and/or epigenetic changes in any genes or proteins of the pathway. Due to these defects, tumor cells overexpress E2F and thus, binding of Rb by E1A CR2, that is normally needed for effective replication, is unnecessary.

Any cancers or tumors, including both malignant and benign tumors as well as primary tumors and metastasis may be targets of gene therapies. In a specific embodiment of the invention the cancer is any solid tumor. In a preferred embodiment of the invention, the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, Hp cancer, meninges cancer, mouth cancer, nerve cancer, palate cancer, parotid gland cancer, peritoneum cancer, pharynx cancer, pleural cancer, salivary gland cancer, tongue cancer and tonsil cancer.

A pharmaceutical composition of the invention comprises at least one type of the vectors of the invention. Furthermore, the composition may comprise at least two or three different vectors of the invention. In addition to the vector of the invention, a pharmaceutical composition may also comprise any other vectors, such as other adenoviral vectors, other therapeutically effective agents, any other agents such as pharmaceutically acceptable carriers, buffers, excipients, adjuvants, antiseptics, filling, stabilising or thickening agents, and/or any components normally found in corresponding products.

The pharmaceutical composition may be in any form, such as solid, semisolid or liquid form, suitable for administration. A formulation can be selected from a group consisting of, but not limited to, solutions, emulsions, suspensions, tablets, pellets and capsules.

The effective dose of vectors depends on at least the subject in need of the treatment, tumor type, location of the tumor and stage of the tumor. The dose may vary for example from about 10e8 viral particles (VP) to about 10e14 VP, preferably from about 5x10e9 VP to about 10e13 VP and more preferably from about 8x10e9 VP to about 10e12 VP. In one specific embodiment of the invention the dose is in the range of about 5x10e10 - 5x10e11 VP.

The vector or pharmaceutical composition of the invention may be administered to any eukaryotic subject selected from a group consisting of plants, animals and human beings, in a preferred embodiment of the invention, the subject is a human or an animal. An animal may be selected from a group consisting of pets, domestic animals and production animals.

Any conventional method may be used for administration of the vector or composition to a subject. The route of administration depends on the formulation or form of the composition, the disease, location of tumors, the patient, comorbidities and other factors. In a preferred embodiment of the invention, the administration is conducted through an intratumoral, intramuscular, intra-arterial, intravenous, intrapleural, intravesicular, intracavitary or peritoneal injection, or an oral administration.

Only one administration of oncolytic adenoviral vectors of the invention may have therapeutic effects. However, in a preferred embodiment of the invention, oncolytic adenoviral vectors or pharmaceutical compositions are administered several times during the treatment period. Oncolytic adenoviral vectors or pharmaceutical compositions may be administered for example from 1 to 10 times in the first 2 weeks, 4 weeks, monthly or during the treatment period. In one embodiment of the invention, administration is done three to seven times in the first 2 weeks, then at 4 weeks and then monthly. In a specific embodiment of the invention, administration is done four times in the first 2 weeks, then at 4 weeks and then monthly. The length of the treatment period may vary, and for example may last from two to 12 months or more.

The gene therapy of the invention is effective alone, but combination of adenoviral gene therapy with any other therapies, such as traditional therapy, may be more effective than either one alone. For example, each agent of the combination therapy may work independently in the tumor tissue, the adenoviral vectors may sensitize cells to chemotherapy or radiotherapy and/or chemotherapeutic agents may enhance the level of virus replication or effect the receptor status of the target cells. The agents of combination therapy may be administered simultaneously or sequentially.

As used herein, the term "immune checkpoint inhibitor" or "checkpoint inhibitor" refers to molecules that totally or partially reduce, inhibit, interfere with or modulate one or more checkpoint proteins. Checkpoint proteins regulate T-cell activation or function. Central to the immune checkpoint process are the cytotoxic T-lymphocyte-associated antigen 4 (CTLA-4) and programmed death 1 (PD-1) immune checkpoint pathways. The CTLA-4 and PD-1 pathways are thought to operate at different stages of an immune response. CTLA-4 is considered the "leader" of the immune checkpoint inhibitors, as it stops potentially autoreactive T cells at the initial stage of naive T-cell activation, typically in lymph nodes. The PD-1 pathway regulates previously activated T cells at the later stages of an immune response, primarily in peripheral tissues.

Inhibition of the immune checkpoint pathways has led to the approval of several new drugs: ipilimumab (anti-CTLA-4; Yervoy®), pembrolizumab (anti-PD-1; Keytruda®), and nivolumab (anti-PD-1; Opdivo®). Also, PD-L1 inhibitors, such as Atezolizumab (MPDL3280), Avelumab (MSB0010718C) and Durvalumab (MEDI4736), are available. These antagonistic antibodies have been associated with objective clinical responses in cancer patients. Antibodies targeting CTLA-4 are already marketed (e.g. Ipilimumab, Yervoy, Bristol-Myers Squibb, BMS) for metastatic melanoma. Antibody therapies with anti PD-L1 (e.g. MPDL3280A, Roche), anti PD-1 (e.g. Nivolumab, BMS) are also ongoing.

Other immune-checkpoint inhibitors include lymphocyte activation gene-3 (LAG-3) inhibitors, such as IMP321, a soluble Ig fusion protein. Other immunecheckpoint inhibitors include B7 inhibitors, such as B7-H3 and B7-H4 inhibitors. In particular, the anti-B7-H3 antibody MGA271. Also included are TIM3 (T-cell immunoglobulin domain and mucin domain 3) inhibitors. In certain embodiments the PD-1 blockers include anti-PD-L1 antibodies. In certain other embodiments the PD-1 blockers include anti-PD-1 antibodies and similar binding proteins such as nivolumab (MDX 1106, BMS 936558, ONO 4538), a fully human IgG4 antibody 30 that binds to and blocks the activation of PD-1 by its ligands PD-L1 and PD-L2; lambrolizumab (MK-3475 or SCH 900475), a humanized monoclonal IgG4 antibody against PD-1 ; CT-011 a humanized antibody that binds PD-1 ; AMP-224 is a fusion protein of B7-DC; an antibody Fc portion; BMS-936559 (MDX- 1105-01) for PD-L1 (B7-H1) blockade. Further examples of PD-L1 inhibitors that can be used in certain embodiments are Atezolizumab (MPDL3280), Avelumab (MSB0010718C) and Durvalumab.

Preferably, anti-PD-1 antibodies pembrolizumab (Keytruda), and nivolumab (Opdivo) are used in the invention. Also, PD-L1 inhibitors, such as durvalumab can be used in combination with anti-PD-1-antibodies. The preferred checkpoint inhibitors of the present invention are thus those for PD-1 and PD-L1.

In a preferred embodiment of the invention, the method or use further comprises administration of concurrent radiotherapy to a subject. In another preferred embodiment of the invention, the method or use further comprises administration of concurrent chemotherapy to a subject. As used herein "concurrent" refers to a therapy, which has been administered before, after or simultaneously with the gene therapy of the invention. The period for a concurrent therapy may vary from minutes to several weeks. Preferably the concurrent therapy lasts for some hours.

Agents suitable for combination therapy include but are not limited to All-trans retinoic acid, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imattnib, Mechlorethamine, Mercaptopurine, Methotrexate, Mitoxantrone, Oxaliplatin, Paclitaxel, Pemetrexed, Temozolomide, Teniposide, Tioguanine, Valrubicin, Vinblastine, Vincristine, Vindesine and Vinorelbine.

In a preferred embodiment of the invention, the method or use further comprises administration of verapamil or another calcium channel blocker to a subject. "Calcium channel blocker" refers to a class of drugs and natural substances which disrupt the conduction of calcium channels, and it may be selected from a group consisting of verapamil, dihydropyridines, gallopamil, diltiazem, mibefradil, bepridil, fluspirilene and fendiline.

In a preferred embodiment of the invention, the method or use further comprises administration of autophagy inducing agents to a subject. Autophagy refers to a catabolic process involving the degradation of a cell's own components through the lysosomal machinery. "Autophagy inducing agents" refer to agents capable of inducing autophagy and may be selected from a group consisting of, but not limited to, mTOR inhibitors, P13K inhibitors, lithium, tamoxifen, chloroquine, bafilomycin, temsirolimus, sirolimus and temozolomide. In a specific embodiment of the invention, the method further comprises administration of temozolomide to a subject. Temozolomide may be either oral or intravenous temozolomide.

In one embodiment of the invention, the method or use further comprises administration of chemotherapy or anti-CD20 therapy or other approaches for blocking of neutralizing antibodies. "Anti-CD20 therapy" refers to agents capable of killing CD20 positive cells and may be selected from a group consisting of rituximab and other anti-CD20 monoclonal antibodies. "Approaches for blocking of neutralizing antibodies" refers to agents capable of inhibiting the generation of anti-viral antibodies that normally result from infection and may be selected from a group consisting of different chemotherapeutics, immunomodulatory substances, corticoids and other drugs. These substances may be selected from a group consisting of, but not limited to, cyclophosphamide, cyclosporin, azathioprine, methylprenisolone, etoposide, CD40L, CTLA4Ig4, FK506 (tacrolismus), IL-12, IFN-gamma, interleukin 10, anti-CD8, anti-CD4 antibodies, myeloablation and oral adenoviral proteins.

The oncolytic adenoviral vector of the invention induces virion mediated oncolysis of tumor cells and activates human immune response against tumor cells. In a preferred embodiment of the invention, the method or use further comprises administration of substances capable to downregulating regulatory T-cells in a subject. "Substances capable to down- regulating regulatory T-cells" refers to agents that reduce the number of cells identified as T-suppressor or Regulatory T-cells. These cells have been identified as consisting one or many of the following immunophenotypic markers: CD4+, CD25+, FoxP3+, CD127- and GITR+. Such agents reducing T- suppressor or Regulatory T-cells may be selected from a group consisting of anti-CD25 antibodies or chemotherapeutics.

An object of the invention is an oncolytic adenoviral vector comprising an oncolytic adenoviral vector comprising an adenovirus serotype 5 (Ad5) nucleic acid backbone, a 24 bp deletion (D24) in the Rb binding constant region 2 of E1, a nucleic acid sequence encoding granzyme and a nucleic acid sequence encoding perforin. Another object of the invention is the oncolytic adenoviral vector, wherein the nucleic acid sequence encoding granzyme is located at the E3 region and the nucleic acid sequence encoding perforin between the L5 and E4 polyA signals. More specifically, the transgenes are located between the L5 and E4 polyA signals and/or in E3, with 2.7 kb deletion in E3. Still another object of the invention is the oncolytic adenoviral vector, wherein the nucleic acid sequence encoding granzyme is under CMV promoter. Still another object of the invention is the oncolytic adenoviral vector, wherein the nucleic acid sequence encoding granzyme is under CMV(CuO) promoter. In one aspect of the invention the nucleic acid sequence encoding perforin is under RSV promoter. According to another aspect of the invention, the nucleic acid sequence encoding granzyme and the nucleic acid sequence encoding perforin are connected with IRES and in the E3 and/or between the L5 and E4 polyA signals region. According to one preferred object of the invention, the nucleic acid sequence encoding perforin and the nucleic acid sequence encoding granzyme are under E3 promoter. According to a preferred embodiment the granzyme is Granzyme B and perforin is Perforin1. In one preferred embodiment the oncolytic adenoviral vector comprises a native E1 A promoter.

One object of the invention is the oncolytic adenoviral vector comprising at least one expression cassette. In another object of the invention, the oncolytic adenoviral vector is capable of selectively replicating in cells having defects in the Rb-pathway.

Also, a cell comprising the adenoviral vector is one aspect of the invention.

Another aspect of the invention is a pharmaceutical composition comprising the adenoviral vector of the invention. The adenoviral vector for use in the treatment of cancer or in the manufacture of a medicament for treating cancer in a subject is also an aspect of the invention. The cancer can be selected selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

The subject of the treatment can be a human or an animal.

The treatment of cancer with adenoviral vector in a subject can be conducted through an intratumoral, intramuscular, intra-arterial, intravenous, intrapleural, intravesicular, intracavitary or peritoneal injection, or an oral administration. The injection or the administration can be conducted several times during the treatment period. Also, an oncolytic adenoviral vector having a different fiber knob of the capsid compared to the vector of the earlier treatment can be used. The treatment of cancer in a subject can further comprise an administration of concurrent radiotherapy and/or concurrent chemotherapy to a subject. The treatment of cancer in a subject can further comprise an administration of checkpoint inhibitors, verapamil or another calcium channel blocker, autophagy inducing agents, temozolomide, chemotherapy or anti-CD20 therapy or other approaches for blocking of neutralizing antibodies, and/or substances capable to downregulating regulatory T-cells in a subject.

A method of producing perforin and granzyme in a cell *in vitro,* wherein the method comprises:
a) carrying a vehicle comprising an oncolytic adenoviral vector to a cell, and
b) expressing perforin and granzyme of said vector in the cell,
is an aspect of the present invention.

Also, a use of the oncolytic adenoviral vector for producing perforin and granzyme in a cell *in vitro* is an aspect of the invention.

The nucleotide sequences of vectors show at least 66%, preferably at least 70%, more preferably at least 75%, even more preferably at least 80% identity to SEQ ID NO: 1, SEQ ID NO: 2, SEQ ID NO:3 or SEQ ID NO:4. Still more preferably the nucleotide sequences show at least 85% or at least 90% or 95%, more preferably at least 98%, most preferably 99% identity to the nucleic acid sequence of SEQ ID NO: 1, SEQ ID NO: 2, SEQ ID NO:3 or SEQ ID NO:4.

A further object of the invention is a method of treating cancer in a human subject in need thereof, wherein a therapeutically effective amount of a pharmaceutical formulation comprising an oncolytic adenovirus comprising nucleotide sequence having at least 70% identity to SEQ ID NO: 1 or SEQ ID NO:2 and a diluent or carrier is administered. In the most preferred embodiment the nucleotide sequence is SEQ ID NO: 1 or SEQ ID NO: 2. Another object of the invention is a method, wherein a therapeutically effective amount of a pharmaceutical formulation comprising a combination of oncolytic adenovirus comprising a nucleotide sequence having at least 70% identity to SEQ ID NO: 3 and oncolytic adenovirus comprising a nucleotide sequence having at least 70% identity to SEQ ID NO: 4 is administered. In the most preferred embodiment, the combination of adenoviruses has nucleotide sequences according to SEQ ID NO: 3 and SEQ ID NO:4. Said methods can further comprise administering an effective amount of an oncolytic adenoviral vector or a pharmaceutical composition comprising the oncolytic adenoviral vector to the subject through intratumoral injection, wherein the oncolytic adenoviral vector comprises the adenovirus according to the invention. In said methods the effective amount of the oncolytic adenoviral vector contains between 5X10e10 - 5X10e11 viral particles. They also induce a specific antitumor immune response, including activation of CD8+ T cells, in tumors expressing the perforin and granzyme from the oncolytic adenoviral vector. In said methods for treatment, the oncolytic adenoviral vector can be administered three to seven times in the first two weeks of treatment.

In said treatment methods cancer can be selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

According to one embodiment, the administration of the adenoviral vector or vectors or pharmaceutical composition can be conducted through an intratumoral, intramuscular, intra-arterial, intravenous, intrapleural, intravesicular, intracavitary or peritoneal injection, or an oral administration. According to another embodiment, the adenoviral vector or vectors or pharmaceutical composition can be administered several times during a treatment period. According to a preferred embodiment, concurrent radiotherapy can be administered to the subject. According to a more preferred embodiment, concurrent chemotherapy can be administered to the subject. According to a still more preferred embodiment verapamil or another calcium channel blocker can be administered to the subject.

In one preferred object of the invention, is a method for treatment with the adenoviral vector or vectors further comprising administering substances capable to downregulating regulatory T-cells in the subject.

Any method or use of the invention may be either *in vivo, ex vivo* or *in vitro* method or use.

The present invention is illustrated by the following examples, which are not intended to be limiting in any way.

### EXAMPLES

### Cloning of oncolytic viruses

Oncolytic viruses were constructed using genetic engineering tools. The construction of oncolytic vectors included the following steps:

### 1. Shuttle Plasmid Constructions:

Shuttle plasmid constructions for the viruses TGX-01/02/13: Insertion of the human Granzyme B (GrB), human Perforin (PERF1) and PERF1-IRES-GrB coding sequences into shuttle plasmid pAd1129-27. Shuttle plasmid construction for TGX-14: insertion of the sequence: GrB expression under the CMV promoter into E3 region (utilizing shuttle plasmid for derivatives for E3 genes). PERF1 expression under the control of RSV promoter and bGH A signal, inserted between the fiber and the E4 genes (utilizing shuttle plasmid for derivatives for E4 genes, pAd1130). Plasmid DNA purification, and confirmation of their identity by restriction analysis and sequencing.

pAd1129-27 contains the Ad5 E3 region and a hybrid Ad5/3 fiber gene. A 2.7 kb BglII fragment is deleted from the E3 region and is replaced with a multiple cloning site. At first, an empty CMV-CuO-IRES-SV40pA cassette will be inserted into the MCS. The CMV(CuO) promoter contains a copy of the cumate operator (CuO) (Mullick et al., 2006). In "regular" 293 cells and other cell lines, the CuO operator is inactive and transcription takes place from the CMV promoter. In 293-CymR cells, which express the CymR repressor, the CymR repressor binds onto the CuO operator, thereby preventing the transcription of the transgene. In a second step the sequences encoding GrB, PERF1, PERF1 -IRES-GrB will be inserted into the pAd1129-27 derivative accordingly for the TGX, to generate the expression cassettes depicted in the figure below (Figure 1).

### 2. Cosmid Construction

The entire genomes of the recombinant adenoviruses were reconstituted in cosmids (Figure 2). Transfection-grade cosmid DNAs were purified and their identity was confirmed by restriction analysis.

### Virus rescue and characterization

Excision of the adenovirus genome from the cosmids and transfection into 293 or 293-Cymr or A549 cells. Three virus plaques per construct were harvested and amplified in small scale. Their identity will be verified by restriction analysis of their genome using 3 enzymes (Hirt supernatant).

### Western Blot

Expression of the various transgenes from the viruses were assessed by infecting a reporter cell line with the various virus clones, lysing the cells post-infection, separating the total protein content by PAGE, and performing western blottings (WB), anti-GrB, and anti-PERF1 antibodies to detect the various transgene products by chemiluminescence. These experiments will enable us to select virus clones for amplification and purification.

### Small-scale amplification

Small-scale amplification of the viral clones, purification on two CsCI gradients, dialysis against an isotonic buffer (GTS buffer: 2.5% glycerol, 25 mM NaCl, 20 mM Tris-HCI, pH 8.0), filtration through a low-protein binding 0.22 µm filter, and virus particle (VP) concentration determination by ultraviolet absorbance (Abs260-SDS method) was carried out.

### Confirmation of virus identity by sequencing

The identity of the purified recombinant adenoviruses was confirmed by sequencing regions of the virus genomes specific for these recombinant viruses, including the 24-bp deletion in the E1A CDS, the entire mono- or dicistronic cassettes inserted into the E3 region, the Ad5/3 hybrid fiber, E4 region and the junctions between the DNA fragments ligated to each other during the cosmid construction. Experimental sequences were assembled into contigs and aligned with the expected sequences of the viruses.

Viral genomic DNAs were extracted from CsCI-purified virus particles using proteinase K and resuspended into 30 µL TE pH 7.5. Their quality was assessed by spectrophotometry (Abs260/280/320 nm) and agarose gel electrophoresis. TGX viruses' DNAs were sent to a third party for shotgun library making and sequencing. Libraries were generated using a Nextera XT kit (Illumina, Inc). They were pooled, quantified by qPCR, and sequenced on a 2x300 MiSeq run (Illumina, Inc)..fastq files were processed using the program "BWA Aligner". The software Geneious v11.1.5 (Biomatters Ltd) was used to visualize the .bam files and calculate the consensus sequences. The consensus sequences were aligned to the predicted sequences of the virus genomes using the software SnapGene v4.2.6.

### Titration of the viral suspension

Titration of the viral suspension: determination of the concentration of infectious units per mL virus suspension was performed by TCID50 assay.

### Cloning of TGX-01 (Ad5/3-D24-GrB) in more detail

To construct, amplify, purify, titrate and characterize an oncolytic adenovirus vector characterized by the following features:
- 24-bp deletion in E1A CR2
- a CMV-GrB-SV40pA cassette inserted in place of the E3 region
- a hybrid Ad5/3 fiber

Recombinant adenovirus TGX01 was constructed by reconstituting its entire genome in a cosmid (pTGX01). The following 5 DNA fragments were ligated to each other:
1. The 27 kb Pacl-Spel fragment from pAd5/3-d24-WTE3
2. The 813 bp Spel-Csi fragment from pAd5/3-d24-WTE3
3. The 3024 bp CsiI-PflMI fragment from pAd1129-GrB-F
4. The 4036 bp PflMI-PacI fragment from pAd5/3-d24-WTE3
5. The 8 kb-long Pacl fragment from pAd5dSfi

Where:
- pAd5/3-d24-WTE3 (pAd5/3-d24-GMCSF)
- pAd1129-GrB-F is a shuttle plasmid that contains a CMV-GrB-SV40pA cassette inserted in place of the Ad5 E3 region, and a hybrid Ad5/3 fiber
- pAd5dSfil is a cosmid that contains the entire Ad5 genome. The 8 kb Pacl fragment provides the cosmid backbone for pTGX01
   pAd1129-GrB-F was constructed by inserting the following 3 DNA fragments into the Spel site of pAd1129-27:
   1. The 753 bp Spel-Bsal PCR fragment obtained by amplifying the CMV-CuO promoter using pAd1127-17 as template and primers 282-1 and 282-2
   2. The 783 bp Bsal PCR fragment obtained by amplifying the GrB coding sequence using pAd1127-GrB as template and primers 282-3 and 282-4
   3. The 147 bp Bsal-Spel PCR fragment obtained by amplifying the SV40pA signal using pAd1127-WT-eGFP-F as template and primers 282-5 and 282-6
- pAd1129-27 is a shuttle plasmid that contains the Ad5 E3 region with a multiple cloning site in place of a 2.7 kb BglII deletion and a hybrid Ad5/3 fiber
- pAd1127-17 is a shuttle plasmid that contains a copy of the inducible CMV-CuO promoter
- pAd1127-GrB is a shuttle plasmid that contains a copy of the human Granzyme B (GenBank NM_004131).
- pAd1127-WT-eGFP-F is a plasmid that contains a copy of the SV40pA signal pAd1127-GrB was constructed by inserting a synthetic dsDNA fragment containing the Granzyme B coding sequence between the Dralll sites of pAd1127-16R.

Where:
pAd1127-16R is an E1/plX shuttle plasmid containing an empty CMV expression cassette.

Primers:
282-1: TTGACATTGATTATTGACTAGTT
282-2: GCTCATGGTCTCTCGGCCCGCGGAGGCTGGATCGGTCC
282-3: ATGATCGGTCTCAGCCGCCACCATGCAACCAATCC
282-4: CGTGACGGTCTCTAGTTTTAGTGGCGTTTCATGGTTTTC
282-5: AGTGACGGTCTCAAACTTGTTTATTGCAGCTTATAATGG
282-6: GCTCATACTAGTTAAGATACATTGATGAGTTTGGACAAAC

### Cloning of TGX-02 (Ad5/3-D24-PERF) in more detail

To construct, amplify, purify, titrate and characterize an oncolytic adenovirus vector characterized by the following features:
- 24-bp deletion in E1A CR2
- a CMV-PERF1-SV40pA cassette inserted in place of the E3 region
- a hybrid Ad5/3 fiber

Recombinant adenovirus TGX02 was constructed by reconstituting its entire genome in a cosmid (pTGX02). The following 5 DNA fragments were ligated to each other:
1. The 27 kb Pacl-Spel fragment from pAd5/3-d24-WTE3
2. The 813 bp Spel-Csi fragment from pAd5/3-d24-WTE3
3. The 3948 bp CsiI-PflMI fragment from pAd1129-PERF1 -F
4. The 4036 bp PflMI-PacI fragment from pAd5/3-d24-WTE3
5. The 8 kb-long Pacl fragment from pAd5dSfi

Where:
- pAd5/3-d24-WTE3 (pAd5/3-d24-GMCSF)
- pAd1129-PERF1-F is a shuttle plasmid that contains a CMV-PERF1-SV40pA cassette inserted in place of the Ad5 E3 region, and a hybrid Ad5/3 fiber
- pAd5dSfil is a cosmid that contains the entire Ad5 genome. The 8 kb Pacl fragment provides the cosmid backbone for pTGX02
pAd1129-PERF1-F was constructed by inserting the following 3 DNA fragments into the Spel site of pAd1129-27:
1. The 753 bp Spel-Bsal PCR fragment obtained by amplifying the CMV-CuO promoter using pAd1127-17 as template and primers 282-1 and 282-2.
2. The 1707 bp BsmBI PCR fragment obtained by amplifying the PERF1 coding sequence using pCMV6-PERF1 as template and primers 282-7 and 282-8.
3. The 147 bp Bsal-Spel PCR fragment obtained by amplifying the SV40pA signal using pAd1127-WT-eGFP-F as template and primers 282-5 and 282-6.

Where:
- pAd1129-27 is a shuttle plasmid that contains the Ad5 E3 region with a multiple cloning site in place of a 2.7 kb BglII deletion and a hybrid Ad5/3 fiber
- pAd1127-17 is a shuttle plasmid that contains a copy of the inducible CMV-CuO promoter
- pCMV6-PERF1 is a plasmid that contains a copy of the human Perforin1 gene (GenBank NM_005041).
- pAd1127-WT-eGFP-F is a plasmid that contains a copy of the SV40pA signal

Primers:
282-1: TTGACATTGATTATTGACTAGTT
282-2: GCTCATGGTCTCTCGGCCCGCGGAGGCTGGATCGGTCC
282-5: AGTGACGGTCTCAAACTTGTTTATTGCAGCTTATAATGG
282-6: GCTCATACTAGTTAAGATACATTGATGAGTTTGGACAAAC
282-7: ATGATCCGTCTCAGCCGCCACCATGGCAGCCCGTCTGCTC
282-8: CGTGACCGTCTCTAGTTTCACCACACGGCCCCACTCCGG.

### Cloning of TGX-13 (Ad5/3-D24-PERF-GrB) in more detail

To construct, amplify, purify, titrate and characterize an oncolytic adenovirus vector characterized by the following features:
- 24-bp deletion in E1A CR2
- a dicistronic cassette expressing PERF1 and GrB in place into the E3 region (2.7 kb BglII deletion)
- a hybrid Ad5/3 fiber

TGX13 was constructed using the AdenoQuick2.0 method. The following plasmids were combined to reconstitute in cosmid pTGX13 the entire genome of the virus:
- pAd1127-18 is a E1-plX shuttle plasmid that contains the Ad5 left ITR, packaging signal, and the E1A E1B and pIX genes. The plasmid is characterized by the presence of a 24-bp deletion in the E1A region, which deletes amino acids "L T C H E A G F" (121-128) from the conserved region CR2
- pAd1128 is a 25 kb-long plasmid that contains the Ad5 E2 region and most of the late genes
- pTGX112 is a pAd1129 plasmid derivative that contains a CMV-PERF1 -IRES-GrB-pA expression cassette inserted in place of the E3 region
- pAd1130 contains the sequence encompassing psn 32796-right end in the Ad5 genome, including the right ITR and the entire E4 region
pTGX112 was constructed by ligating the following 6 DNA fragments to each other:
1. the 2756 bp Earl-Sfil fragment from pAd1129-GrB-F
2. the 1823 bp SfiI fragment from pAd1129-GrB-F
3. the 2430 bp SfiI-DralII fragment from pAd1129-PERF1-F
4. a 353 bp PCR fragment encompassing the 3'end of the PERF1 coding sequence, obtained using pAd1129-PERF1-F as template and primers 288-26 and 288-27
5. a 605 bp fragment encompassing an IRES element, obtained by digesting plasmid pAd1129-IRES-att with SalI
6. a 176 bp PCR fragment encompassing the 5'end of the GrB coding sequence, obtained using pAd1129-GrB-F as template and primers 288-28 and 288-29

Where:
- pAd1129-GrB-F and pAd1129-PERF1-F were made for the construction of TGX01 and TGX02, respectively.
- pAd1129-IRES-att is a plasmid that contains an attenuated version of the EMCV Internal Ribosome Entry Site (IRES).

Primers:
288-26: CAGGAGCTGAGGACGAGCACCGTGTGGGACAATAACAACC
288-27: AGGGGGGGGAGGGAGAGGGGTCACCACACGGCCCCACTCC
288-28: CGGGATCCTCTAGAGTCGACATGCAACCAATCCTGCTTCT
288-29: GAAGCCACCGCACCTCTTCAGAGACTTCTGATCCCAGATC.

### Cloning of TGX-14 (Ad5/3-D24-GrB-PERF) in more detail

To construct, amplify, purify, titrate and characterize an oncolytic adenovirus vector characterized by the following features:
- 24-bp deletion in E1A CR2
- a CMV-GrB-SV40pA expression cassette inserted in place of the E3 region (2.7 kb BglII deletion)
- A RSV-PERF1-bGHpA expression cassette inserted between the L5 and E4 polyA signal.
- a hybrid Ad5/3 fiber

TGX14 was constructed using the AdenoQuick2.0 method. The following plasmids were combined to reconstitute in a cosmid the entire sequence of TGX14:
1) pAd1127-18 is a E1-plX shuttle plasmid that contains the Ad5 left ITR, packaging signal, and the E1A E1B and pIX genes. The plasmid is characterized by the presence of a 24-bp deletion in the E1A region, which deletes amino acids "L T C H E A G F" (121-128) from the conserved region CR2
2) pAd1128 is a 25 kb-long plasmid that contains the Ad5 E2 region and most of the late genes
3) pTGX113b is a pAd1129 plasmid derivative that contains a CMV-GrB-pA expression cassette inserted in place of the E3 region, and an RSV-PERF1-pA expression cassette inserted between the L5 and E4 polyA signals.
4) pAd1130 contains the sequence encompassing psn 32796-right end in the Ad5 genome, including the right ITR and the entire E4 region.

Where:
- pTGX113b was constructed by inserting the PflMI-SfiI fragment from pTGX113a into plasmid pAd1129-GrB-F
- pAd1129-GrB-F is a E3/fiber shuttle plasmid characterized by a CMV-GrB-SV40pA cassette inserted in place of the Ad5 E3 region (2.7 kb BglII deletion), and a hybrid Ad5/3 fiber. pAd1129-GrB-F had been used to construct pTGX01
- pTGX113a is a pAd1129 derivative that includes a hybrid Ad5/3 fiber and a RSV-PERF1-bGHpA expression cassette inserted between the L5 and E4 polyA signals. pTGX113a was constructed by inserting PCR-amplified RSV promoter, PERF1 coding sequence and bGH polyA signal into the unique Spel site of pAd1129-32.

### Preclinical testing

### Cell lines

Melanoma cell lines, A2058 (ATCC® CRL-1147™), A375 (ATCC® CRL-1619™) and SK-Mel-28 (ATCC® HTB-72™), were cultured in DMEM high glucose medium supplemented with 10% FCS and penicillin/streptomycin. Trypsin-EDTA was used to detach and subculture cells at 80% confluence for *in vitro* assays.

### In vitro - cell viability assay (MTS)

Cell viability was evaluated by using the MTS Cell Proliferation Assay kit (Abcam) according to the manufacturer's instructions with the following modifications. MTS assay kit is a colorimetric method for the sensitive quantification of viable cells. It can be used to assess cell proliferation, cell viability and cytotoxicity. The MTS assay is based on the reduction of the MTS tetrazolium compound by viable mammalian cells to generate a colored formazan dye that is soluble in cell culture media. This conversion is thought to be carried out by NAD(P)H-dependent dehydrogenase enzymes in metabolically active cells. The formazan dye is quantified by measuring the absorbance at 490 nm. Cells were seeded in 96 well plate at a concentration of 3000 and 5000 cells/well. Treatment was initiated in a final volume of 200µL. 72 hours later, MTS assay was performed according to manufacturer protocol. Cells were incubated with and without 0.1, 1, 10, 100 and 1000 viral particles (VP) of tested virus. The plates were shaken briefly on a shaker and absorbance measured at 490 nm. Absorbance values for untreated cells were set to correspond to 100% viability, and viability of treated cells was expressed as a percentage of the untreated control absorbance value.

### Data analyses

All parameters were analysed using GraphPad Prism software (version 7). Statistical analyses have been performed using RM-one-way ANOVA followed by a Tukey post-test or T test (Mann Whitney Test).

### EXAMPLE 1 In vitro - cell viability assay (MTS)

Double transgene viruses coding for GrB and PERF1 (TGX-13 and TGX-14) have shown anti-cancer superiority when administered at the concentration of 1000VP/cell (respectively 35,05 and 30,33% of untreated cells) over singe transgene viruses TGX-01 (coding for GrB, 64,90% of untreated cells) and TGX-02 (coding for PERF1, 50,14% of untreated cells) *in vitro* against A375 cell line (Figure 7a).

Double transgene vector showed also anti-cancer superiority (TGX-14) over single transgene vectors (TGX-01 and TGX-02) in SK-MEL-28 cell lines when tested at the concentration of 100VP/cell (respectively 59; 64,97; 95,39 and 76,89 % of untreated cells), (Figure 7b).

Improved oncolytic properties of TGX-14 has been observed in A2058 cell line, infected with 1000VP/cell (TGX-14 was superior over TGX-01 and 02 respectively: 56,55; 72,05 and 62,77 % of untreated cells) (Figure 7c).

### REFERENCES

Hlongwane, P., Mungra, N., Madheswaran, S., Akinrinmade, O., Chetty, S. & Barth, S. 2018. Human Granzyme B Based Targeted Cytolytic Fusion Proteins. Biomedicines, 6.
Krzewski, K. & Coligan, J. E. 2012. Human NK Cell Lytic Granules and Regulation of Their Exocytosis. Front Immunol, 3, 335.
Kuryk, L., Haavisto, E., Garofalo, M., Capasso, C., Hirvinen, M., Pesonen, S., Ranki, T., Vassilev, L. & Cerullo, V. 2016. Synergistic Anti-Tumor Efficacy of Immunogenic Adenovirus Oncos-102 (Ad5/3-D24-Gm-Csf) And Standard of Care Chemotherapy in Preclinical Mesothelioma Model. Int J Cancer, 139, 1883-93.
Kuryk, L., Møller, A.-S. W. & Jaderberg, M. 2018. Combination of Immunogenic Oncolytic Adenovirus Oncos-102 With Anti-Pd-1 Pembrolizumab Exhibits Synergistic Antitumor Effect in Humanized A2058 Melanoma Hunog Mouse Model. Oncoimmunology, 8, 1-11.
Mullick, A., Xu, Y., Warren, R., Koutroumanis, M., Guilbault, C., Broussau, S., Malenfant, F., Bourget, L., Lamoureux, L., Lo, R., Caron, A. W., Pilotte, A. & Massie, B. 2006. The Cumate Gene-Switch: A System for Regulated Expression in Mammalian Cells. Bmc Biotechnol, 6, 43.
Osinska, I., Popko, K. & Demkow, U. 2014. Perforin: An Important Player in Immune Response. Cent Eur J Immunol, 39, 109-15.
Rousalova, I. & Krepela, E. 2010. Granzyme B-Induced Apoptosis in Cancer Cells and its Regulation (Review). International Journal of Oncology, 37*.*
Voskoboinik, I., Whisstock, J. C. & Trapani, J. A. 2015. Perforin and Granzymes: Function, Dysfunction and Human Pathology. Nat Rev Immunol, 15, 388-400.

## Claims

1. An oncolytic adenoviral vector comprising an adenovirus serotype 5 (Ad5) nucleic acid backbone, a 24 bp deletion (D24) in the Rb binding constant region 2 of E1, a nucleic acid sequence encoding granzyme and a nucleic acid sequence encoding perforin.

2. The oncolytic adenoviral vector according to claim 1, wherein the nucleic acid sequence encoding granzyme is located at the E3 region and the nucleic acid sequence encoding perforin is located between the L5 and E4 polyA signals.

3. The oncolytic adenoviral vector according to claim 1 or 2, wherein the nucleic acid sequence encoding granzyme is under CMV promoter.

4. The oncolytic adenoviral vector according to any one of claims 1-3, wherein the nucleic acid sequence encoding perforin is under RSV promoter.

5. The oncolytic adenoviral vector according to any one of claims 1-4, wherein the nucleic acid sequence encoding granzyme and the nucleic acid sequence encoding perforin are connected with IRES.

6. The oncolytic adenoviral vector according to any one of claims 1, 2 or 5, wherein the nucleic acid sequence encoding perforin and the nucleic acid sequence encoding granzyme are under E3 promoter.

7. The oncolytic adenoviral vector according to any one of claims 1-6, wherein granzyme is Granzyme B is and perforin is Perforin1.

8. The oncolytic adenoviral vector according to any one of claims 1-7 comprising a native E1 A promoter.

9. The oncolytic adenoviral vector according to any one of claims 1-8 further comprising an Ad5/3 chimerism as a capsid modification.

10. The oncolytic adenoviral vector according to any one of claims 1-4 or 6-9 comprising a nucleotide sequence having at least 70% identity to SEQ ID NO: 1 or according to any one of claims 1-9 comprising a nucleotide sequence having at least 70% identity to SEQ ID NO:2.

11. A cell comprising the adenoviral vector according to any one of claims 1-10.

12. A pharmaceutical composition comprising the adenoviral vector according to any one of claims 1-10.

13. The adenoviral vector according to any one of claims 1-10 for use in the treatment of cancer or in the manufacture of a medicament for treating cancer in a subject.

14. The adenoviral vector for use according to claim 13, wherein the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

15. The adenoviral vector for use according to claim 13 or 14, wherein the treatment of cancer in a subject is conducted through an intratumoral, intramuscular, intra-arterial, intravenous, intrapleural, intravesicular, intracavitary or peritoneal injection, or an oral administration.

16. The adenoviral vector for use according to claim 15, wherein the injection or the administration is conducted several times during the treatment period, or wherein an oncolytic adenoviral vector having a different fiber knob of the capsid compared to the vector of the earlier treatment is used.

17. The adenoviral vector for use according to any one of claims 13-16, wherein the treatment of cancer in a subject further comprises an administration of a checkpoint inhibitor, verapamil or another calcium channel blocker, autophagy inducing agents, temozolomide, chemotherapy or anti-CD20 therapy or other approaches for blocking of neutralizing antibodies, substances capable to downregulating regulatory T-cells in a subject.

18. A method of producing granzyme and perforin in a cell *in vitro,* wherein the method comprises:
i. carrying a vehicle comprising an oncolytic adenoviral vector according to any one of claims 1-10 to a cell, and
ii. expressing granzyme and perforin of said vector in the cell.

19. A use of the oncolytic adenoviral vector according to any one of claims 1-10 for producing granzyme and perforin in a cell *in vitro.*
